# EUROPEAN PATENT SPECIFICATION

(11) **EP 3 456 192 B1**
(45) Date of publication and mention of the grant of the patent: **03.09.2025**
(21) Application number: 18183994.5
(22) Date of filing: 29.06.2009
(51) Int. Cl.: A01K 67/027, C12N 15/13, C07K 16/00, C12N 15/85, C12N 5/10

(54) **USE OF ANTIBODY-PRODUCING MURINE MAMMALS TO GENERATE HIGH AFFINITY ANTIBODIES**
VERWENDUNG VON ANTIKÖRPER-PRODUZIERENDEN MURINISCHEN SÄUGETIEREN ZUR ERZEUGUNG VON ANTIKÖRPERN MIT HOHER AFFINITÄT
UTILISATION DE MAMMIFÈRES MURINS PRODUISANT UN ANTICORPS POUR GÉNÉRER DES ANTICORPS DE HAUTE AFFINITÉ

(30) Priority: 27.06.2008 WO PCT/NL2008/050430
(43) Date of publication of application: 20.03.2019
(62) Divisional of application: 12175544.1
(73) Proprietor: Merus N.V., 3584 CT Utrecht (NL)
(72) Inventor: Houtzager, Erwin, 3702 SC Zeist (NL); Pinto, Rui Daniel, 3581 JS Utrecht (NL); Logtenberg, Ton, 3584 CM Utrecht (NL); Throsby, Mark, 3584 CM Utrecht (NL)
(74) Representative: Simmons & Simmons LLP (Munich)

(56) References cited:
- EP-A1- 1 439 234
- EP-A2- 0 814 159
- WO-A1-02/066630
- WO-A1-2004/106375
- WO-A2-2005/068622
- WO-A2-2006/117699
- SIRAC C ET AL: "Role of the monoclonal k chain V domain and reversibility of renal damage in a transgenic model of acquired Fanconi syndrome", BLOOD, AMERICAN SOCIETY OF HEMATOLOGY, US, vol. 108, no. 2, 15 July 2006 (2006-07-15), pages 536 - 543, XP003030514, ISSN: 0006-4971, DOI: 10.1182/BLOOD-2005-11-4419
- PELANDA R ET AL: "A prematurely expressed Ig(kappa) transgene, but not V(kappa)J(kappa) gene segment targeted into the Ig(kappa) locus, can rescue B cell development in lambda5-deficient mice.", IMMUNITY SEP 1996, vol. 5, no. 3, September 1996 (1996-09-01), pages 229 - 239, XP002556633, ISSN: 1074-7613
- LONBERG NILS: "Human antibodies from transgenic animals", NATURE BIOTECHNOLOGY, NATURE PUBLISHING GROUP, NEW YORK, NY, US, vol. 23, no. 9, 1 September 2005 (2005-09-01), pages 1117 - 1125, XP002442149, ISSN: 1087-0156
- PRESTA ET AL: "Engineering of therapeutic antibodies to minimize immunogenicity and optimize function", ADVANCED DRUG DELIVERY REVIEWS, ELSEVIER BV, AMSTERDAM, NL, vol. 58, no. 5-6, 7 August 2006 (2006-08-07), pages 640 - 656, XP024892146, ISSN: 0169-409X, [retrieved on 20060807]
- WEINER LOUIS M: "Fully human therapeutic monoclonal antibodies", JOURNAL OF IMMUNOTHERAPY, LIPPINCOTT WILLIAMS & WILKINS, HAGERSTOWN, MD, US, vol. 29, no. 1, 1 January 2006 (2006-01-01), pages 1 - 9, XP009118693, ISSN: 1524-9557
- JAKOBOVITS AYA ET AL: "From XenoMouse technology to panitumumab, the first fully human antibody product from transgenic mice.", NATURE BIOTECHNOLOGY OCT 2007, vol. 25, no. 10, October 2007 (2007-10-01), pages 1134 - 1143, XP002556637, ISSN: 1087-0156
- DE KRUIF JOHN ET AL: "Human immunoglobulin repertoires against tetanus toxoid contain a large and diverse fraction of high-affinity promiscuous V(H) genes.", JOURNAL OF MOLECULAR BIOLOGY 3 APR 2009, vol. 387, no. 3, 3 April 2009 (2009-04-03), pages 548 - 558, XP002556330, ISSN: 1089-8638
- WAGNER KOEN: "Declaration by Koen Wagner, Ph.D", 10 November 2020 (2020-11-10), XP093134694, Retrieved from the Internet <URL:https://batavia.internal.epo.org/digital-file-repository/digital-file-repository-frontend-prod/dossier/EP18183994>
- BRINSTER RALPH L ET AL: "Expression of a microinjected immunoglobulin gene in the spleen of transgenic mice", NATURE, vol. 306, 24 November 1983 (1983-11-24), pages 332 - 336, XP093135034, Retrieved from the Internet <URL:https://www.nature.com/articles/306332a0>
- LARRICK J W ET AL: "Polymerase chain reaction using mixed primers: cloning of human monoclonal antibody variable region genes from single hybridoma cells", BIOTECHNOLOGY. THE INTERNATIONAL MONTHLY FOR INDUSTRIAL BIOLOGY, NATURE PUBLISHING GROUP, US, vol. 7, 1 September 1989 (1989-09-01), pages 934 - 938, XP000400715, ISSN: 0733-222X, DOI: 10.1038/NBT0989-934
- HEIRINCHS A ET AL: "Universal cloning and direct sequencing of rearranged antibody V genes using C region primers, biotin-captured cDNA and one-side PCR", JOURNAL OF IMMUNOLOGICAL METHODS, ELSEVIER SCIENCE PUBLISHERS B.V.,AMSTERDAM, NL, vol. 178, 1 January 1995 (1995-01-01), pages 241 - 251, XP000572653, ISSN: 0022-1759, DOI: 10.1016/0022-1759(94)00262-U
- NAKATANI TOMOYUKI ET AL: "Functional Expression of Human Monoclonal Antibody Genes Directed Against Pseudomonal Exotoxin A in Mouse Myeloma Cells", BIOTECHNOLOGY, vol. 7, 7 August 1989 (1989-08-07), pages 805 - 810, XP093136133, Retrieved from the Internet <URL:https://www.nature.com/articles/nbt0889-805>
- HIETER P.A. ET AL: "Evolution of human immunoglobulin kappa J region genes.", JOURNAL OF BIOLOGICAL CHEMISTRY, vol. 257, no. 3, 1 February 1982 (1982-02-01), US, pages 1516 - 1522, XP093133759, ISSN: 0021-9258, DOI: 10.1016/S0021-9258(19)68223-2
- HIETER P A ET AL: "Cloned human and mouse kappa immunoglobulin constant and J region genes conserve homology in functional segments", CELL, ELSEVIER, AMSTERDAM NL, vol. 22, no. 1, 1 November 1980 (1980-11-01), pages 197 - 207, XP023909659, ISSN: 0092-8674, [retrieved on 19801101], DOI: 10.1016/0092-8674(80)90168-3
- C. SIRAC ET AL: "Light chain inclusion permits terminal B cell differentiation and does not necessarily result in autoreactivity", PROCEEDINGS OF THE NATIONAL ACADEMY OF SCIENCES, vol. 103, no. 20, 16 May 2006 (2006-05-16), pages 7747 - 7752, XP055368772, ISSN: 0027-8424, DOI: 10.1073/pnas.0509121103

## Description

### TECHNICAL FIELD

The invention relates to the use of a transgenic murine mammals for producing high affinity antibodies, which are expressed from at least partially exogenous nucleic acids (transgenes). Transgenes to produce such transgenic murine mammals and methods to produce such heterologous antibodies; methods and vectors for producing such transgenic murine mammals are disclosed. The present invention is defined by the appended claims.

### BACKGROUND OF THE INVENTION

B cells mediate humoral immunity by producing specific antibodies. The basic structural subunit of an antibody (Ab) is an immunoglobulin (Ig) molecule. Ig molecules consist of a complex of two identical heavy (H) and two identical light (L) polypeptide chains. At the amino terminus of each H chain and L chain is a region that varies in amino acid sequence named the variable (V) region. The remaining portion of the H and L chains is relatively constant in amino acid sequence and is named the constant (C) region. In an Ig molecule, the H and L chain V regions (VH and VL) are juxtaposed to form the potential antigen-binding site. The genes that encode H and L chain V regions are assembled somatically from segments of germline DNA during precursor B (pre-B) cell differentiation: V, D and J gene segments for the H chain and V and J gene segments for the L chain. Within Ig V regions are three regions of greatest amino acid sequence variability that interact to form the antigen-recognition site and are thus referred to as complementarity determining regions (CDRs).

The V gene segment encodes the bulk of the V region domain, including CDR1 and CDR2. Diversity in CDR1 and CDR2 derives from sequence heterogeneity among multiple different germline-encoded V segments. CDR3 is encoded by sequences that are formed by the joining of H chain V, D, and J gene segments and L chain V and J segments and by mechanisms that create nucleotide sequence heterogeneity where these segments are combined. Additional diversity may be derived from pairing of different H and L chain V regions. Collectively these processes yield a primary repertoire of antibodies encoded by germline gene segments and expressed by newly-formed B cells.

An additional source of antibody diversity is imposed on top of the diversity generated by recombination of Ig gene segments. B cells are able to introduce mutations into the antibody V regions that they express, a process called somatic hypermutation. Thus, when an animal first encounters an antigen, the antigen binds to a specific B cell which happens to carry antibodies which have a V domain which binds the antigen. This primary response may activate this B cell to go on to secrete the cognate antibody. These activated B cells can also now target a somatic mutation process to their rearranged antibody gene segments and thus allow the production of daughter cells which make variants of the antibodies of the primary response. A selection process amplifies those variant B cell descendants which make an antibody of improved affinity of the antigen. In B cells, somatic hypermutations are targeted to a restricted genomic region including both the rearranged VH and VL genes. Thus somatic mutation allows affinity maturation - the production and selection of high affinity antibodies. Therefore, somatic mutation is important for the generation of high affinity antibodies.

The exquisite specificity and high affinity of antibodies and the discovery of hybridoma technology allowing the generation of monoclonal antibodies (mAbs) has generated great expectations for their utilization as targeted therapeutics for human diseases. MAbs are identical because they are produced by a single B cell and its progeny. MAbs are made by fusing the spleen cells from a mouse that has been immunized with the desired antigen with myeloma cells to generate immortalized hybridomas. One of the major impediments facing the development of in vivo applications for mAbs in humans is the intrinsic immunogenicity of non-human Igs. Patients respond to therapeutic doses of mouse mAbs by making antibodies against the mouse Ig sequences (Human Anti Mouse Antibodies; HAMA), causing acute toxicity, alter their biodistribution and accelerate clearance, thus reducing the efficacy of subsequent administrations (Mirick, et al., (2004) Q. Nucl. Med. Mol. Imaging 48, 251-257).

To circumvent the generation of HAMA, antibody humanization methods have been developed in an attempt to produce mAbs with decreased immunogenicity when applied to humans. These endeavors have yielded various recombinant DNA-based approaches aimed at increasing the content of human amino acid sequences in mAbs while retaining the specificity and affinity of the parental non-human antibody. Humanization began with the construction of mouse-human chimeric mAbs (Morrison, S. L., et al., (1984). Proc. Natl. Acad. Sci. USA., 81, 6851-5), in which the Ig C regions in murine mAbs were replaced by human C regions. Chimeric mAbs contain 60-70% of human amino acid sequences and are considerably less immunogenic than their murine counterparts when injected into humans, albeit that a human anti-chimeric antibody response was still observed (Hwang, W. Y., et al. (2005). Methods, 36, 3-10).

In attempts to further humanize murine mAbs, CDR grafting was developed. In CDR grafting, murine antibodies are humanized by grafting their CDRs onto the VL and VH frameworks of human Ig molecules, while retaining those murine framework residues deemed essential for specificity and affinity (Jones, P.T., et al., (1986). Nature, 321, 522). Overall, CDR-grafted antibodies consist of more than 80% human amino acid sequences (Queen, C. et al. (1989) Proc. Natl. Acad. Sci. U. S. A. 86, 10029; Carter, P. et al. (1992) Proc. Natl. Acad. Sci. U. S. A. 89, 4285). Despite these efforts, CDR-grafted, humanized antibodies were shown to still evoke an antibody response against the grafted V region (Hwang, W. Y., et al. (2005). Methods, 36, 3).

Subsequently to CDR grafting, humanization methods based on different paradigms such as resurfacing (Padlan, E. A., et al., (1991). Mol. Immunol., 28, 489), superhumanization (Tan, P., D. A., et al., (2002) J. Immunol., 169, 1119), human string content optimization (Lazar, G. A., et al., (2007). Mol. Immunol., 44, 1986) and humaneering have been developed in an attempt to further decrease the content of non-human sequences in therapeutic mAbs (Almagro, J. C., et al., (2008). Frontiers in Bioscience 13, 1619). As in CDR grafting approaches, these methods rely on analyses of the antibody structure and sequence comparison of the non-human and human mAbs in order to evaluate the impact of the humanization process into immunogenicity of the final product. When comparing the immunogenicity of chimeric and humanized antibodies, humanization of variable regions appears to decrease immunogenicity further (Hwang, W. Y., et al. (2005). Methods, 36, 3-10).

De-immunization is another approach developed to reduce the immunogenicity of chimeric or mouse antibodies. It involves the identification of linear T-cell epitopes in the antibody of interest, using bioinformatics, and their subsequent replacement by site-directed mutagenesis to human or non-immunogenic sequences (WO09852976A1). Although de-immunized antibodies exhibited reduced immunogenicity in primates, compared with their chimeric counterparts, some loss of binding affinity was observed (Jain, M., et al., (2007). Trends in Biotechnol. 25, 307).

The development of phage display technology complemented and extended humanization approaches in attempts to obtain less immunogenic mAbs for therapy in humans. In phage display, large collections ('libraries') of human antibody VH and VL regions are expressed on the surface of filamentous bacteriophage particles. From these libraries, rare phages are selected through binding interaction with antigen; soluble antibody fragments are expressed from infected bacteria and the affinity of binding of selected antibodies is improved by mutation (Winter, G., et al. (1994). Annu. Rev. Immunol. 12, 433). The process mimics immune selection, and antibodies with many different bindings specificities have been isolated using this approach (Hoogenboom, H. R., et al. (2005). Nat. Biotechnol., 23, 1105). Various sources of H and L chain V regions have been used to construct phage display libraries including those isolated from non-immune or immune donors. In addition, phage display libraries have been constructed of V regions that contain artificially randomized synthetic CDR regions in order to create additional diversity. Often, antibodies obtained from phage display libraries are subjected to in vitro affinity maturation to obtain high affinity antibodies (Hoogenboom, H. R., et al. (2005). Nat. Biotechnol., 23, 1105).

The creation of transgenic mouse strains producing human antibodies in the absence of mouse antibodies has provided another technology platform for the generation of specific and high affinity human mAbs for application in humans. In these transgenic animals, the endogenous mouse antibody machinery is inactivated and replaced by human Ig loci to substantially reproduce the human humoral immune system in mice (Jakobovits, A., et al. (2007). Nat. Biotechnol. 25, 1134. Lonberg, N. (2005). Nat. Biotechnol. 23, 1117). B cell development as well as Ig diversification by recombination of gene segments is faithfully reproduced in these mice, leading to a diverse repertoire of murine B cells expressing human Igs. By immunizing these mice with antigens, it was further demonstrated that these transgenic animals accumulated somatic mutations in the V regions of both heavy and light chains to produce a wide diversity of high-affinity human mAbs (Lonberg, N. (2005). Nat. Biotechnol. 23, 1117).

The question, whether "fully human" mAbs such as derived from phage display libraries or transgenic mice are less immunogenic than humanized mAbs cannot be answered yet, because full immunogenicity data are available for just two human mAbs. An anti-tumor necrosis factor mAb, developed from phage-displayed human libraries induced antibody responses in 12% of patients - at the higher end of the incidence of anti-antibody responses of the humanized antibodies (Hwang, W. Y., et al. (2005). Methods, 36, 3-10).

Evaluation of the immunogenicity of the first registered human mAb generated by the transgenic approach demonstrated that mAb treatment resulted in the generation of antibodies in approximately 5.5% of treated cancer patients (Jakobovits, A., et al. (2007). Nat. Biotechnol. 25, 1134., Lofgren, J. A., et al. (2007). J. Immunol. 178, 7467).

EP0814159 discloses a theoretical experiment for the production of a transgenic mouse for the production of antibodies, comprising a rearranged human V domain linked to a human light chain constant domain. No transgenic mice have been produced.

A need therefore remains for a method and means for producing antibodies that are specific for their targets, but are less immunogenic. As described herein, the reduction of immunogenicity is at least partially achieved by providing a transgenic murine mammal comprising, at least in its B cell lineage, a nucleic acid encoding at least an immunoglobulin light chain, wherein the light chain encoding sequence is provided with a means that renders it resistant to DNA rearrangements and/or somatic hypermutations, preferably such a murine mammal is a rodent, more specifically a mouse. The nucleic acid encodes a rearranged human VL region of a human immunoglobulin.

### SUMMARY OF THE INVENTION

As claimed, the present invention relates to the use of a transgenic murine mammal comprising integrated in its genome a rearranged nucleic acid encoding a rearranged human immunoglobulin light chain variable region, wherein said light chain variable region is capable of pairing with at least two different heavy chains encoded by the murine mammal, such that variety in specificity of antibodies is retained through rearrangements and hypermutations in the heavy chains, for the generation of high affinity antibodies comprising said rearranged human immunoglobulin light chain variable region, wherein the antibodies are encoded by VH genes that have undergone somatic hypermutation upon immunization and the non-mutated rearranged human VL gene integrated in the genome.

### DETAILED DESCRIPTION OF THE INVENTION

In the remainder of the specification mice are typically used as examples of the murine mammals. The transgenic murine hosts are capable of mounting an immune response to an antigen, where the response produces antibodies having human light chain variable regions. Mice have been used for the production of B-lymphocytes for immortalization for the production of antibodies. Since mice are easy to handle, can be bred in large numbers, and are known to have an extensive immune repertoire, mice will usually be the animal of choice. Therefore, in the following discussion, the discussion will refer to mice, but it is described that it should be understood that other murine animals, may be readily substituted for the mice, following the same procedures.

The reason for preventing rearrangements and hypermutation is that in this manner a non-immunogenic polypeptide can be chosen beforehand knowing that this polypeptide chain will remain non-immunogenic. At least the light chain of the resulting immunoglobulin is thus less immunogenic. The resulting antibody needs to have (usually) both a light- and a heavy chain. The non-immunogenic chain must therefore be capable of pairing with the other chain. The other chain may be an endogenous chain, an exogenous chain or a hybrid of both. For human therapy, the non-immunogenic chain should be as close to human as possible.

A means for rendering a gene encoding an immunoglobulin chain (or chains) resistant to DNA rearrangement and/or mutation is of course removal of all genetic elements responsible for said rearrangement and/or mutation. The drawback thereof is that the variability of the two chains is eliminated, whereas the the murine mammal used in accordance with the invention retains the variability in the heavy chain and inhibits and/or prevents the rearrangement-mutation of the light chain.

The elements for rearrangement and/or hypermutation characterized so far are located within the loci for immunoglobulins. Therefore the means for rendering the immunoglobulin encoding sequence resistant to DNA rearrangement and/or mutation is inserting the gene in a locus outside the immunoglobulin loci.

Thus, according to the invention a transgenic murine mammal is used wherein the light chain encoding sequence is integrated in the genome of the murine mammal in a locus outside the immunoglobulin loci. In one embodiment, the insertion is in a locus that is resistant to gene silencing. According to one embodiment of the invention, the integration is in the Rosa-locus or a comparable locus.

It is preferred to provide an expression cassette which can be inserted in a Rosa locus or comparable locus with a means that allows expression of the immunoglobulin chain(s) essentially limited to cells of B cell lineage, preferably with a means that allows expression of the light chain encoding nucleic acid during a certain stage of the development of B cells. The term "essentially limited expression" indicates that expression is predominantly in cells of the B-cell lineage, but that lower levels of expression in other cells, as compared to the level of expression in B-cells, is possible. In a preferred embodiment, the term "essentially limited expression" indicates that the expression is exclusively present in cells of the B-cell lineage. Such means typically and preferably include B cell (developmental stage) specific promoters such as CD19, CD20, µHC (all V-genes), VpreB 1, VpreB2, VpreB3, λ5, Igα, Igβ, κLC (all genes), λLC (all genes), BSAP (Pax5). Although it is very well possible to direct the expression of the DNA rearrangement and/or mutation resistant chain by such promoters, they are relatively weak. A strong promoter will typically be required to ensure adequate surface expression of the B cell receptor (made up of the membrane attached Ig H and L chain) and to compete with the expression and pairing of endogenous chains (if present) through allelic exclusion. Such a promoter, however is usually not tissue specific. To confer tissue specificity, an indirect system employing Cre/lox or the like is preferred. The desired chain is put under control of a strong promoter inhibited by an element that can be removed by the action of a Cre-protein, leading to activation of the desired immunoglobulin encoding gene. This system is described in detail in Wunderlich F. T. (2004), "Generation of inducible Cre systems for conditional gene inactivation in mice", Inauguraldissertation zur Erlangung des Doktorgrades der Mathematisch-Naturwissenschaftlichen Fakultät der Universität zu Köln; http://deposit.ddb.de/cgi-bin/dokserv?idn= 97557230x&dok_var=d1&dok_ext=pdf&filename=97557230x.pdf.

The immunoglobulin light chain is capable of pairing with different heavy chains encoded by the murine mammal. The light chain will then be the same (and less immunogenic) in all antibodies, but variety in specificity is retained through rearrangements and hypermutations in the heavy chains. It may in that case be preferable to silence at least one of the endogenous loci encoding a light chain, although allelic exclusion may render this unnecessary.

According to this embodiment, preferably the endogenous kappa (κ) light chain locus is functionally silenced.

If the endogenous κ light chain locus is silenced, but also for other reasons, it is preferred that the resistant light chain is a κ light chain, preferably a light chain that has a germline-like sequence. According to the invention such a light chain would lead to an antibody with reduced immunogenicity. The preferred germline sequence is based on the human IGKV1-39 (012) as this light chain is very frequently observed in the human repertoire (de Wildt et al. 1999. J. Mol. Biol. 285(3):895) and has superior thermodynamic stability, yield and solubility (Ewert et al. 2003. J. Mol. Biol. 325(3):531).

The following gives more specific embodiments of the expression cassette with which the murine mammal for use according to the invention can be provided. This is typically advantageous for immunoglobulins.

Thus the invention uses in a specific embodiment a transgenic murine mammal wherein the light chain encoding nucleic acid comprises in 5'-3' direction: a B cell specific promoter, a leader, a rearranged human V gene, optionally a MoEκi enhancer, a constant region (κ) and optionally a (truncated) MoEκ3' enhancer. Neuberger identified and examined a novel B-cell specific enhancer located downstream of the kappa constant region (EP004690251). This enhancer has been shown to play a crucial role in the expression of kappa genes as removal of the 808bp enhancer strongly reduced expression. Deletion of the 3' kappa enhancer also strongly reduced the level of somatic hypermutations (SHM). In transgenic and cell expression studies it has been revealed that reduced, mutated or deleted 3' kappa enhancers not only lowered expression levels but also decreased the level of somatic hypermutations. Currently, it can not be determined whether the 3' kappa enhancer is involved in SHM processes, expression regulation or both (review Odegard, V. H., et al. (2006). Nat. Rev. Immunol. 6, 573; Inlay, M., et al. (2002). Nat. Immunol. 3, 463.).

Detailed expression studies using engineered variants of the 3' kappa enhancer indicated that a 50 nucleotide region is sufficient to drive expression. However for proper expression a reduced sequence of 145 nucleotides is preferred (EP04690251; Meyer, K. B., et al. (1990) Nucleic Acids Res. 18(19):5609-15)

Thus the invention in one aspect provides in the context of the claimed use a nucleic acid for insertion into the genome of a murine mammal that is an expression cassette for the expression of a desired proteinaceous molecule in cells developing into mature B cells during a certain stage of development, said cassette comprising means for preventing silencing of expression of the desired proteinaceous molecule after introduction into a host cell, and means for timing expression of the desired proteinaceous molecule with the desired developmental stage of the host cell.

An expression cassette is defined as a nucleic acid that has been provided with means for introduction into the genome of a host cell, such as sequences which allow for homologous recombination with a certain site in the genome. Usually the nucleic acid will be DNA, typically double stranded. Typically the expression cassette will be provided to the cell in a vector from which it is transferred to the genome of the cell. The expression cassette further comprises all elements necessary for expression of the gene in a host cell, although in certain aspects some of such elements may be present on a second nucleic acid to be introduced, whereby these elements act in trans. Elements necessary for expression in a host cell include promoters, enhancers and other regulatory elements. Only those elements are necessary that are not provided by the host cell.

It is important that the expression of the gene of interest is not silenced in the genome of the host cell, especially not in the development stage where expression is required. This can be done by various means, such as insertion into the endogenous locus or by providing the cassette with nucleic acid elements that prevent silencing (Kwaks et al. (2006) Trends Biotechnol. 24(3), p.137-142). It is preferred that the expression cassette is inserted in a locus that is not silenced in the host cells (EP 01439234).

Said means for prevention of silencing comprise STabilizing Anti-Repression-sequences (STAR^{®}-sequences) and Matrix Attachment Regions (MARs). A STAR sequence is a nucleic acid sequence that comprises a capacity to influence transcription of genes in cis. Typically, although not necessarily, a STAR sequence does not code by itself for a functional protein element. In one embodiment one STAR element is used. Preferably, however, more than one STAR element is used. In a particularly preferred embodiment an expression cassette for use in accordance with the invention is provided with two STAR sequences; one STAR sequence at the 5' side of the coding sequence of the immunoglobulin gene and one STAR sequence at the 3' side of the coding sequence of the immunoglobulin gene. MARs are DNA sequences that are involved in anchoring DNA/chromatin to the nuclear matrix and they have been described in both mammalian and plant species. MARs possess a number of features that facilitate the opening and maintenance of euchromatin. MARs can increase transgene expression and limit position-effects.

According to one aspect of the invention it is important that expression from the cassette only occurs during a certain period in the development of a cell, in particular a developing B cell, more in particular a B cell in a transgenic murine mammal, in particular a mouse. In this particular case the developmental period is chosen such that the expression of the light chain does not significantly interfere with the normal differentiation and/or maturation of the cell and when applicable, allows for pairing of the polypeptide chain produced with its counterpart.

This may, in one embodiment, be achieved by providing a nucleic acid useful for the invention, wherein said means for timing expression is a promoter of which the activity is essentially limited to the certain stage of development. In a developing B cell, which, e.g. after immunization is maturing and/or differentiating, the expression of the gene of interest, which is the rearranged human VL region of a human immunoglobulin, must not interfere (significantly) with said maturation and/or differentiation and it needs to be timed such that the resulting polypeptide can pair with its counterparts. Therefore described is a nucleic acid useful for the invention wherein said certain stage starts at a stage immediately preceding or coinciding with the onset of the expression of light chain molecules by said cells at a certain stage of development into a mature B cell.

This may be achieved by selecting a promoter which is active only during said suitable period. Such a promoter may be a CD19 promoter, the Ig-α promoter, the Ig-β promoter, the µhc (all genes) promoter, the Vk promoter or analogues or homologues thereof.

The promoter as disclosed above may not drive the expression of the gene of interest directly. Instead it drives the expression of a gene of which the product activates in trans the expression of the gene of interest. Such an activating gene may be a gene encoding a so-called Cre recombinase or Cre-like protein. The expression cassette for the gene of interest may e.g. be provided with a sequence that inhibits expression of the gene of interest. Said sequence can be removed by the action of the Cre recombinase, which is under control of the desired promoter (active during the proper stage of development). A set of expression cassettes may be required.

Therefore described is a set of nucleic acids that are expression cassettes, wherein one nucleic acid comprises an expression cassette encoding a Cre-like protein under control of a promoter active during the desired stage of development of the host cell and the second nucleic acid comprises a sequence encoding a desired proteinaceous molecule under control of a constitutive promoter which can be activated by the action of a Cre-like protein. Said activation is may be achieved by removal of a stop sequence flanked by loxP sites. The Cre/lox system is described in detail in Rajewsky et al. (1996) J. Clin. Invest. 98, p.600-603. Such systems are reviewed in Wunderlich F. T. (2004), "Generation of inducible Cre systems for conditional gene inactivation in mice", Inauguraldissertation zur Erlangung des Doktorgrades der Mathematisch-Naturwissenschaftlichen Fakultät der Universität zu Köln; http://deposit.ddb.de/cgi-bin/dokserv?idn=97557230x&dok_var=d1&dok_ext=pdf&filename=9755723 0x.pd.

The invention uses a transgenic murine mammal which has been provided with an expression cassette described herein, wherein the desired proteinaceous molecule is a rearranged human immunoglobulin light chain variable region. A preferred polypeptide is a germline or germline-like light chain. A most preferred polypeptide is O12, preferably the rearranged germline kappa light chain IGKV1-39*0 1/ IGKJ1*01 (nomenclature according to the IMGT database, http://www.imgt.org).

The rearranged human immunoglobulin light chain variable region is rendered incapable of rearrangement and of excluded of any sequence modification such as normally operating on Ig during the process of B cell affinity maturation. Therefore, the invention may use a transgenic murine mammal which has been provided with an expression cassette, wherein said re-arrangement and sequence modifications are prevented by the absence of elements at least partially responsible for somatic hypermutation such as, for example, the MoEκi enhancer.

An expression cassette may comprise means for prevention of silencing. In one aspect, said means for prevention of silencing are means for insertion into a locus in the genome of the host cell that is resistant to silencing. Said means for insertion are preferably means for homologous recombination into said site resistant to silencing. A preferred locus when the murine mammal is a mouse is the rosa-locus.

A further preferred expression cassette comprises in 5'-3' direction: a Vκ promoter, a mouse leader, a human V gene, optionally a MoEκi enhancer, a rat constant region (Cκ) and optionally a (truncated) MoEκ3' enhancer.

Yet a further preferred expression cassette comprises in 5'-3' direction: a Vκ promoter, a human leader, a human V gene, optionally a MoEκi enhancer, a rat constant region (Cκ) and optionally a (truncated) MoEκ3' enhancer.

Of course the ultimate goal of the invention is to produce antibodies to be used in human therapeutics. Thus described herein is a method for producing a desired antibody comprising exposing a murine mammal used according to the invention to an antigen such that an antibody response is induced and isolating the antibodies specific for the antigen.

Further described is a method for producing a desired antibody comprising exposing a murine mammal used according to the invention to an antigen such that an antibody response is induced and isolating cells producing such antibodies, culturing and optionally immortalizing said cells and harvesting said antibodies.

Further described is a method for producing a desired antibody comprising exposing a murine mammal used according to the invention to an antigen such that an antibody response is induced and isolating a nucleic acid encoding at least part of such an antibody, inserting said nucleic acid or a copy or a derivative thereof in an expression cassette and expressing said antibody in a host cell.

The methods for producing antibodies from transgenic mice are known to a person skilled in the art. Particularly preferred are methods for production of mixtures of antibodies from one cell, whereby the nucleic acids encoding these antibodies have been derived from mice according to the invention.

These so-called oligoclonics are disclosed in WO04106375 and WO05068622.

The present invention uses transgenic murine mammals, preferably mice, capable of generating specific and high affinity hybrid mouse-human antibodies with human immunoglobulin light chain variable (VL) regions in or near germline configuration and preferably murine immunoglobulin heavy chain variable (VH) regions that may have accumulated somatic mutations during the process of antigen-driven affinity maturation. It is envisaged that the murine VH regions of the hybrid antibodies may be subjected to humanization procedures to yield mAbs that have reduced immunogenicity when applied in humans based on germline or near-germline VL regions and murine VH regions that have been humanized.

In particular, it has been shown in the present invention that transgenic mice that harbor a DNA expression construct that encodes a rearranged human VL region under the control of cis-acting genetic elements that provide timely and regulated expression of the transgene on a significant proportion of B cells during B cell development, yet lack elements that direct the somatic hypermutation machinery to the transgene, are capable of generating specific and high affinity mouse-human hybrid antibodies with essentially unmutated L chains. It is shown that the rearranged human transgene is capable of pairing with a diversity of endogenous murine immunoglobulin H chains to form mouse-human hybrid immunoglobulins expressed on the surface of B cells and to sufficiently facilitate murine B cell development to obtain a sizeable and diverse peripheral B cell compartment.

In a preferred embodiment, the transgene expression construct for use in accordance with the invention harbors the coding sequences of a human rearranged L chain V region under the control of a human VL promoter to direct B-cell specific expression. In addition, the construct harbors the murine 3' Ck enhancer sequence for B cell specific and inducible and high level expression of the transgene. Furthermore, the construct is designed to lack regulatory elements that facilitate the recruitment of the somatic hypermutation machinery to the transgene, such as the intron enhancer and the 3' C-kappa enhancer.

In a related embodiment, the rearranged human VL gene for use in accordance with the invention is inserted in the murine Rosa26 locus by site-specific integration. The Rosa26 locus is useful in the context of the "targeted transgenesis" approach for efficient generation of transgenic organisms (such as mice) with a predictable transgene expression pattern.

In a preferred embodiment, the rearranged human VL region for use in accordance with the invention is selected for its capacity to pair with at least two different murine VH genes so as to ensure the generation of a population of B cells with a diverse VH gene repertoire. A method of obtaining such VL regions comprises amplifying a repertoire of rearranged VH genes from the B cells of mice and a repertoire of human rearranged germline VL regions from the B cells of humans and cloning them into phagemid display vectors to prepare diverse libraries of hybrid immunoglobulins in bacteria. By nucleotide sequence analysis of collections of unselected and antigen-selected VH/VL pairs, human germline VL genes that pair with many different murine VH genes are identified. A collection of human germline VL genes with this capacity is described.

It is shown that upon immunization with antigen, the B cells are capable of mounting an immune response, leading to the generation of B cells that secrete hybrid antibodies with high specificity and affinity. The V regions encoding these antibodies are characterized by the human transgenic light chain that harbors no or very few mutations and a murine heavy chain that harbors a variable number of mutations introduced by the somatic hypermutation machinery.

Strategies to obtain high affinity hybrid monoclonal antibodies from the transgenic mice by hybridoma and display technologies are contemplated as well as procedures to humanize the murine VH regions to obtain less immunogenic antibodies for application in humans.

In one embodiment, the invention makes use of an immunoglobulin L chain transgene construct comprising DNA sequences that encode a human immunoglobulin VL region in combination with a light chain constant region (CL) of an animal immunoglobulin protein, which sequences are operably linked to transcription regulatory sequences that, when integrated in a transgenic murine mammal, produce an Ig VL-CL polypeptide with a human VL region that is not or marginally subject to somatic hypermutation. The Ig VL is capable of pairing with rearranged VH-CH polypeptides that are generated during B cell development in the transgenic murine mammal, with said VH-CH polypeptides retaining the capacity to undergo somatic hypermutation upon stimulation. The CL region may be of any animal species and is generally capable of pairing with the CH regions of the transgenic murine mammal.

Also described is the use of a transgene construct as above in producing a transgenic murine mammal capable of the production of hybrid antibodies consisting of VL-CL polypeptides and VH-CH polypeptides in which the VL region is of human origin and the CL, VH and CH may be of any animal species, including human. Upon immunization, these transgenic animals are capable of generating high affinity antibodies encoded by somatically hypermutated VH genes and the non-mutated rearranged human VL gene encoded by the transgene.

In another aspect, described is a process for the production of a transgenic murine mammal capable of the production of hybrid antibodies in response to antigenic challenge, comprising functionally disrupting the endogenous immunoglobulin light chain locus and inserting into the animal genome a transgene construct as described.

The invention includes the use of a transgenic murine mammal obtainable by this process in the production of B cells that produce immunoglobulin having human VL light chain as defined in the claims. There is described a process for the production of B cells that produce immunoglobulin having human VL and binding to a selected antigen, comprising challenging a murine mammal obtainable by a process as above with said antigen and screening for B cells from said animal that bind said antigen. Further described are B cells obtainable by this process and hybridomas obtainable by immortalizing such B cells, e.g. hybridomas obtained by fusing B cells as above with myeloma cells. Also described is a process for producing monoclonal antibody comprising cultivating such a hybridoma. Further described is the use of the above B cells in producing a hybridoma or corresponding monoclonal antibody.

Yet further described is a process for the production of immunoglobulin having human VL chain and binding to a selected antigen, comprising challenging a murine mammal obtainable as above with said antigen and obtaining immunoglobulin there from.

In one strategy, as an individual step, a rearranged VL region encoded by human germline V and J gene segments and a light chain constant region of any animal species but preferably a murine constant region is introduced into the mouse germ line. The transgene DNA may be introduced into the pronuclei of fertilized oocytes or embryonic stem cells. The integration may be random or homologous depending on the particular strategy to be employed. For example, the VL transgene may be introduced by random insertion, resulting in mice that bear one or multiple copies of the transgene in the genome. Alternatively, the human VL transgene may be targeted to a specific genomic locus using site-specific recombination as described in the art.

In one preferred embodiment, the VL transgene for use in accordance with the invention is targeted to the murine ROSA26 locus which is a suitable integration site allowing strong and predictable expression of inserted transgenes (EP 1439234). The targeting vector allows insertion of a single copy of a gene expression cassette, thus avoiding modulation of transgene expression by the arrangement of multiple copies. By choosing the autosomal Rosa26 locus as insertion site, the expression pattern of the inserted transgene in the murine mammal is predictable. Furthermore, random X inactivation and/or modulation by chromosomal position effects are avoided. This also eliminates the need to generate and analyse multiple transgenic strains for any given transgene. Finally, the Rosa26 targeting vector for the site-specific integration can be used for multiple gene expression cassettes. Thus, it may be envisaged that 2 or more different rearranged germline human VL regions are inserted into the Rosa26 locus to further increase the diversity of the repertoire of hybrid or human antibodies.

In another embodiment, a rearranged human VL region for use in accordance with the invention may be targeted to the murine Ig kappa or lambda light chain locus so as to functionally inactivate the endogenous locus or mice containing the rearranged human VL region may be bred with mice that lack functional kappa or lambda Ig loci or both. Thus, by using transformation, using repetitive steps or in combination with breeding, transgenic murine mammals may be obtained which are able to produce antibodies harboring the human VL transgene in the substantial absence of endogenous host immunoglobulin light chains.

In one embodiment, a human VL transgene for use in accordance with the invention is selected for its capacity to pair with a substantial portion of murine VH regions to form a diverse repertoire of functional mouse-human hybrid antibodies expressed on the surface of B cells. By a substantial portion of murine VH regions is meant that the human VL pairs with at least with 0.1% of the murine VH regions generated during B cell development, more preferably with at least 1% and most preferably with at least 10%. Methods to identify human VL genes with this characteristic include randomly pairing a repertoire of human VL regions with a repertoire of murine VH regions, co-expression of VH and VL regions in appropriate eukaryotic or prokaryotic expression vectors and screening for human VL regions that pair with a substantial portion of murine VH regions. Phagemid vectors may be used to direct expression of mouse-human antibody fragments in bacterial cells or to the surface of filamentous phage and analysis of binding capacity of antibody fragments by methods known in the art.

In another embodiment, a human VL transgene for use in accordance with the invention is selected for its capacity to pair with a substantial portion of human VH regions to form a diverse repertoire of human antibodies expressed on the surface of B cells. By a substantial portion of human VH regions is meant that the human VL pairs with at least with 0.1% of the human VH regions generated during B cell development, more preferably with at least 1% and most preferably with at least 10%.

The human VL transgenic mice may be crossed with mice that harbor functional rearranged or non-rearranged human H chain immunoglobulin loci and functionally inactivated endogenous H chain Ig loci as described in the art. The functional inactivation of the two copies of each of the three host Ig loci (heavy chain, kappa and lambda light chain), where the host contains the human IgH and the rearranged human VL transgene would allow for the production of purely human antibody molecules without the production of host or host human chimeric antibodies. Such a host strain, by immunization with specific antigens, would respond by the production of mouse B-cells producing specific human antibodies, which B-cells are subsequently fused with mouse myeloma cells or are immortalized in any other manner for the continuous stable production of human monoclonal antibodies. Alternatively, said population of B cells is used as a source of VH regions that can be obtained by constructing cDNA libraries or by PCR amplification using primers for human VH regions as is known in the art.

A human rearranged VL gene is reconstructed in an appropriate eukaryotic or prokaryotic microorganism and the resulting DNA fragments can be introduced into pronuclei of fertilized mouse oocytes or embryonic stem cells. Various constructs that direct B cell specific expression of VL transgenes have been described in the art and have the following general format: a leader sequence and relevant upstream sequences to direct B cell specific expression of the transgene, a coding sequence of a human VL transgene, an enhancer sequence that directs B cell specific and high level expression of the transgene and a murine constant region gene. In a preferred format, the enhancer is the C-kappa 3' enhancer because it directs high level expression in B-lineage cells, but does not recruit somatic hypermutation when used in transgene constructs.

Murine mammals, preferably mice, comprising one or multiple copies of the transgene in the genome are isolated and analyzed for stable expression. Murine mammals are selected that show stable expression of the transgene over longer periods of time, preferably in B-cells. If required, different murine lines comprising independent insertions of one or multiple copies of the transgene, preferably on different chromosomes, are crossed to obtain murine mammals with different insertions of one or multiple copies of the transgene to increase expression of the transgene in murine mammals, preferably in B-cells.

Further described is a progeny of a transgenic murine mammal used according to the invention, the progeny comprising, at least in its B-cell lineage, a light chain encoding sequence together with a means that renders the sequence resistant to DNA rearrangements and/or somatic hypermutations.

Further described is a progeny of a transgenic murine mammal used according to the invention, the progeny comprising an expression cassette for the expression of a desired proteinaceous molecule in cells during a certain stage of development in cells developing into mature B cells.

Further described is a cell that is isolated from a transgenic murine mammal used according to the invention, the cell comprising a light chain encoding sequence together with a means that renders the sequence resistant to DNA rearrangements and/or somatic hypermutations. Also described is a cell that is isolated from a transgenic murine mammal used according to the invention, the cell comprising an expression cassette for the expression of a desired proteinaceous molecule in cells during a certain stage of development in cells developing into mature B cells. A cell, preferably an antibody-producing B-cell or a cell that is capable of differentiating or maturating into an antibody-producing B-cell, can be used for *in vitro* production of antibodies, as is known to the skilled person, for example from Gascan et al. 1991. J. Exp. Med. 173: 747-750. Methods for immortalization of a cell described herein are known in the art and include the generation of hybridomas, for example by fusion with a myeloma cell, transformation with Epstein Barr Virus; expression of the signal transducer of activation and transcription (STAT), activation via CD40 and IL4 receptor signaling, and/or expression of Bcl6 (Shvarts et al. 2002. Genes Dev 16: 681-686).

In a separate step the mouse endogenous Kappa and Lambda light chain loci are rendered essentially non-functional such that at least the majority of B cells in the transgenic mice bear Ig receptors that contain the transgenic human VL region. Inactivation of the endogenous mouse immunoglobulin loci is achieved by targeted disruption of the appropriate loci by homologous recombination in mouse embryonic stem cells. Said targeted disruption comprises alteration of the genomic sequence such that substantially no functional endogenous mouse immunoglobulin Kappa and/or Lambda light chain is produced. The term "substantially no functional endogenous mouse immunoglobulin" indicates that the endogenous Kappa and/or Lambda light chain loci are functionally silenced such that the level of functional protein expression of the endogenous Kappa and/or Lambda light chain loci, preferably the endogenous Kappa light chain locus, is reduced to about 20% of the level of expression in a reference mouse, more preferred to about 10%, more preferred to about 5%, more preferred to about 2% and more preferred to about 1%. In a most preferred embodiment, the level of functional protein expression of the endogenous Kappa and/or Lambda light chain loci is reduced to 0%. The level of functional protein expression can be determined by means known to the skilled person, including western blotting and pairing with a mouse heavy chain. Said reference mouse is a mouse in which the endogenous Kappa and/or Lambda light chain loci is not disrupted. Said alteration comprises mutation and/or deletion of gene sequences that are required for functional expression of the endogenous immunoglobulin genes. Alternatively, said alteration comprises insertion of a nucleic acid into the endogenous mouse immunoglobulin Kappa and/or Lambda light chain loci such that the functional expression of the endogenous immunoglobulin genes is reduced. In one aspect, said nucleic acid comprises a silencing element resulting in transcriptional silencing of the endogenous immunoglobulin gene. In a further aspect, or in addition, said nucleic acid comprises a sequence that disrupts splicing and/or translation of the endogenous immunoglobulin gene, for example by introducing an exon that renders a frame shift in the coding sequence, or that comprises a premature stop codon. In each case chimeric animals are generated which are derived in part from the modified embryonic stem cells and are capable of transmitting the genetic modifications through the germ line. The mating of mouse strains with human immunoglobulin loci to strains with inactivated mouse loci yields animals which produce antibodies comprising essentially only human light chains.

A construct for homologous recombination is prepared by means known in the art and any undesirable sequences are removed, e.g., procaryotic sequences. Any convenient technique for introducing a construct for homologous recombination into a target cell may be employed. These techniques include spheroplast fusion, lipofection, electroporation, calcium phosphate-mediated DNA transfer or direct microinjection. After transformation or transfection of the target cells, target cells are selected by means of positive and/or negative markers, for example by neomycin resistance and/or acyclovir and/or gancyclovir resistance. Those cells which show the desired phenotype may then be further analyzed by restriction analysis, electrophoresis, Southern analysis, PCR, or the like. By identifying fragments which show the presence of the lesion(s) at the target locus, cells in which homologous recombination has occurred to inactivate a copy of the target locus are identified.

Furthermore, it is shown that upon immunization, the murine and human VH regions in the afore-mentioned transgenic mice but not the VL regions are capable of undergoing somatic hypermutations to generate high affinity antibodies. Advantageously, these antibodies encoded by germline VL regions are predicted to contribute to lower immunogenicity when applied in humans and result in more stable antibodies that are less prone to aggregation and thus safer for therapeutic use in humans.

MAbs derived from the afore-mentioned transgenic murine mammal all share the same identical human VL regions. It has been described that mAbs that share the same identical VL region may be co-expressed in a single clonal cell for the production of mixtures of recombinant antibodies with functional binding sites (see WO04106375 and WO05068622). Thus, the transgenic murine mammal suitable for use according to the invention provides a platform for the generation of specific and high affinity mAbs that constitute the basis for mixtures of mAbs produced by clonal cells.

It is preferred that mAbs derived from the afore-mentioned transgenic murine mammal are directed against cellular targets. Preferred targets are human surface-expressed or soluble proteins or carbohydrate molecules. Further preferred targets are surface-expressed proteins or carbohydrate molecules that are expressed on the surface of bacteria, viruses, and other pathogens, especially of humans.

More specifically, preferred targets include cytokines and chemokines, including but not limited to InterLeukin 1beta (IL1beta), IL2, IL4, IL5, IL7, IL8, IL12, IL13, IL15, IL18, IL21, IL23 and chemokines such as, for example, CXC chemokines, CC chemokines, C chemokines (or y chemokines) such as XCL1 (lymphotactin-α) and XCL2 (lymphotactin-B), and CX3C chemokines. Further included as preferred targets are receptor molecules of the cytokines and chemokines, including type I cytokine receptors such as, for example, the IL-2 receptor, type II cytokine receptors such as, for example interferon receptors, immunoglobulin (Ig) superfamily receptors, tumor necrosis factor receptor family including receptors for CD40, CD27 and CD30, serine/threonine-protein kinase receptors such as TGF beta receptors, G-protein coupled receptors such as CXCR1-CXCR7, and tyrosine kinase receptors such as fibroblast growth factor receptor (FGFR) family members, EGF receptor family members including erbB 1 (EGF-R; HER1), erbB2, (HER2), erbB3 (HER3), and erbB4 (HER4), insulin receptor family members including IGF-R1 and IGF-RII, PDGF receptor family members, Hepatocyte growth factor receptor family members including c-Met (HGF-R), Trk receptor family members, AXL receptor family members, LTK receptor family members, TIE receptor family members, ROR receptor family members, DDR receptor family members, KLG receptor family members, RYK receptor family members, MuSK receptor family members, and vascular endothelial growth factor receptor (VEGFR) family members.

Further preferred targets are targets that are over-expressed or selectively expressed in tumors such as, for example, VEGF, CD20, CD38, CD33, CEA, EpCAM, PSMA, CD54, Lewis Y, CD52, CD40, CD22, CD51/CD61, CD74, MUC-1, CD38, CD19, CD262 (TRAIL-R2), RANKL, CTLA4, and CD30; targets that are involved in chronic inflammation such as, for example, CD25, CD11a, TNF, CD4, CD80, CD23, CD3, CD14, IFNgamma, CD40L, CD50, CD122, TGFbeta and TGFalpha.

Preferred surface-expressed proteins or carbohydrate molecules that are expressed on the surface of bacteria, viruses, and other parasitic pathogens, especially of humans, include surface markers of influenza A and B viruses such as hemagglutinin (HA) and neuraminidase (NA), filoviruses such as Ebola virus, rabies, measles, rubella, mumps, flaviviruses such as Dengue virus types 1-4, tick-borne encephalitis virus, West Nile virus, Japanese encephalitis virus, and Yellow fever virus, Paramyxoviruses including Paramyxovirus such as Parainfluenza 1, 3, Rubulavirus such as Mumpsvirus and Parainfluenza 2, 4, Morbillivirus, and Pneumovirus such as Respiratory syncytial virus, Vaccinia, small pox, coronaviruses, including Severe Acute Respiratory Syndrome (SARS) virus, hepatitis virus A, B and C, Human Immunodeficiency Virus, Herpes viruses, including cytomegalovirus, Epstein Barr virus, Herpes simplex virus, and Varicella zoster virus, parvoviruses such as, for example, B19; Legionella pneumophila; Listeria monocytogenes; Campylobacter jejuni; Staphylococcus aureus; E. coli O157:H7; Borrelia burgdorferi; Helicobacter pylori; Ehrlichia chaffeensis; Clostridium difficile; Vibrio cholera; Salmonella enterica Serotype Typhimurium; Bartonella henselae; Streptococcus pyogenes (Group A Strep); Streptococcus agalactiae (Group B Strep); Multiple drug resistant S. aureus (e.g. MRSA); Chlamydia pneumoniae; Clostridium botulinum; Vibrio vulnificus; Parachlamydia pneumonia; Corynebacterium amycolatum; Klebsiella pneumonia; Linezolid-resistant enterococci (E. faecalis and E. faecium); and Multiple drug resistant Acinetobacter baumannii.

Most preferred targets are IL-6 and its receptor, IL-6Ralpha, glycoprotein-denominated gp130, RSV, especially the surface proteins F, G and SH and non-structural proteins such as N and M, and receptor tyrosine kinases, in particular erbB1 (EGF-R; HER1), erbB2, (HER2), erbB3 (HER3), erbB4 (HER4), IGF-R1 and IGF-RII, c-Met (HGF-R).

Therefore, the murine mammal suitable for use according to the invention provides a platform for the generation of specific and high affinity mAbs against the above mentioned targets that constitute the basis for mixtures of mAbs produced by clonal cells. In a preferred aspect, said specific and high affinity mAbs comprise mAbs that are directed against different epitopes on at least one of the targets. In a further preferred embodiment, said specific and high affinity mAbs comprise mAbs that are directed against different targets, such as, for example, one or more members of the EGF-receptor family, including erbB1 (EGF-R; HER1), erbB2, (HER2), erbB3 (HER3) and erbB4 (HER4).

Unless otherwise defined, scientific and technical terms used in connection with the present invention shall have the meanings that are commonly understood by those of ordinary skill in the art. Further, unless otherwise required by context, singular terms shall include pluralities and plural terms shall include the singular. Generally, nomenclatures utilized in connection with, and techniques of, cell and tissue culture, molecular biology, and protein and oligo- or polynucleotide chemistry and hybridization described herein are those well known and commonly used in the art. Standard techniques are used for recombinant DNA, oligonucleotide synthesis, and tissue culture and transformation (e.g., electroporation, lipofection). Enzymatic reactions and purification techniques are performed according to manufacturer's specifications or as commonly accomplished in the art or as described herein. The foregoing techniques and procedures are generally performed according to conventional methods well known in the art and as described in various general and more specific references that are cited and discussed throughout the present specification. See e.g., Sambrook et al. Molecular Cloning: A Laboratory Manual (3rd edition, Cold Spring Harbor Laboratory Press, Cold Spring Harbor, N.Y. (2001)). The nomenclatures utilized in connection with, and the laboratory procedures and techniques of, analytical chemistry, synthetic organic chemistry, and medicinal and pharmaceutical chemistry described herein are those well known and commonly used in the art. Standard techniques are used for chemical syntheses, chemical analyses, pharmaceutical preparation, formulation, and delivery, and treatment of patients.

### Figure legends

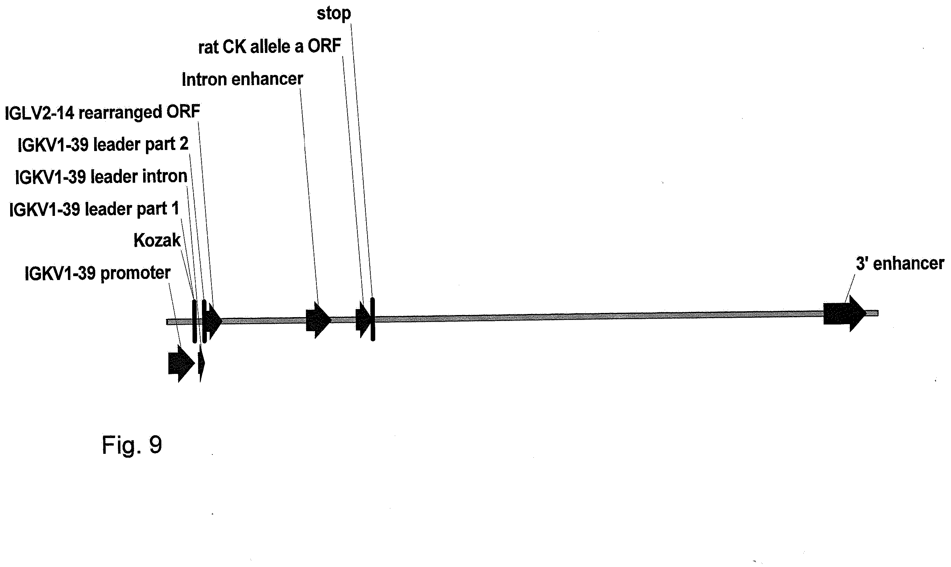

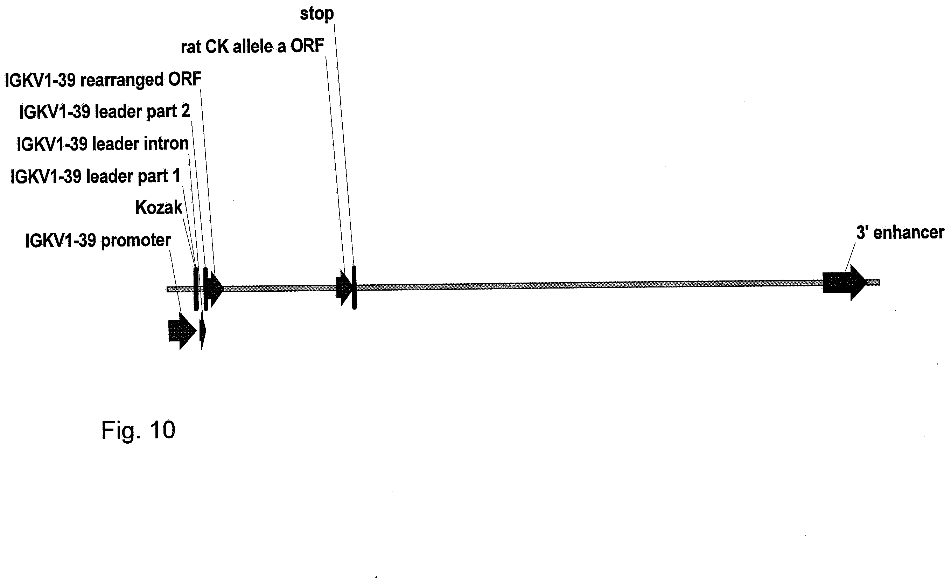

Figure 1
   A topology map of the annealing locations of mouse specific VH primers and the position of required restriction sites that are introduced by overhanging sequences at the 3' end of primers.
Figure 2
   PCR amplification steps (Amplification, Intermediate and Site introduction). The location and names of the mouse VH amplification primers (and mixtures of primers) are indicated per step.
Figure 3
   Topology of the MV1043 vector. This vector is used for the cloning of human or murine VH fragments. 012 (IGKV1-39) is indicated as the VL gene. Products of this vector in combination with helper phages in E. coli cells allow the generation of phages that display Fab fragments on the surface of the phage particles as a fusion product to the g3 protein and presence of the vector in the phage as the genetic content (F1 ORI).
Figure 4
   The topology of the mouse Ckappa locus downstream of the J-segments. Both enhancers and Ckappa region are indicated. The lower arrow indicates the region that is removed in order to silence the locus.
Figure 5
   The topology of the mouse C-lambda locus. All three active V-regions are indicated (Igl-V1, V2 and V3) as are the J-segments (Igl-J1, Igl-J2, Igl. J3, Igl-J4 and the pseudo segment Igl-J3p) and constant regions (Igl-C1, Igl-C2, Igl-C3 and Igl-C4). The regions that are deleted in order to silence the locus are indicated by deletion markers. These deletions include all active V genes (1, 2 and 3) and the intergenic segment between V2 and V3.
Figure 6
   Construct topology of IGKV1-39/J-Ck with an intron located in the leader open reading frame (ORF).
Figure 7
   Construct topology of IGLV2-14/J-Ck with an intron located in the leader open reading frame (ORF).
Figure 8
   Construct topology of VkP-IGKV1-39/J-Ck (VkP-012). The promoter originates from the IGKV1-39 gene and is placed directly in front of the required elements for efficient transcription and translation. Intergenic sequences (including the enhancers) are derived from mice and obtained from BAC clones. The C-kappa sequence codes for the kappa constant region of rat.
Figure 9
   Construct topology of VkP-IGLV2-14/J-Ck (VkP-2a2). The promoter originates from the IGKV1-39 gene and is placed directly in front of the required elements for efficient transcription and translation. Intergenic sequences (including the enhancers) are derived from mice and obtained from BAC clones. The C-kappa sequence codes for the kappa constant region of rat.
Figure 10
   Construct topology of VkP-IGKV1-39/J-Ck-Δ1 (VkP-O12-del1) is identical to VkP-IGKV1-39/J-Ck from figure 9 except that the intron enhancer region is removed.
Figure 11
   Construct topology of VkP-IGKV1-39/J-Ck-Δ2 VkP-O12-del2) is identical to VkP-IGKV1-39/J-Ck-Δ1 from figure 10 except that a large piece of the intergenic region between the Ck gene and 3'enhancer is deleted. In addition, the 3' enhancer is reduced in size from 809 bp to 125 bp.
Figure 12
   Overview of the sequences used or referred to in this application.
Figure 13
   Generation of Rosa26-IgVk1-39 KI allele. (A) Schematic drawing of the pCAGGS-IgVK1-39 targeting vector. (B) Nucleotide sequence of the pCAGGS-IgVK1-39 targeting vector. (C) Targeting strategy.
Figure 14
   (A) Southern blot analysis of genomic DNA of ES clones comprising an insertion of the pCAGGS-IgVK1-39 targeting vector. Genomic DNA of 4 independent clones was digested with AseI and probed with 5e1 indicating the 5'-border of the targeting vector. All clones comprise a correct insertion of the targeting vector at the 5' end.
   (B) Southern blot analysis of genomic DNA of ES clones comprising an insertion of the pCAGGS-IgVK1-39 targeting vector. Genomic DNA of 4 independent clones was digested with MscI and probed with 3e1 indicating the 3'-border of the targeting vector. All clones comprise a correct insertion of the targeting vector at the 3' end.
   (C) Southern blot analysis of genomic DNA of ES clones comprising an insertion of the pCAGGS-IgVK1-39 targeting vector. Genomic DNA of 4 independent clones was digested with BamHI and probed with an internal Neo probe indicating the 5'-border of the targeting vector. All clones comprise a correct, single insertion of the targeting vector.
Figure 15
   Generation of Rosa26-IgVI2-14 KI allele. (A) Schematic drawing of the pCAGGS-IgVL2-14 targeting vector. (B) Nucleotide sequence of the pCAGGS-IgVL2-14 targeting vector containing the CAGGS expression insert based on the rearranged germline IGLV2-14/J V lambda region (IGLV2-14/J-Ck). (C) Targeting strategy.
Figure 16
   Epibase^{®} profile of IGKV1-39 residues 1-107. Sub-figure A displays the binding strength for DRB1 allotypes, while C displays the binding strength for DRB3/4/5, DQ and DP allotypes. The values in the figure represent dissociation constants (Kd's) and are plotted on a logarithmic scale in the range 0.01 µM - 0.1 pM (very strong binders may have run off the plot). For medium binding peptides, qualitative values are given only, and weak and non-binders are not shown. Values are plotted on the first residue of the peptide in the target sequence (the peptide itself extends by another 9 residues). Importantly, only the strongest binding receptor for each peptide is shown: cross-reacting allotypes with lower affinity are not visible in this plot. The strongest binding receptor is indicated by its serotypic name. Finally, any germline-filtered peptides are plotted with a lighter color in the epitope map (in this case, no non-self epitopes were found). Sub-figure B shows the HLA binding promiscuity for every decameric peptide (Y-axis: the number of HLA allotypes recognizing critical epitopes in each of the peptides starting at the indicated residue shown on the X-axis). The promiscuity is measured as the number of allotypes out of the total of 47 for which the peptide is a critical binder. White columns refer to self-peptides, and black columns (absent here) to non-self peptides.
Figure 17
   Epitope map of IGKV1-39 showing the presence of peptide binders predicted in the sequence of IGKV1-39 by serotype in the 15-mer format. Each 15-mer is numbered as indicated in the top of the figure. The full sequence of the corresponding 15-mer is listed in Table 7. Black boxes indicate the presence of one or more critical self-epitopes in the 15-mer for the serotype listed on the left. Critical epitopes are operationally defined as strong or medium DRB1 binders and strong DRB3/4/5 or DP or DQ binders.
Figure 18
   Constitutive knock-out (KO) of the Ig kappa locus. (A) Targeting strategy. (B) Schematic drawing of the pIgKappa targeting vector.
Figure 19
   Constitutive KO of the Ig lambda locus. (A) First step of the targeting strategy. (B) Second step of the targeting strategy.
Figure 20
   Schematic drawing of targeting vectors. (A) pVkP-O12 (VkP-IGKV1-39/J-Ck); (B) pVkP-O12-del1 (VKP-IGKV1-39/J-Ck-Δ1); (C) pVkP-O12-del2 (VkP-IGKV1-39/J-Ck-Δ2).
Figure 21
   Targeting strategies for insertion of transgene into the Rosa26 locus by targeted transgenesis using RMCE. (A) VkP-O12 (VkP-IGKV1-39/J-Ck); (B) VkP-O12-del1 (VkP-IGKV1-39/J-Ck-Δ1); (C) VkP-O12-del2 (VkP-IGKV1-39/J-Ck-Δ2).
Figure 22
   Topology of the MV1057 vector. Replacing the indicated stuffer fragment with a VH fragment yields an expression vector that can be transfected to eukaryotic cells for the production of IgG1 antibodies with light chains containing an 012 (IGKV1-39) VL gene.
Figure 23
   Lack of transgenic human Vk1 light chain expression in non-B cell populations of the spleen.
Figure 24
   Transgenic human Vk1 light chain is expressed in all B cell populations of the spleen.
Figure 25
   Transgenic human Vk1 light chain is expressed in B1 cells of the peritoneal cavity.
Figure 26
   Transgenic human Vk1 light chain is not expressed in pro- and pre-B cells but in the immature and recirculating populations B cells in the bone marrow. (A) Gating of bone marrow cells. (B) Histograms of transgene expression with overlay from one WT control.
Figure 27
   Transgenic human Vk1 light chain is directly correlated with endogenous light chain and IgM expression in circulating B cells in the blood.

### Examples.

### Example 1

### Human light chain V-gene clones

This example describes the rationale behind the choice of two human light chain V- genes, one gene of the kappa type and one gene of the lambda type, that are used as a proof of concept for light chain expressing transgenic mice. De Wildt et al. 1999 (de Wildt et al. (1999) J. Mol. Biol. 285(3):895) analysed the expression of human light chains in peripheral IgG-positive B-cells. Based on these data, IGKV1-39 (012) and IGLV2-14 (2a2) were chosen as light chains as they were well represented in the B-cell repertoire. The J-segment sequence of the light chains has been chosen based upon sequences as presented in GenBank ABA26122 for IGKV1-39 (Rabquer,B.J., Smithson,S.L., Shriner,A.K. and Westerink,M.A.J.) and GenBank AAF20450 for IGLV2-14 (Ignatovich,O., Tomlinson,I.M., Popov,A.V., Bruggemann,M. and Winter,G. J. Mol. Biol. 294 (2), 457-465 (1999)).

All framework segments are converted into germline amino acid sequences to provide the lowest immunogenicity possible in potential clinical applications.

### Example 2

Obtaining mouse heavy chain V-genes that pair with human IGKV1-39 gene segment to form functional antibody binding sites

This example describes the identification of mouse heavy chain V-genes that are capable of pairing with a single, rearranged human germline IGKV1-39/J region. A spleen VH repertoire from mice that were immunized with tetanus toxoid was cloned in a phage display Fab vector with a single human IGKV1-39-C kappa light chain and subjected to panning against tetanus toxoid. Clones obtained after a single round of panning were analyzed for their binding specificity. The murine VH genes encoding tetanus toxoid-specific Fab fragments were subjected to sequence analysis to identify unique clones and assign VH, DH and JH utilization.

Many of the protocols described here are standard protocols for the construction of phage display libraries and the panning of phages for binding to an antigen of interest and described in Antibody Phage Display: Methods and Protocols (editor(s): Philippa M. O'Brien, Robert Aitken).

### Immunizations

BALB/c mice received one immunization with tetanus toxoid and were boosted after 6 weeks with tetanus toxoid.

### Splenocyte isolation

Preparation of spleen cell suspension. After dissection, the spleen was washed with PBS and transferred to a 60 mm petridish with 20 ml PBS. A syringe capped with 20 ml PBS and a G20 needle was used to repeatedly flush the spleen. After washing the flushed cells with PBS, the cells were carefully brought into suspension using 20 ml PBS and left on a bench for 5 minutes to separate the splenocytes from the debris and cell clusters. The splenocytes suspension was transferred on top of a Ficoll-Paque^{™} PLUS-filled tube and processed according to the manufacturer's procedures for lymphocyte isolation (Amersham Biosciences).

### RNA isolation and cDNA synthesis

After isolation and pelleting of lymphocytes, the cells were suspended in TRIzol LS Reagent (Invitrogen) for the isolation of total RNA according to the accompanying manufacturer's protocol and subjected to reverse transcription reaction using 1 microgram of RNA, Superscript III RT in combination with dT20 according to manufacturer' a procedures (Invitrogen).

### PCR amplification of cDNA

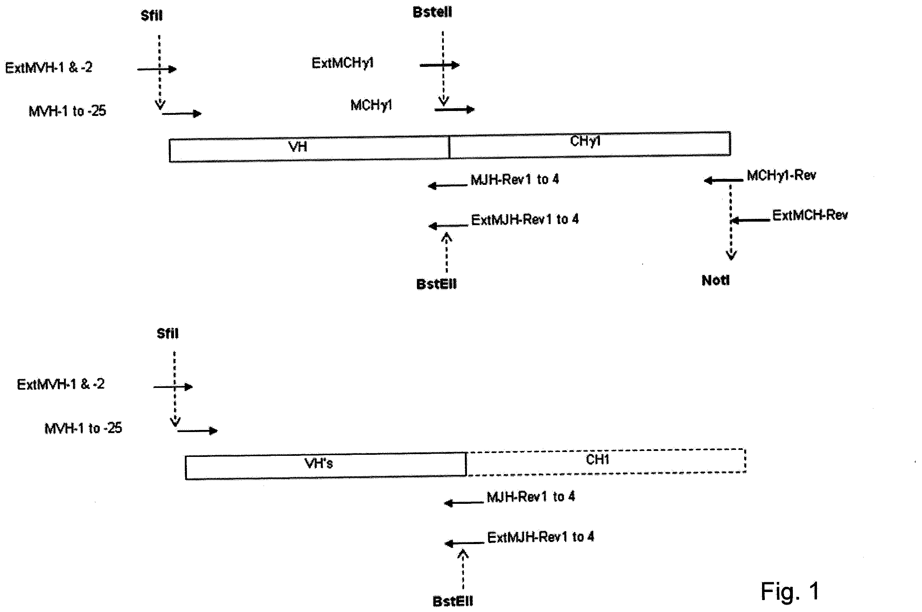

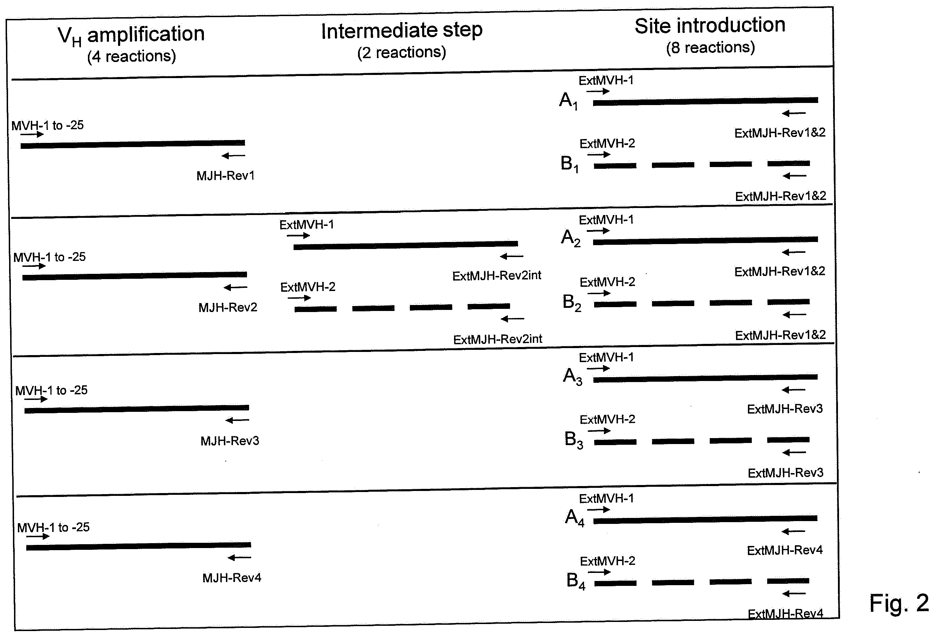

The cDNA was amplified in a PCR reaction using primer combinations that allow the amplification of approximately 110 different murine V-genes belonging to 15 VH families (table 1; RefSeq NG_005838; Thiebe et al., 1999. European Journal of Immunology 29: 2072 - 2081). In the first round, primer combinations that bind to the 5' end of the V-genes and 3' end of the J regions were used. In the second round, PCR products that were generated with the MJH-Rev2 primer were amplified in order to introduce modifications in the 3' region to enable efficient cloning of the products. In the last round of amplification, all PCR products were amplified using primers that introduce a SfiI restriction site at the 5' end and a BstEII restriction site at the 3' end (see figures 1 and 2, and table 1).

Reaction conditions for 1st round PCR: 4 different reactions combining all 25 forward primers (MVH1 to MVH25, table 1 and figure 2) and 1 reverse primer per reaction (MJH-Rev1, MJH-Rev2, MJH-Rev3 or MJH-Rev4; see table 1 and figure 2). 50 microliter PCR volumes were composed of 2 microliter cDNA (from RT reactions), 10 microliter 5* Phusion polymerase HF buffer, 40 nM of each of the 25 forward primers (total concentration of 1 micromolar), 1 micromolar reverse primer, 1 microliter 10 mM dNTP stock, 1.25 unit Phusion polymerase and sterile MQ water. The thermocycler program consisted of a touch down program: 1 cycle 98°C for 30 seconds, 30 cycles 98°C for 10 seconds, 58°C decreasing 0.2°C per cycle 10 seconds, 72°C 20 seconds and 1 cycle 72°C for 3 minutes. The second round PCR program was set up only for the products of the 1st PCR that contain the MJH-Rev2 primer: 2 different reactions combining either the ExtMVH-1 or ExtMVH-2 primers (table 1 and figure 2) in combination with the reverse primer ExtMJH-Rev2int (table 1 and figure 2). 50 microliter PCR volumes were composed of 50 ng PCR product (from first PCR round), 10 microliter 5* Phusion polymerase HF buffer, 500 nM of each forward primer, 1 micromolar reverse primer, 1 microliter 10 mM dNTP stock, 1.25 unit Phusion polymerase and sterile MQ water. The thermocycler program consisted of a touch down program followed by a regular amplification step: 1 cycle 98°C for 30 seconds, 10 cycles 98°C for 10 seconds, 65°C decreasing 1.5°C per cycle 10 seconds, 72°C 20 seconds, 10 cycles 98°C for 10 seconds, 55°C 10 seconds, 72°C 20 seconds and 1 cycle 72°C for 3 minutes. The third round PCR program was setup as described in figure 2. 50 microliter PCR volumes were composed of 50 ng PCR product (from earlier PCR rounds, figure 2), 10 microliter 5* Phusion polymerase HF buffer, 1 micromolar forward primer (table 1 and figure 2), 1 micromolar reverse primer, 1 microliter 10 mM dNTP stock, 1.25 unit Phusion polymerase and sterile MQ water. The program consists of a touch down program followed by a regular amplification step: 1 cycle 98°C for 30 seconds, 10 cycles 98°C for 10 seconds, 65°C decreasing 1.5°C per cycle 10 seconds, 72°C 20 seconds, 10 cycles 98°C for 10 seconds, 55°C 10 seconds, 72°C 20 seconds and 1 cycle 72°C for 3 minutes. After PCR amplifications, all PCR products were gel purified using Qiaex II according to the manufacturer's protocols.

### Restriction enzyme digestions

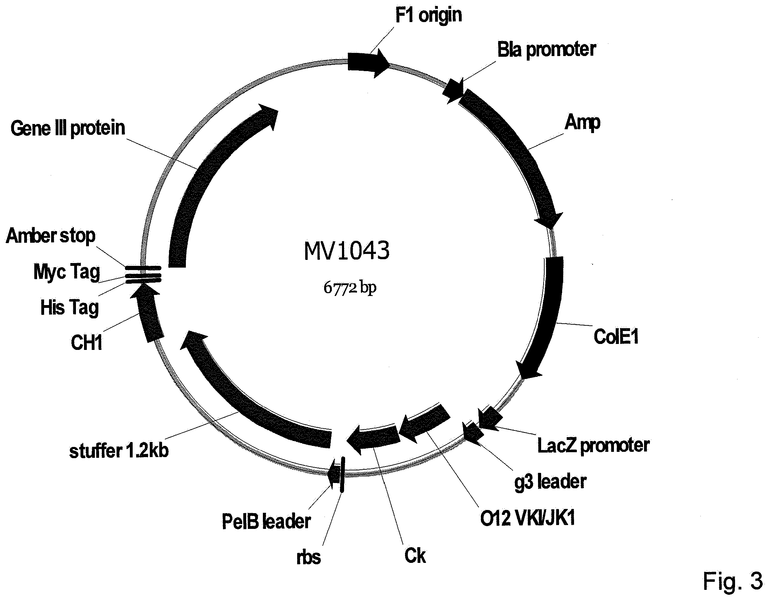

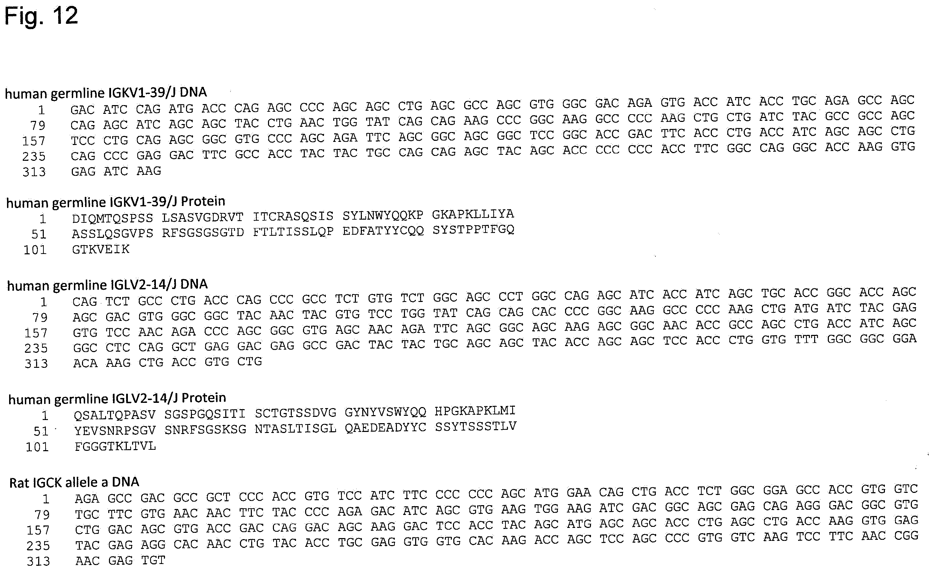

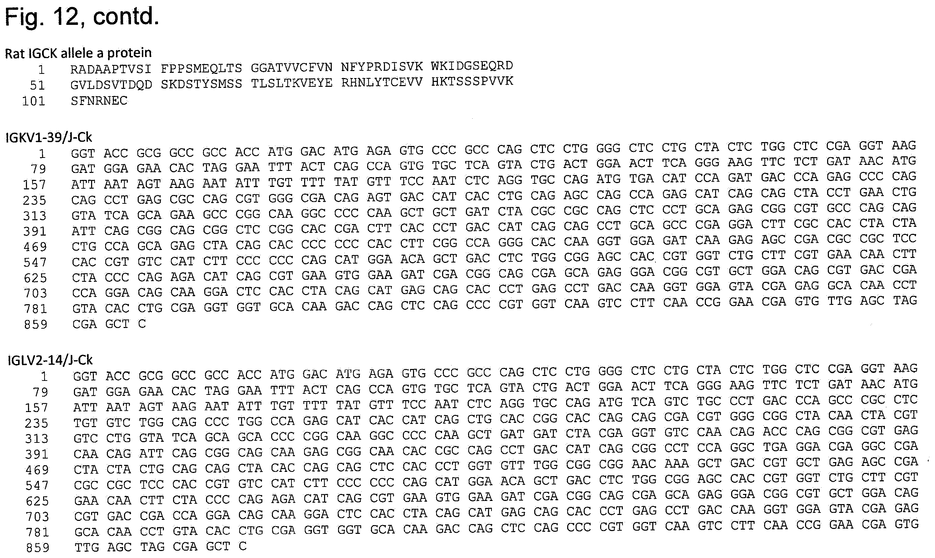

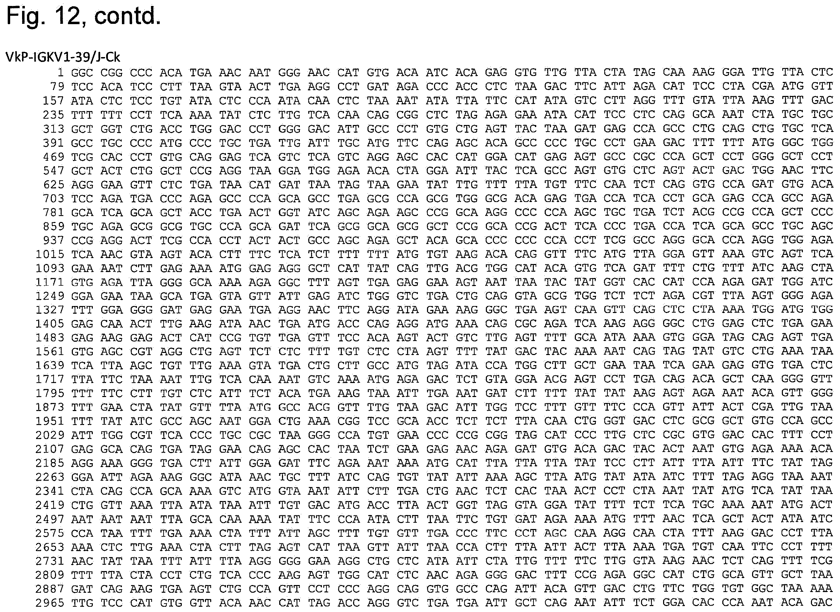

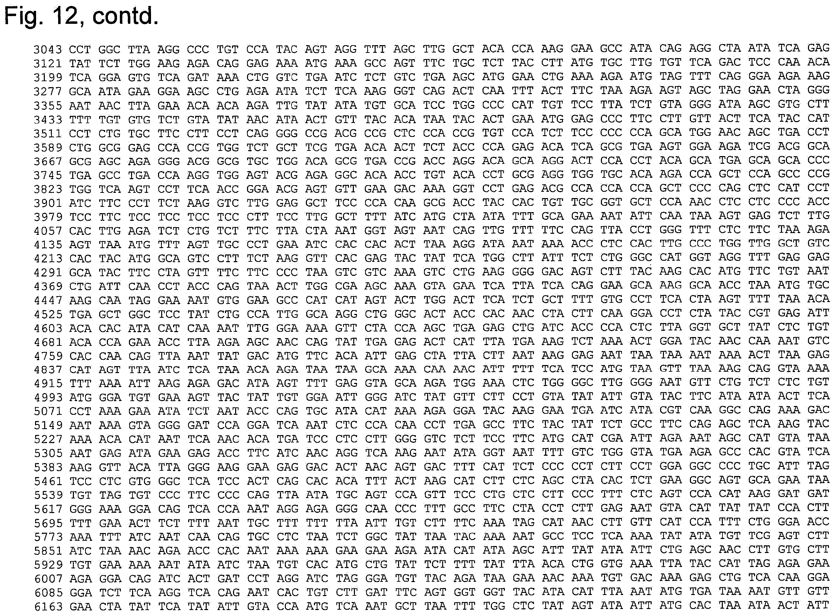

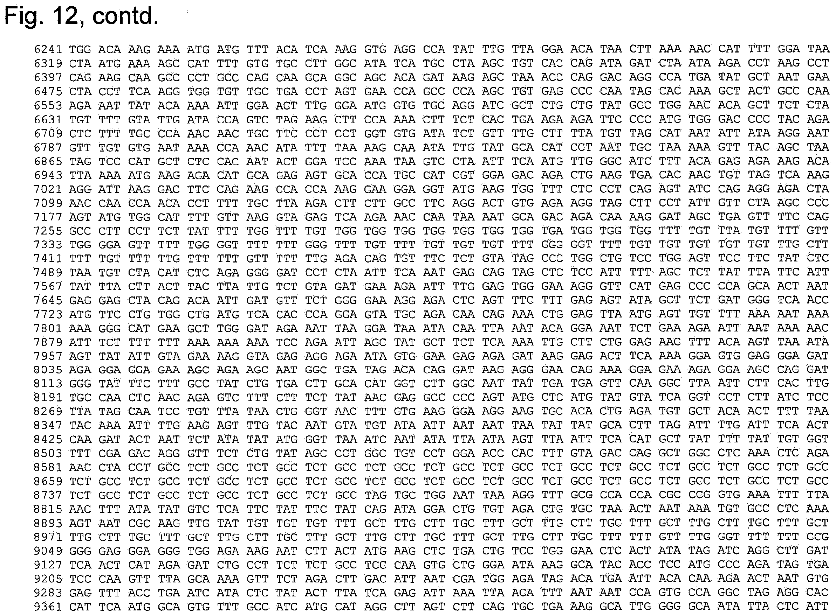

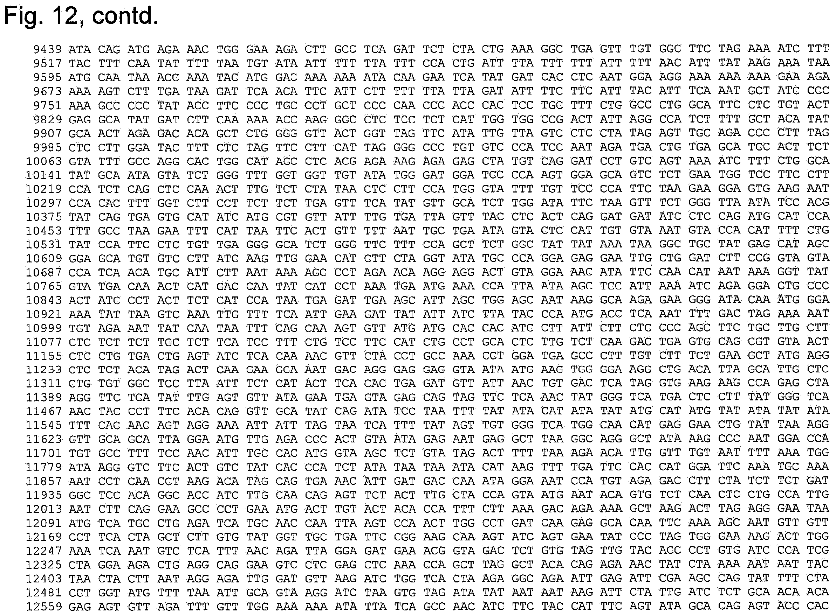

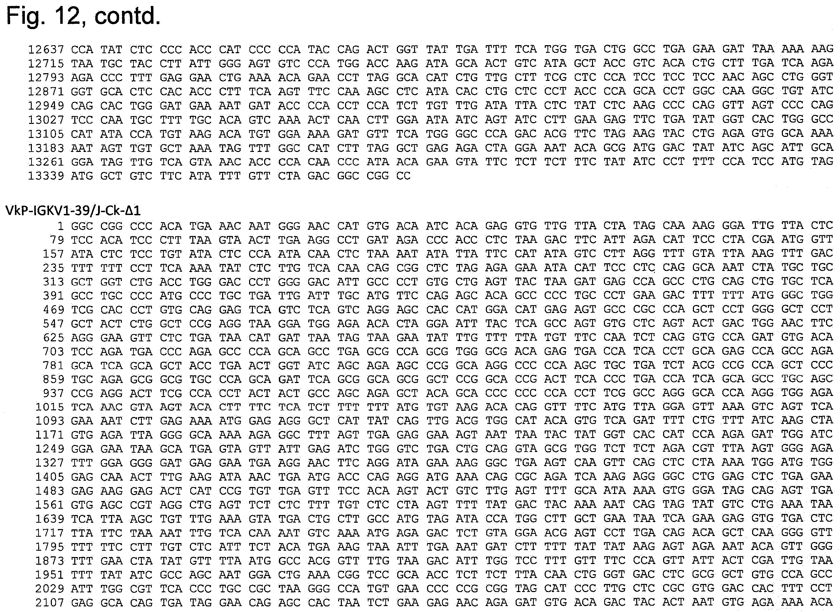

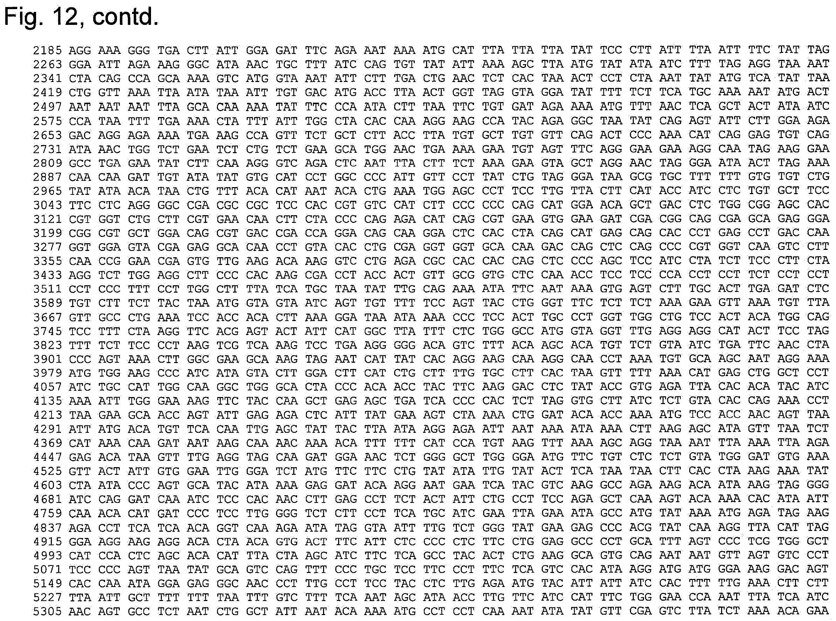

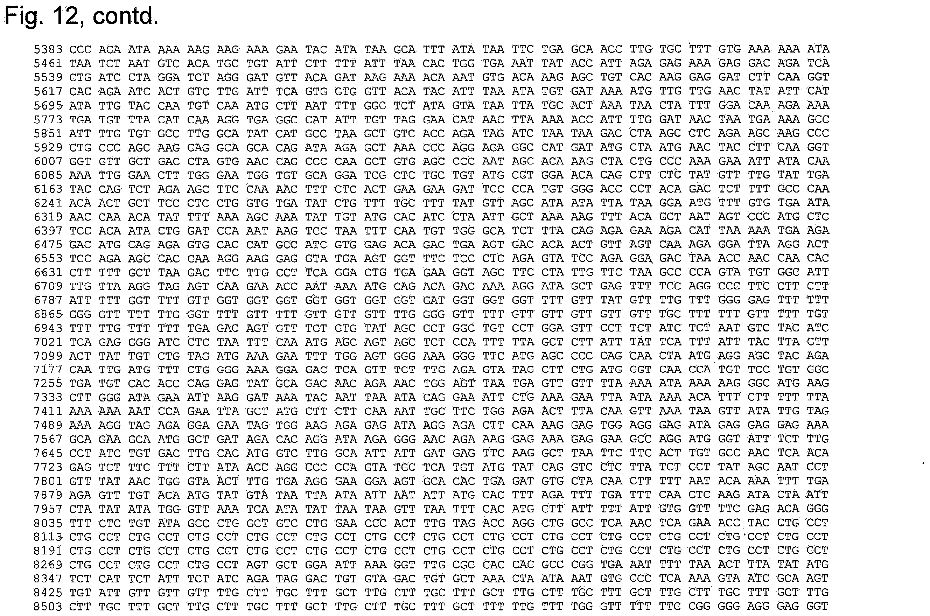

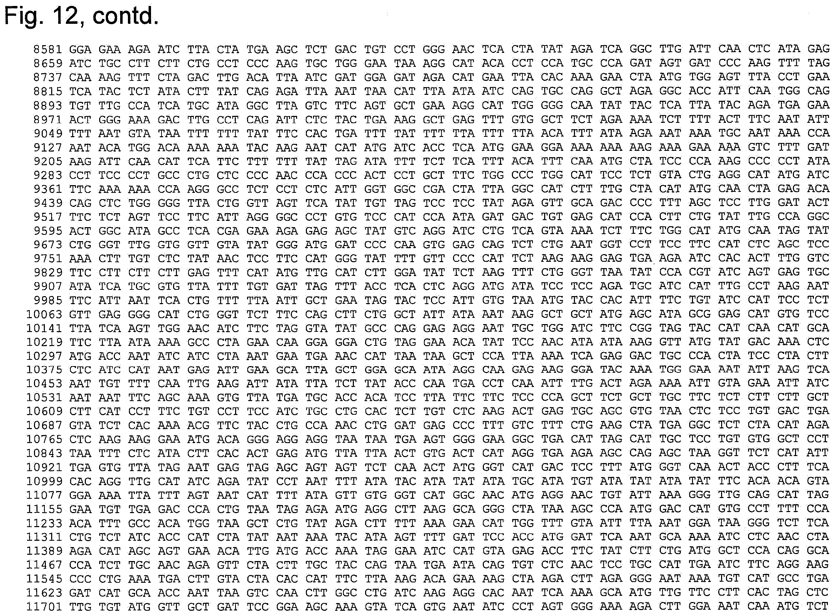

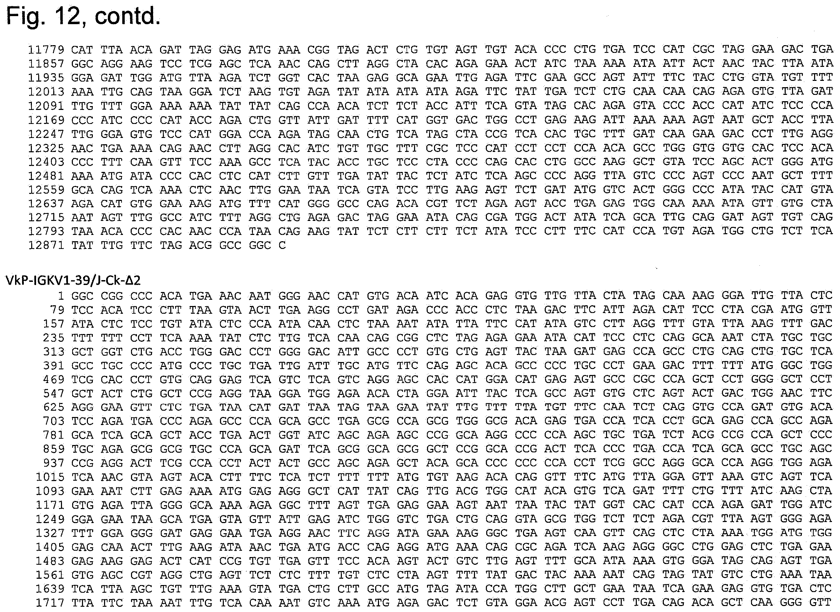

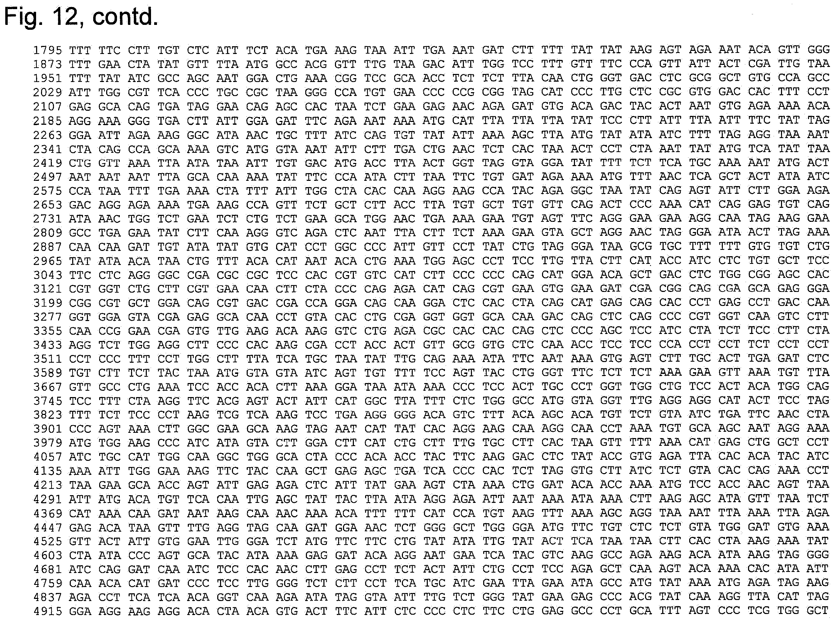

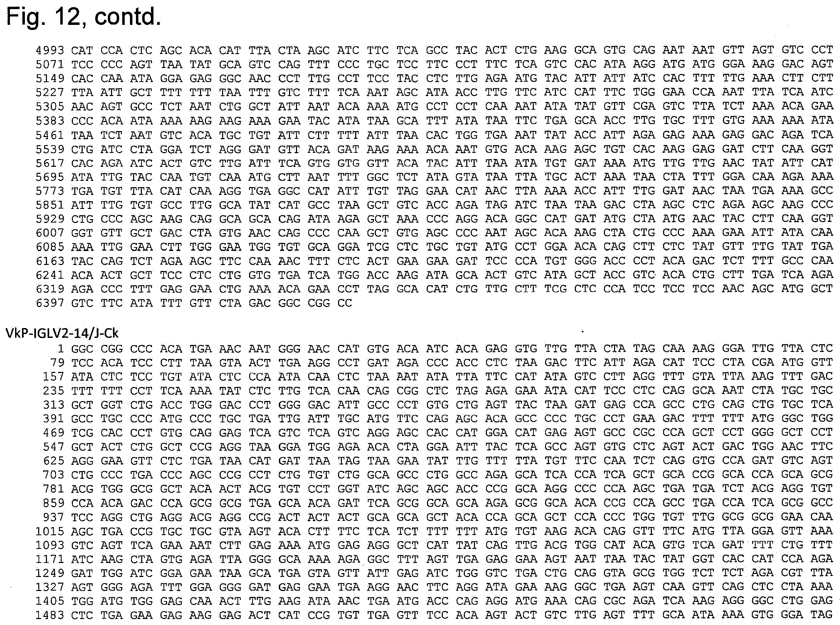

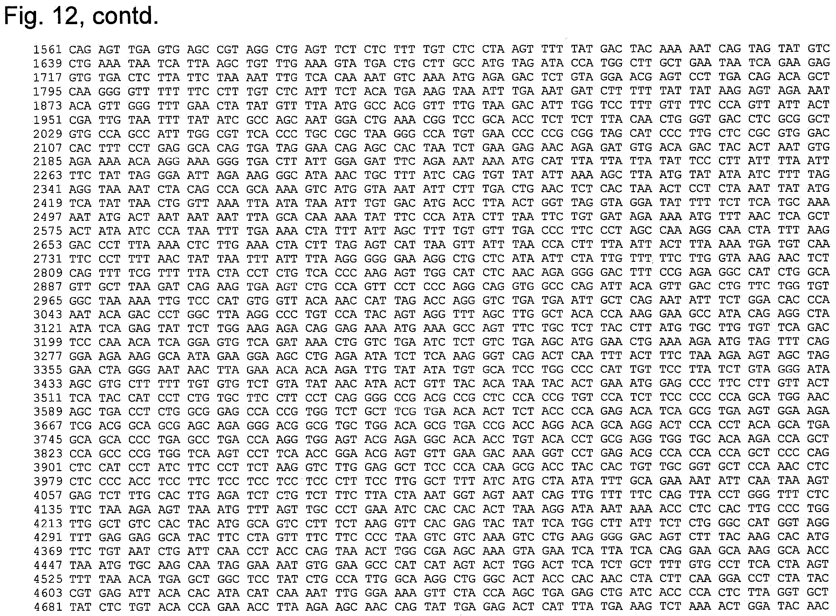

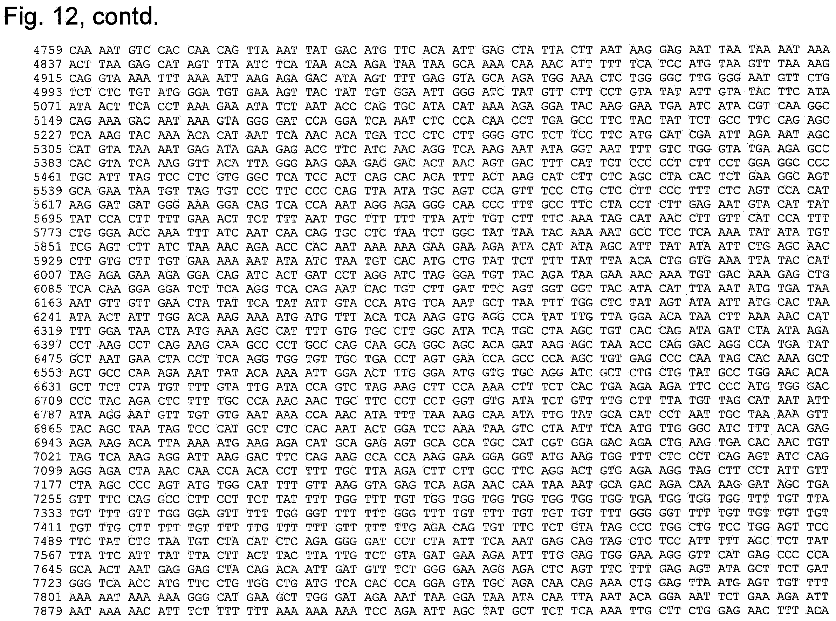

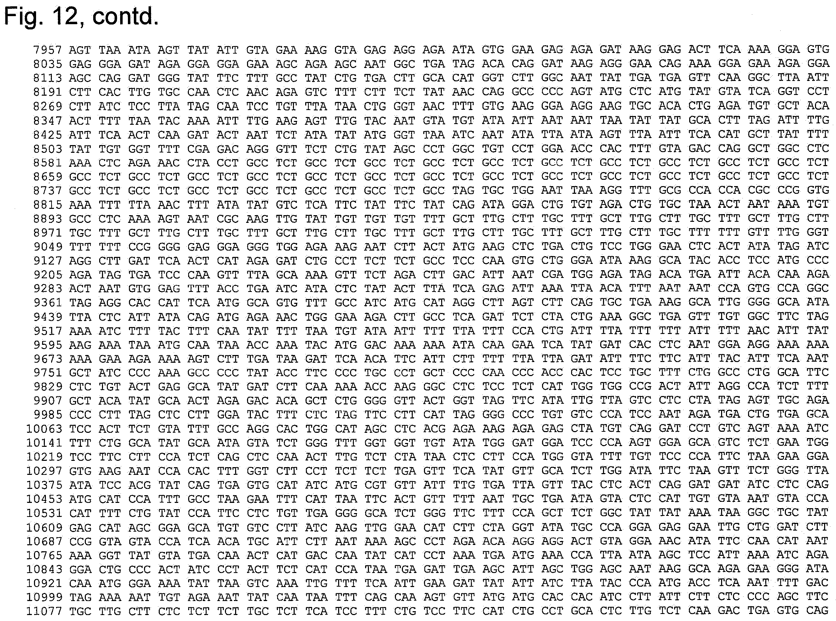

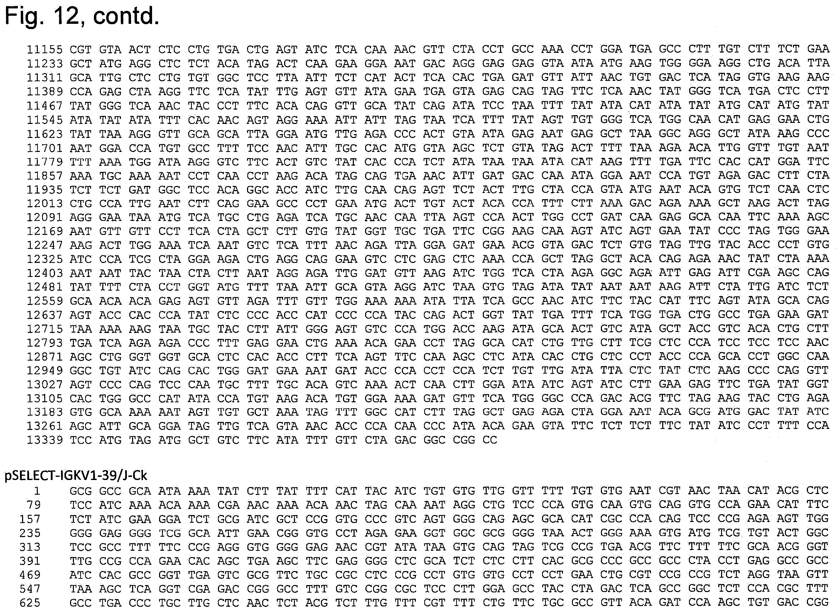

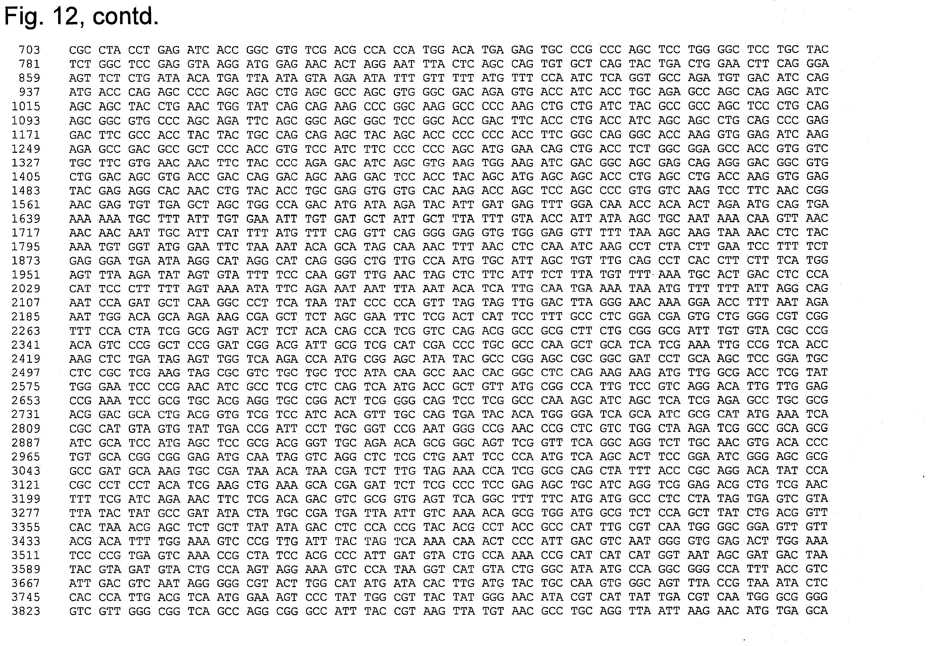

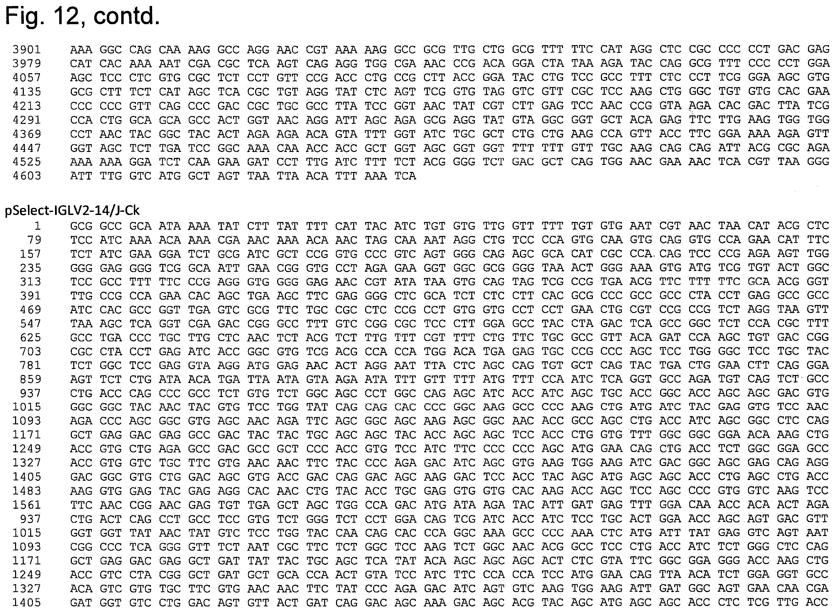

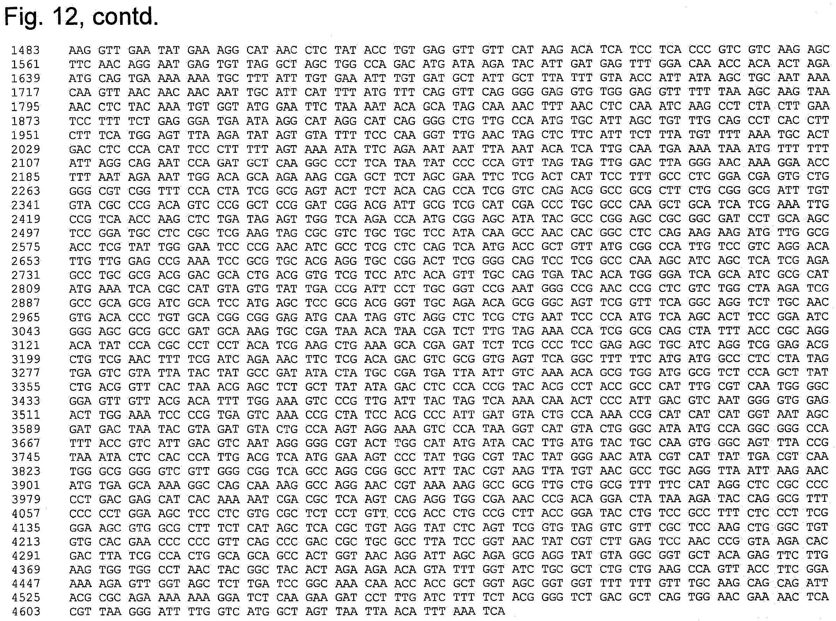

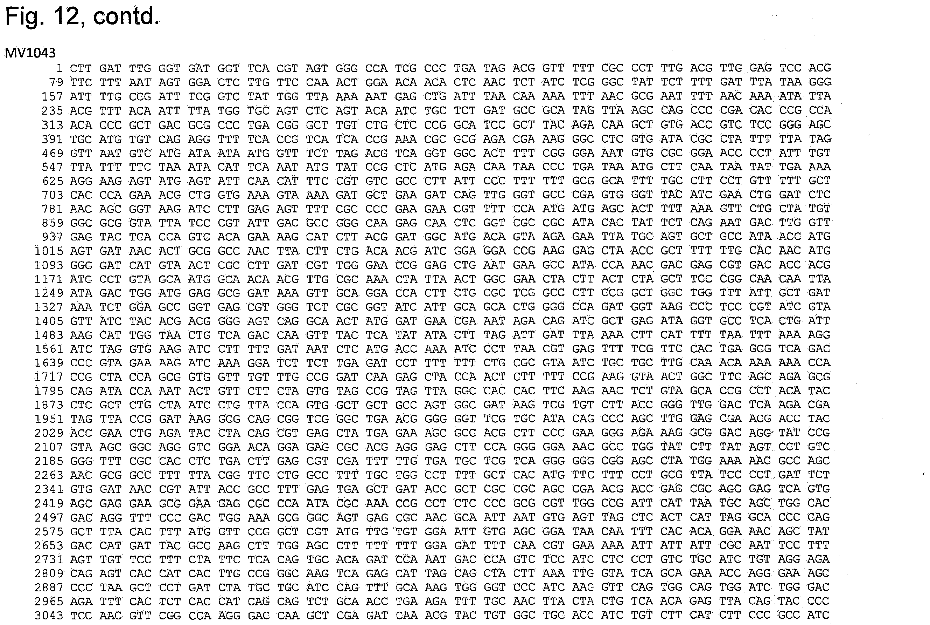

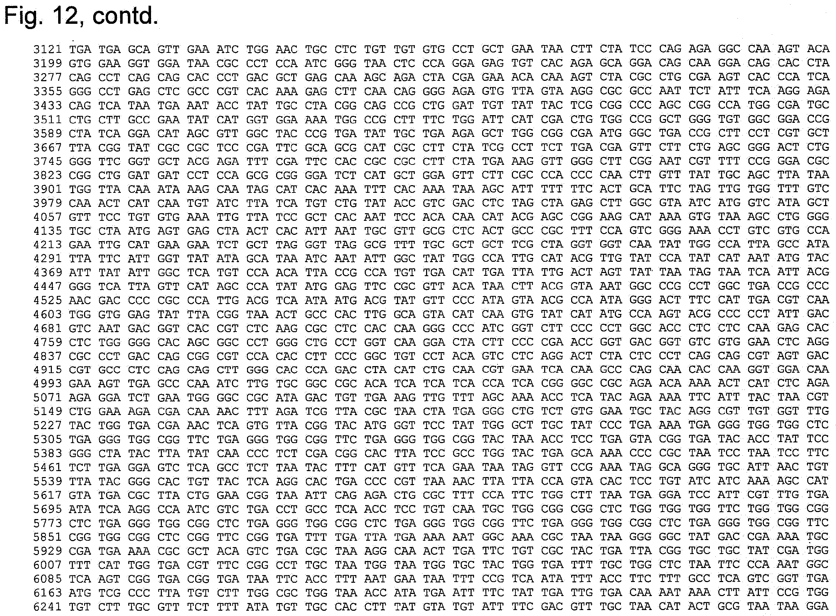

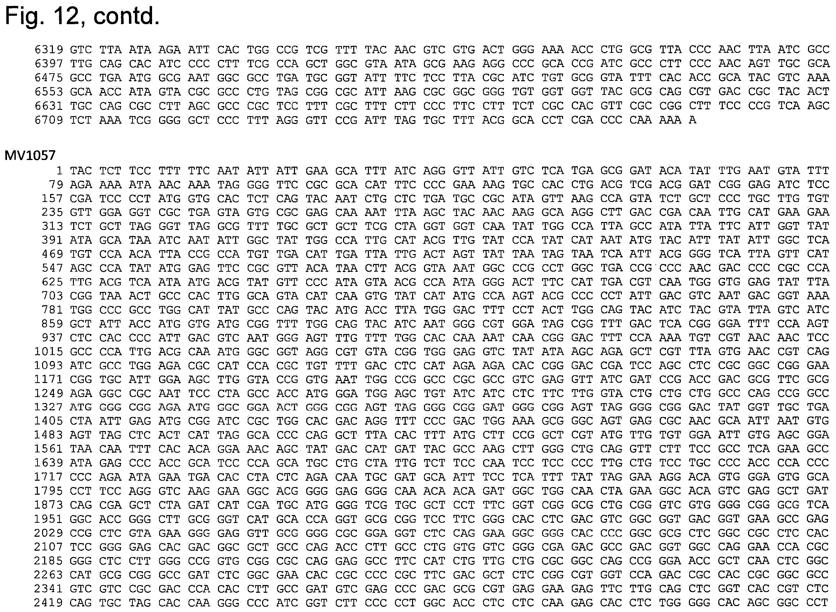

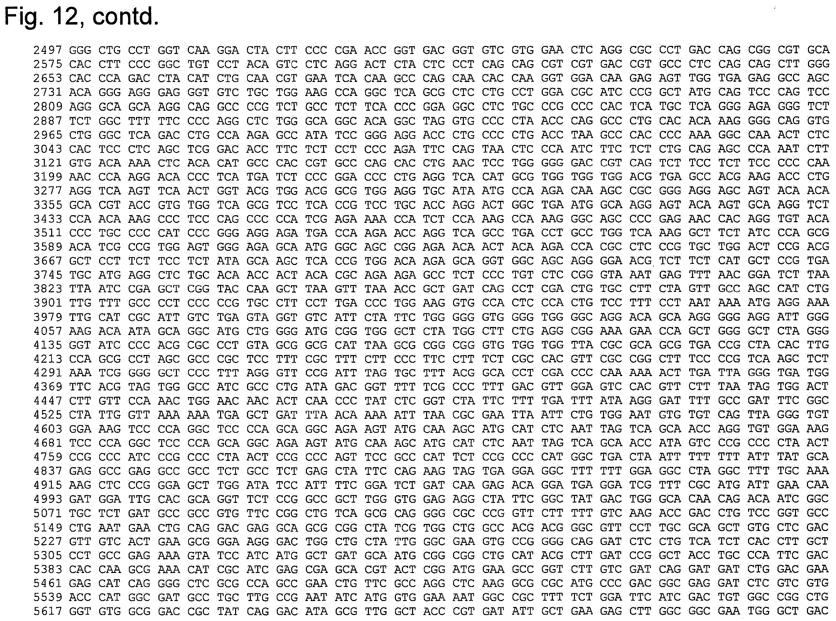

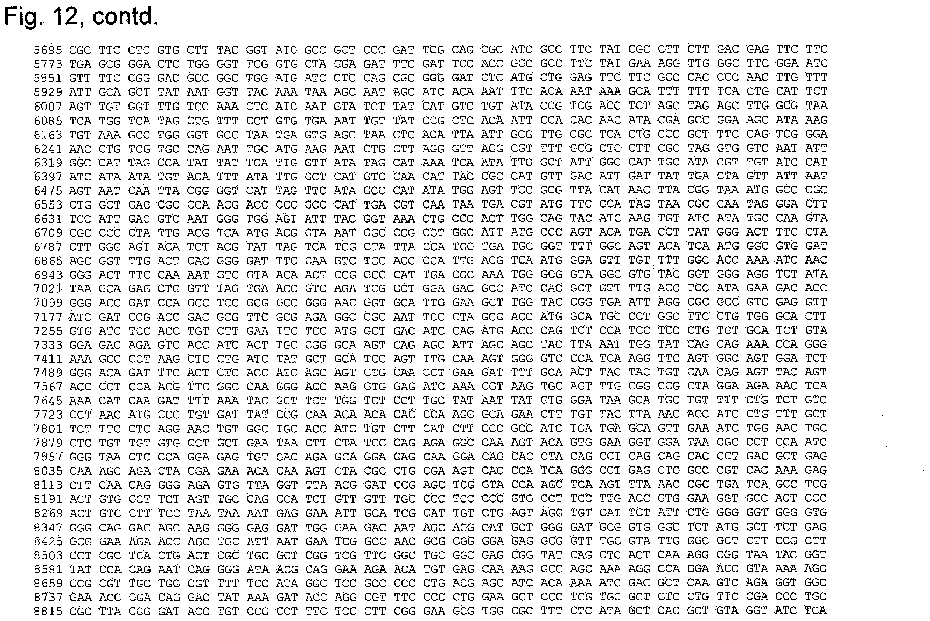

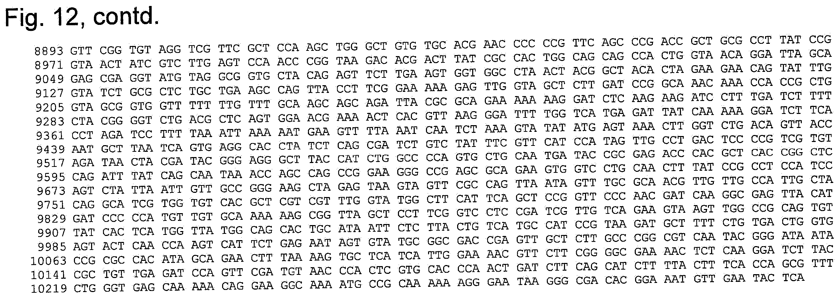

Purified products were digested with BstEII and SfiI in two steps. First 1 microgram of DNA was digested in 100 microliter reactions consisting of 10 microliters of 10* NEB buffer 3 (New England Biolabs), 1 microliter 100* BSA, 12.5 unit BstEII and sterile water for 6 hours at 60°C in a stove. The products were purified using Qiaquick PCR Purification kit from Qiagen according to the manual instructions and eluted in 40 microliter water. Next all products were further digested with SfiI in 100 microliter reactions consisting of 10 microliters of 10* NEB buffer 2 (New England Biolabs), 1 microliter 100* BSA, 12.5 unit SfiI and sterile water for 12 hours at 50°C in a stove. The digested fragments were purified by Qiaquick Gel Extraction kit following gel separation on a 20 cm 1.5% agarose TBE plus ethidium bromide gel at 80 V. 100 microgram of the acceptor vector (MV1043, figures 3 and 12) was digested with 50 units Eco91I in 600 microliter under standard conditions (Tango buffer) and next purified on a 0.9% agarose gel. After a second digestion step under prescribed conditions with 400 units SfiI in 500 microliter for 12 hours, 100 units BsrGI were added for 3 hours at 50°C.

### Ligations

Each PCR product was ligated separately according to the following scheme: 70 ng digested PCR products, 300 ng digested acceptor vector, 100 units T4 Ligase (NEB), 1* ligase buffer in 30 microliters for 16 hours at 12°C. The ligation reactions were purified with phenol/chloroform/isoamyl alcohol extractions followed by glycogen precipitations (Sigma-Aldrich #G1767) according to the manufacturer' s protocol and finally dissolved in 25 microliter sterile water.

### Transformations and library storage

The purified ligation products were transformed by electroporation using 1200 microliter TG1 electrocompetent bacteria (Stratagene #200123) per ligation batch and plated on LB carbenicillin plates containing 4% glucose. Libraries were harvested by scraping the bacteria in 50 ml LB carbenicillin. After centrifugation at 2000g for 20 minutes at 4°C, the bacterial pellets were resuspended carefully in 2 ml ice cold 2*TY/30% glycerol on ice water and frozen on dry-ice/ethanol before storage at -80°C.

### Library amplification

Libraries were grown and harvested according to procedures as described by Kramer et al. 2003 (Kramer et al. 2003. Nucleic Acids Res. 31(11): e59) using VCSM13 (Stratagene) as helper phage strain.

### Selection of phages on coated immunotubes

Tetanus toxoid was dissolved in PBS in a concentration of 2 µg/ml and coated to MaxiSorp Nunc-Immuno Tube (Nunc 444474) overnight at 4°C. After discarding the coating solution, the tubes were blocked with 2% skim milk (ELK) in PBS (blocking buffer) for 1 hour at RT. In parallel, 0.5 ml of the phage library was mixed with 1 ml blocking buffer and incubated for 20 minutes at room temperature. After blocking the phages, the phage solution was added to the tetanus toxoid coated tubes and incubated for 2 hours at RT on a slowly rotating platform to allow binding. Next, the tubes were washed 10 times with PBS/0.05% Tween-20 followed by phage elution by an incubation with 1 ml 50 mM glycine-HCl pH 2.2 10 min at RT on rotating wheel and directly followed by neutralization of the harvested eluent with 0.5 ml 1 M Tris-HCl pH 7.5.

### Harvesting phage clones

5 ml XL1-Blue MRF (Stratagene) culture at O.D. 0.4 was added to the harvested phage solution and incubated for 30 minutes at 37°C without shaking to allow infection of the phages. Bacteria were plated on Carbenicillin/Tetracycline 4% glucose 2*TY plates and grown overnight at 37°C.

### Phage production

Phages were grown and processed as described by Kramer et al. 2003 (Kramer et al. 2003. Nucleic Acids Res. 31(11): e59) using VCSM13 as helper phage strain.

### Phage ELISA

ELISA plates were coated with 100 microliter tetanus toxoid per well at a concentration of 2 microgram/ml in PBS overnight at 4°C. Plates coated with 100 microliter thyroglobulin at a concentration of 2 microgram/ml in PBS were used as a negative control. Wells were emptied, dried by tapping on a paper towel, filled completely with PBS-4% skimmed milk (ELK) and incubated for 1 hour at room temperature to block the wells. After discarding the block solution, phage minipreps pre-mixed with 50 µl blocking solution were added and incubated for 1 hour at RT. Next 5 washing steps with PBS-0.05% Tween-20 removed unbound phages. Bound phages were detected by incubating the wells with 100 microliter anti-M13-HRP antibody conjugate (diluted 1/5000 in blocking buffer) for 1 hour at room temperature. Free antibody was removed by repeating the washing steps as described above, followed by TMB substrate incubation until color development was visible. The reaction was stopped by adding 100 microliter of 2 M H₂SO₄ per well and analyzed on an ELISA reader at 450 nm emission wavelength (Table 2). Higher numbers indicate stronger signals and thus higher incidence of specific binding of the phage-Fab complex.

### Sequencing

Clones that gave signals at least 3 times above the background signal (Table 2) were propagated, used for DNA miniprep procedures (see procedures Qiagen miniPrep manual) and subjected to nucleotide sequence analysis. Sequencing was performed according to the Big Dye 1.1 kit accompanying manual (Applied Biosystems) using a reverse primer (CH1_Rev1, table 1) recognizing a 5' sequence of the CH1 region of the human IgG1 heavy chain (present in the Fab display vector MV1043, figures 3 and 12). Mouse VH sequences of 28 tetanus toxoid binding clones are depicted in Table 3. The results show that the selected murine VH genes belong to different gene families, and different individual members from these gene families are able to pair with the rearranged human IGKV1-39/J VH region to form functional tetanus toxoid-specific antibody binding sites. From the sequence analyses, it was concluded that the murine VH regions utilize a diversity of DH and JH gene segments.

### Example 3

### Silencing of the mouse kappa light chain locus

This example describes the silencing of the mouse endogenous kappa light chain locus. The endogenous kappa locus is modified by homologous recombination in ES cells, followed by the introduction of genetically modified ES cells in mouse embryos to obtain genetically adapted offspring.

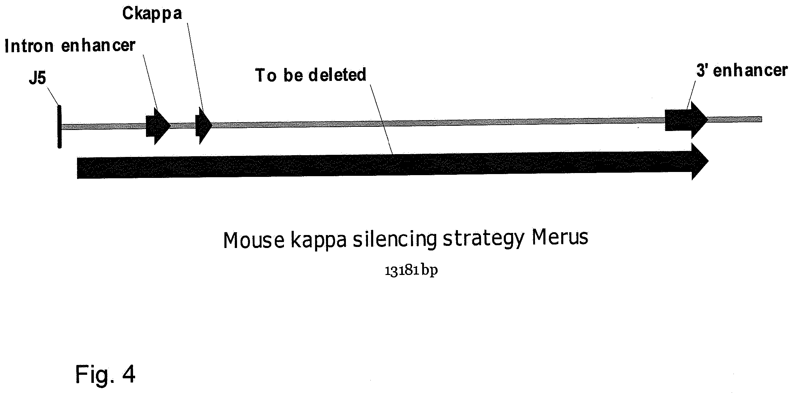

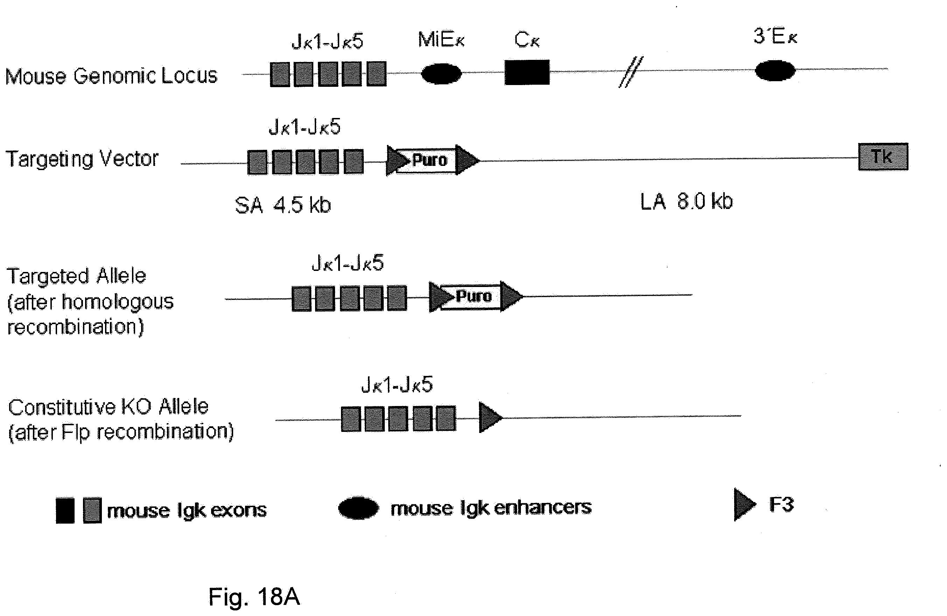

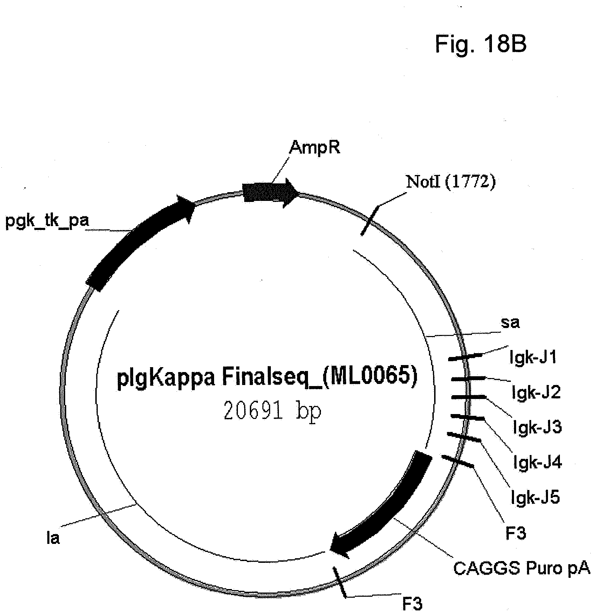

A vector that contains an assembled nucleotide sequence consisting of a part comprising the J-region to 338 bp downstream of the J5 gene segment fused to a sequence ending 3' of the 3' CK enhancer is used for homologous recombination in ES cells. The assembled sequence is used to delete a genomic DNA fragment spanning from 3' of the JK region to just 3' of the 3' CK enhancer. As a consequence of this procedure, the CK constant gene, the 3' enhancer and some intergenic regions are removed (see figures 4 and 18).

### Construction of the targeting vector

A vector that received 4.5-8 kb flanking arms on the 3' and 5' end fused to the deletion segment was used for targeted homologous recombination in an ES cell line. Both arms were obtained by PCR means ensuring maximum homology. The targeting strategy allows generation of constitutive KO allele. The mouse genomic sequence encompassing the Igk intronic enhancer, Igk constant region and the Igk 3' enhancer was replaced with a PuroR cassette, which was flanked by F3 sites and inserted downstream of the Jk elements. Flp-mediated removal of the selection marker resulted in a constitutive KO allele. The replacement of the Igk MiEk-Igk C-Igk 3'E genomic region (approx. 10 kb) with a F3-Puro cassette (approx. 3 kb) was likely to decrease the efficiency of homologous recombination. Therefore, the arms of homology were extended accordingly and more ES cell colonies were analysed after transfection in order to identify homologous recombinant clones.

### Generation of ES cells bearing the deleted kappa fragment

The generation of genetically modified ES cells was essentially performed as described (Seibler et al. Nucleic Acids Res. 2003 Feb 15;31(4):e 12). See also example 14 for a detailed description.

### Generation of ES mice by tetraploid embryo complementation.

The production of mice by tetraploid embryo complementation using genetically modified ES cells was essentially performed as described (Eggan et al., PNAS 98, 6209-6214; Seibler J, et al. Nucleic Acids Res. 2003 Feb 15;31(4):e12; Hogan et al., (Summary of mouse development. Manipulating the Mouse Embryo, (1994) Cold Spring Harbor Laboratory Press, Cold Spring Harbor NY.), pp. 253-289.)).

### Example 4

### Silencing of the mouse lambda light chain locus

This example describes the silencing of the mouse endogenous lambda light chain locus. The endogenous lambda locus is modified by homologous recombination in ES cells followed by the introduction of genetically modified ES cells in mouse embryos to obtain genetically adapted offspring.

Two regions of the murine lambda locus that together contain all functional lambda V regions are subject to deletion.

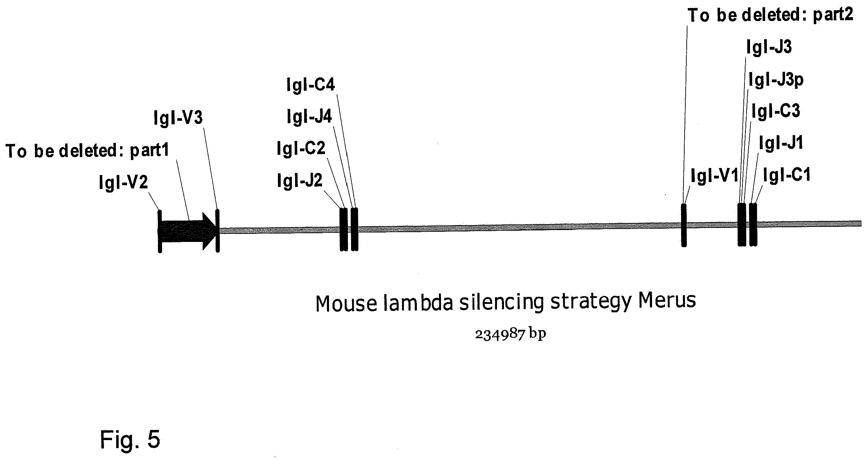

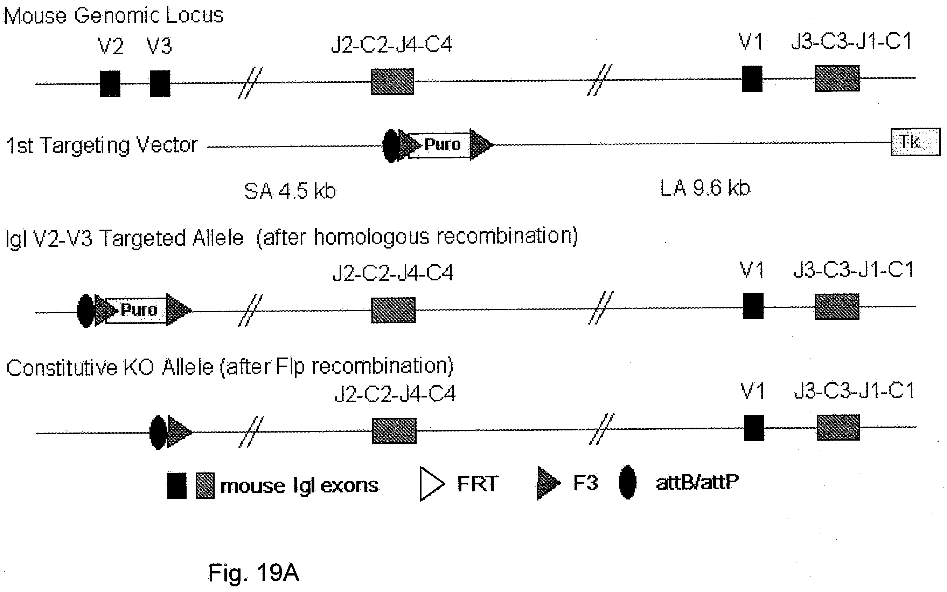

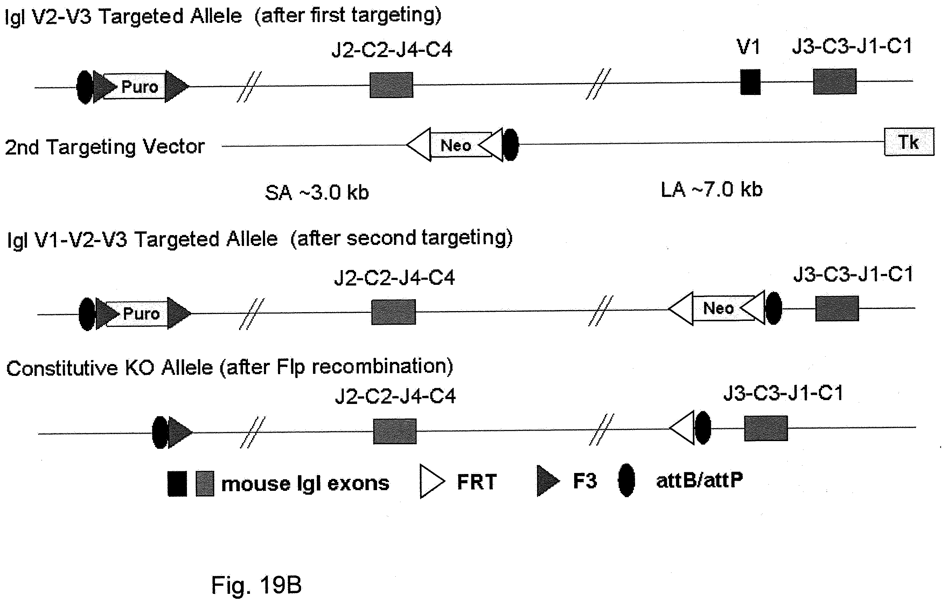

The first region targeted for homologous recombination-based deletion is a region that is located 408 bp upstream of the start site of the IGLV2 gene segment and ends 215 by downstream of IGLV3 gene segment, including the intergenic sequence stretch between these IGLV gene segments. The second region that is subject to a deletion involves the IGLV1 gene segment consisting of a fragment spanning from 392 bp upstream to 171 bp downstream of the IGLV1 gene segment. As a consequence of these two deletion steps, all functional V-lambda genes segments are deleted, rendering the locus functionally inactive (figures 5 and 19).

### Construction of the targeting vectors

Vectors that received 3-9.6 kb flanking arms on the 3' and 5' end fused to the deletion segment were used for targeted homologous recombination in an ES cell line. Both arms were obtained by PCR means ensuring maximum homology. In a first step, the mouse genomic sequence encompassing the Igl V2-V3 regions were replaced with a PuroR cassette flanked by F3 sites, which yields a constitutive KO allele after Flp-mediated removal of selection marker (see figure 19A). In a second step, the mouse genomic sequence encompassing the Igl V1 region was replaced with a Neo cassette in ES cell clones which already carried a deletion of the Igl V2-V3 regions (see figure 19B). The selection marker (NeoR) was flanked by FRT sites. A constitutive KO allele was obtained after Flp-mediated removal of selection markers.

### Generation of ES cells bearing the deleted lambda fragment

The generation of genetically modified ES cells was essentially performed as described (Seibler J, Zevnik B, Küter-Luks B, Andreas S, Kern H, Hennek T, Rode A, Heimann C, Faust N, Kauselmann G, Schoor M, Jaenisch R, Rajewsky K, Kühn R, Schwenk F. Nucleic Acids Res. 2003 Feb 15;31(4):e12). See also example 14 for a detailed description. To show that both targeting events occurred on the same chromosome several double targeted clones were selected for the in vitro deletion with pCMV C31deltaCpG. The clones were expanded under antibiotic pressure on a mitotically inactivated feeder layer comprised of mouse embryonic fibroblasts in DMEM High Glucose medium containing 20% FCS (PAN) and 1200 u/mL Leukemia Inhibitory Factor (Millipore ESG 1107). 1x10⁷ cells from each clone were electroporated with 20 µg of circular pCMV C31deltaCpG at 240 V and 500 µF and plated on four 10 cm dishes each. 2 - 3 days after electroporation cells were harvested and analysed by PCR. Primers used were:
2005_5: CCCTTTCCAATCTTTATGGG
2005_7: AGGTGGATTGGTGTCTTTTTCTC
2005_9: GTCATGTCGGCGACCCTACGCC
PCR reactions were performed in mixtures comprising 5 µl PCR Buffer 10x (Invitrogen), 2 µl MgCl₂ (50 mM), 1 µl dNTPs (10 mM), 1 µl first primer (5 pM), 1 µl second primer (5 pM), 0,4 µl Taq (5 U/ul, Invitrogen), 37,6µl H₂O, and 2 µl DNA. The program used was 95°C 5'; followed by 36 cycles of 95°C 30"; 60°C 30"; 72°C 1'; followed by 72°C 10'.

### Generation of ES mice by tetraploid embryo complementation.

The production of mice by tetraploid embryo complementation using genetically modified ES cells was essentially performed as described (Eggan et al., PNAS 98, 6209-6214; Seibler J, Zevnik B, Küter-Luks B, Andreas S, Kern H, Hennek T, Rode A, HeimannC, Faust N, Kauselmann G, Schoor M, Jaenisch R, Rajewsky K, Kühn R, Schwenk F. Nucleic Acids Res. 2003 Feb 15;31(4):e12; Hogan et al., (Cold Spring Harbor Laboratory Press, Cold Spring Harbor NY.), pp. 253-289).

### Example 5

### Construction of the CAGGS expression insert based on a rearranged human germline IGKV1-39/J-Ck gene (IGKV1-39/J-Ck)

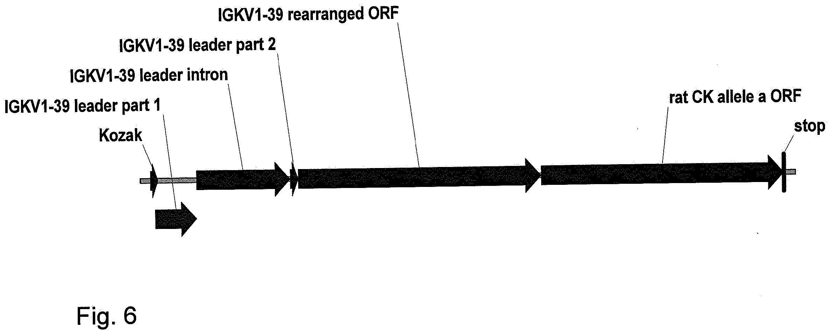

This example describes the construction of a CAGGS expression cassette incorporating the rearranged human germline IGKV1-39/J region. This insert expression cassette encompasses cloning sites, a Kozak sequence, a leader sequence containing an intron, an open reading frame of the rearranged IGKVI-39 region, a rat CK constant region from allele a and a translational stop sequence (IGKV1-39/J-Ck; figure 6). The primary construct consists of naturally occurring sequences and has been analyzed and optimized by removing undesired cis acting elements like internal TATA-boxes, poly adenylation signals, chi-sites, ribosomal entry sites, AT-rich or GC-rich sequence stretches, ARE-, INS- and CRS sequence elements, repeat sequences, RNA secondary structures, (cryptic) splice donor and acceptor sites and splice branch points (GeneArt GmbH). In addition, the codon usage in the open reading frame regions is optimized for expression in mice. The intron sequence is unchanged and thus represents the sequence identical to the coding part of the human IGKV1-39 leader intron.

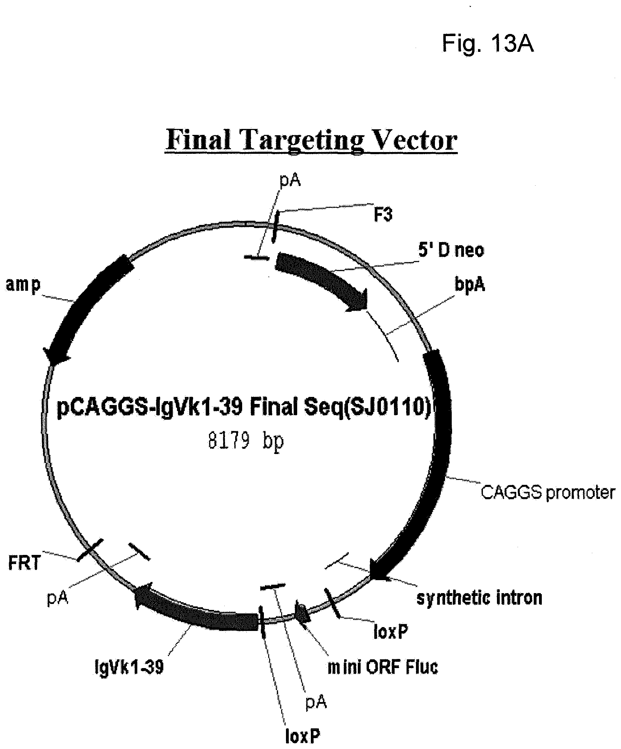

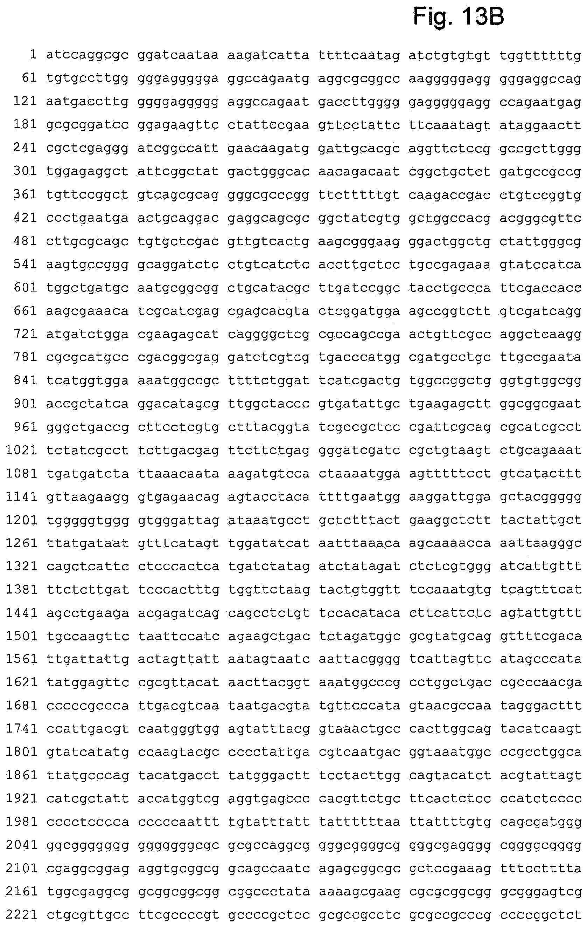

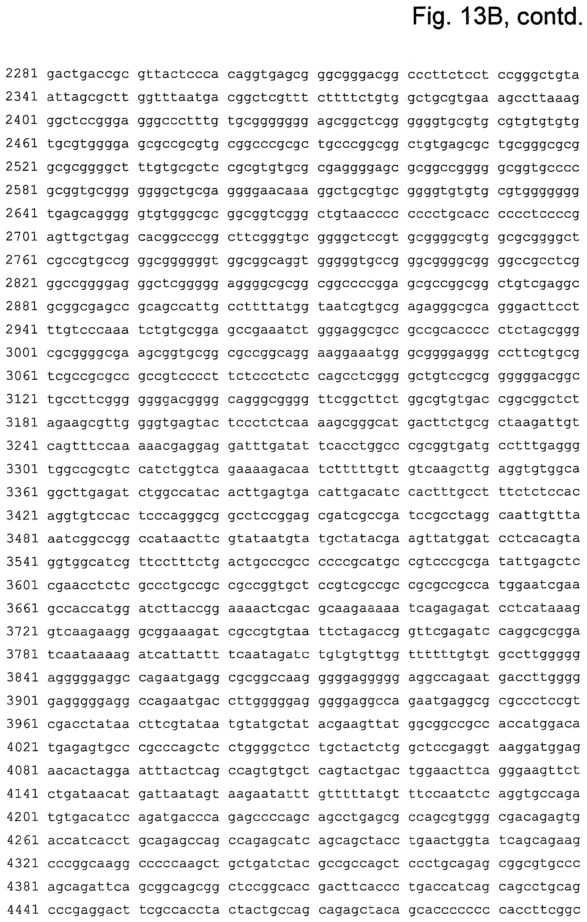

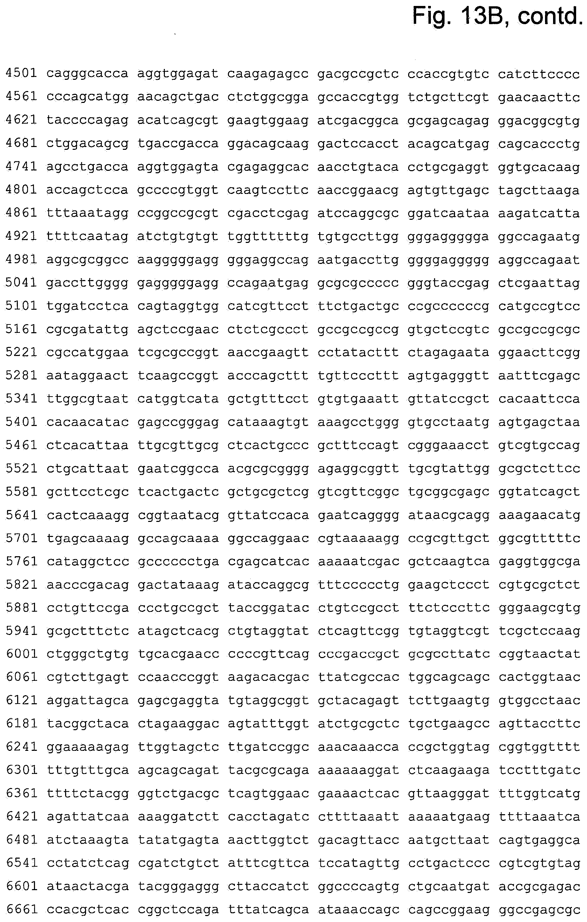

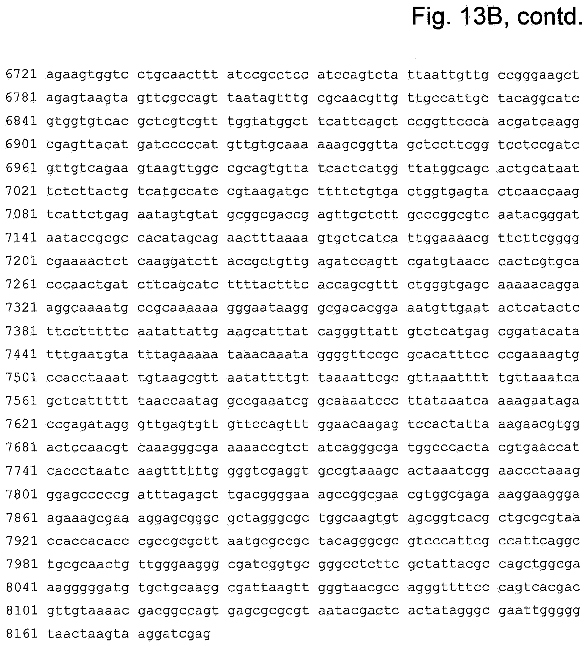

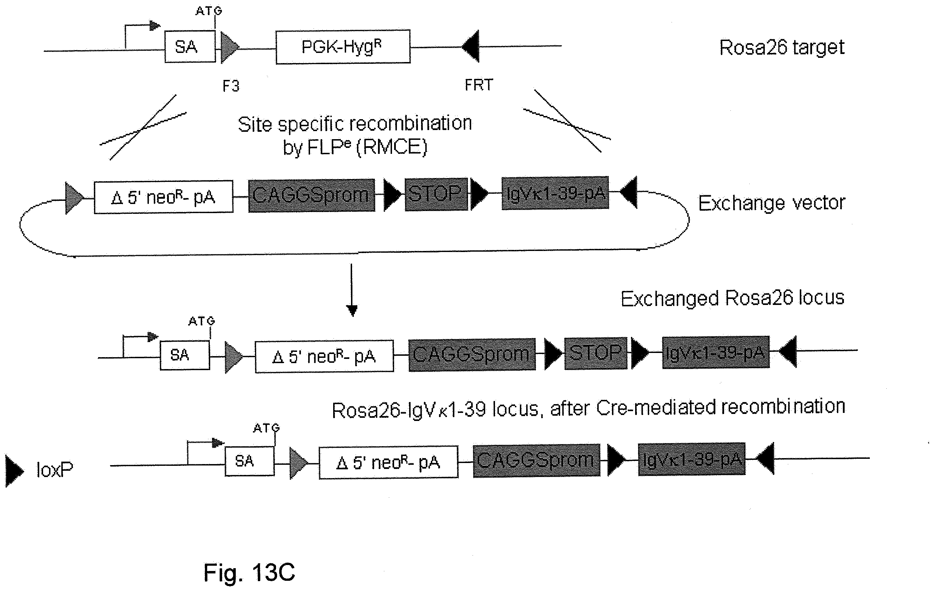

At the 5' end of the expression cassette, a NotI site was introduced and on the 3' site a NheI site. Both sites are used for cloning in the CAGGS expression module. After gene assembly according to methods used by GeneArt, the insert is digested with NotI-NheI and cloned into the expression module containing a CAGGS promoter, a stopper sequence flanked by LoxP sites ('floxed'), a polyadenylation signal sequence and, at the 5' and 3' end, sequences to facilitate homologous recombination into the Rosa26 locus of mouse ES cell lines. Promoter and/or cDNA fragments were amplified by PCR, confirmed by sequencing and/or cloned directly from delivered plasmids into an RMCE exchange vector harbouring the indicated features. A schematic drawing and the confirmed sequence of the final targeting vector pCAGGS-IgVK1-39 are shown in figure 13A and 13B. The targeting strategy is depicted in figure 13C.

### Example 6

### CAGGS expression insert based on the rearranged germline IGLV2-14/J V lambda region (IGLV2-141J-Ck)

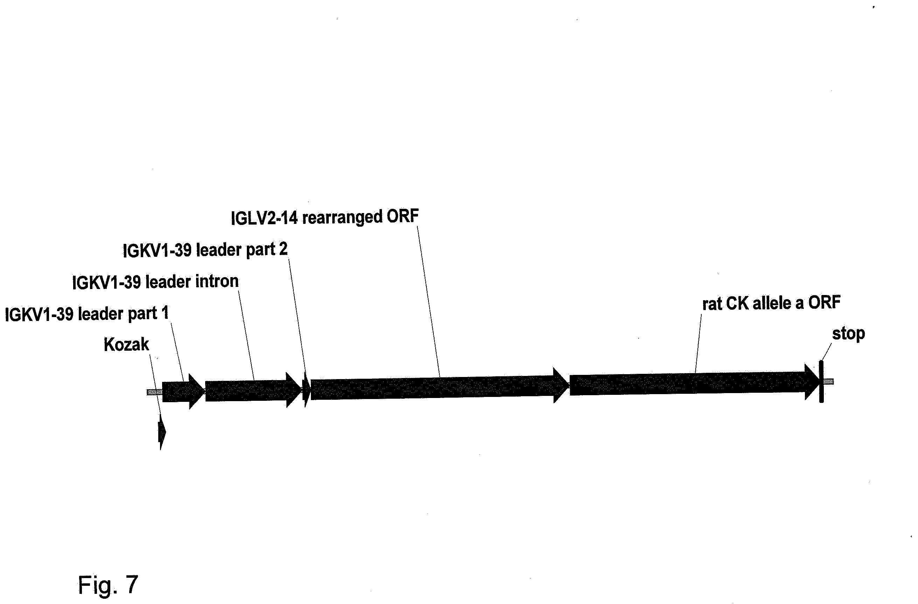

This example describes the sequence and insertion of an expression cassette incorporating the rearranged germline IGLV2-14/J V lambda region. This insert encompasses cloning sites, a Kozak sequence, a leader sequence containing an intron, an open reading frame of the rearranged IGLV2-14/J region, a rat CK constant region from allele a and a translational stop sequence (IGLV2-141J-Ck; figure 7). The primary construct consists of naturally-occurring sequences and has been analyzed and optimized by removing undesired cis acting elements like: internal TATA-boxes, poly adenylation signals, chi-sites, ribosomal entry sites, AT-rich or GC-rich sequence stretches, ARE-, INS- and CRS sequence elements, repeat sequences, RNA secondary structures, (cryptic) splice donor and acceptor sites and splice branch points (GeneArt GmbH). In addition, the codon usage in the open reading frame regions was optimized for expression in mice. The intron sequence is unchanged and thus represents the sequence identical to the human IGKV1-39 leader intron.

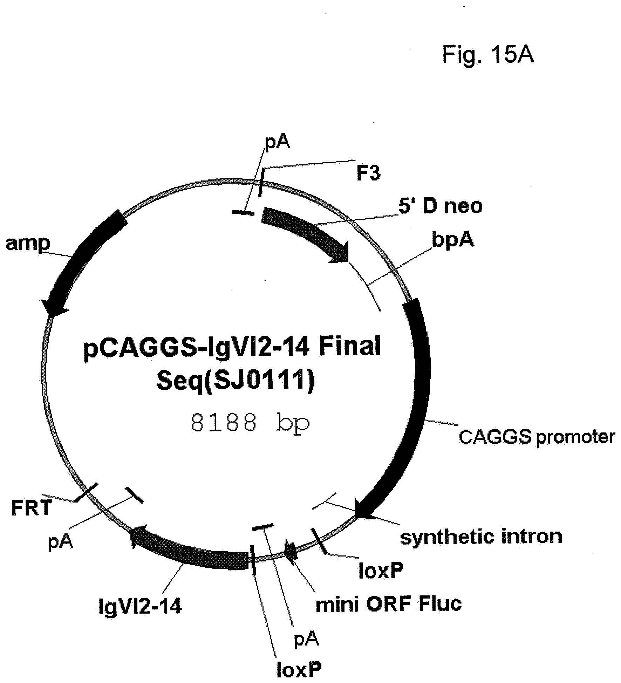

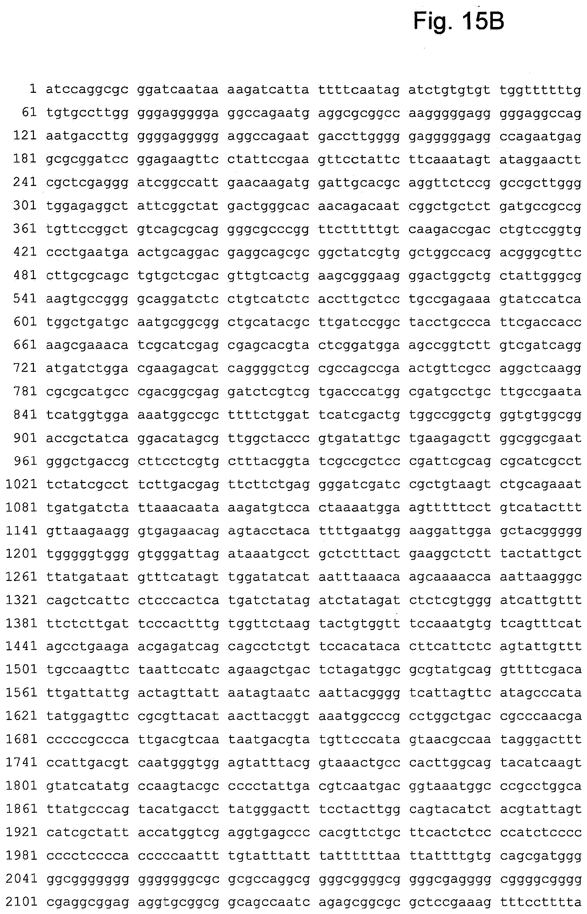

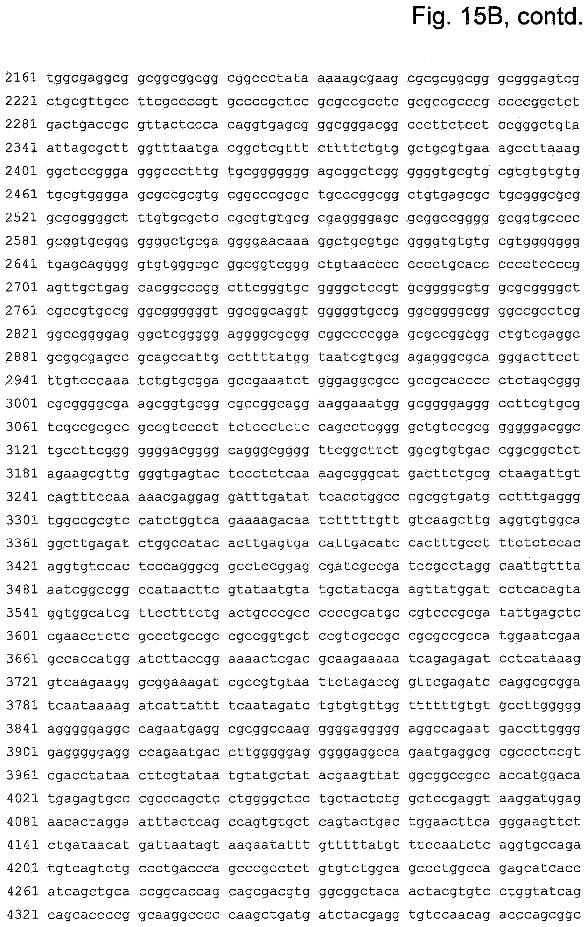

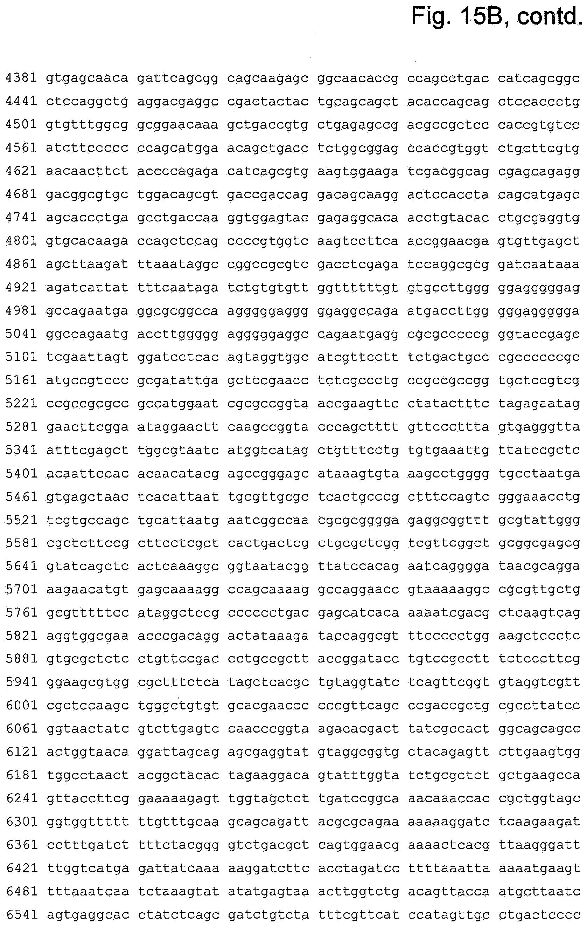

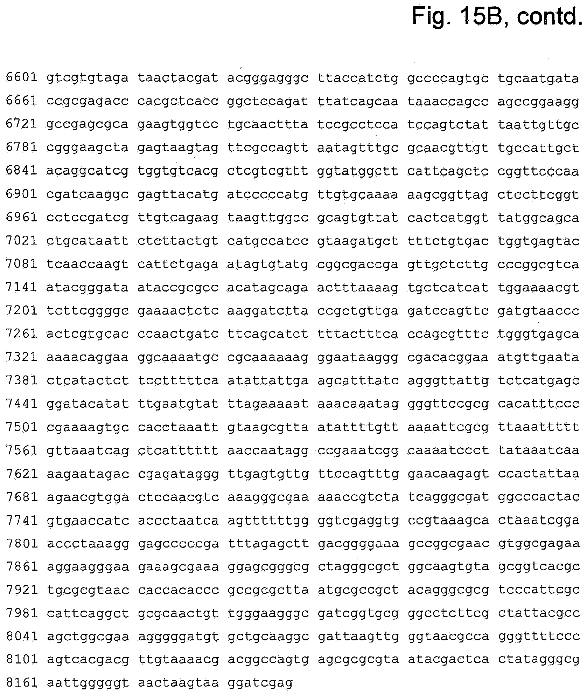

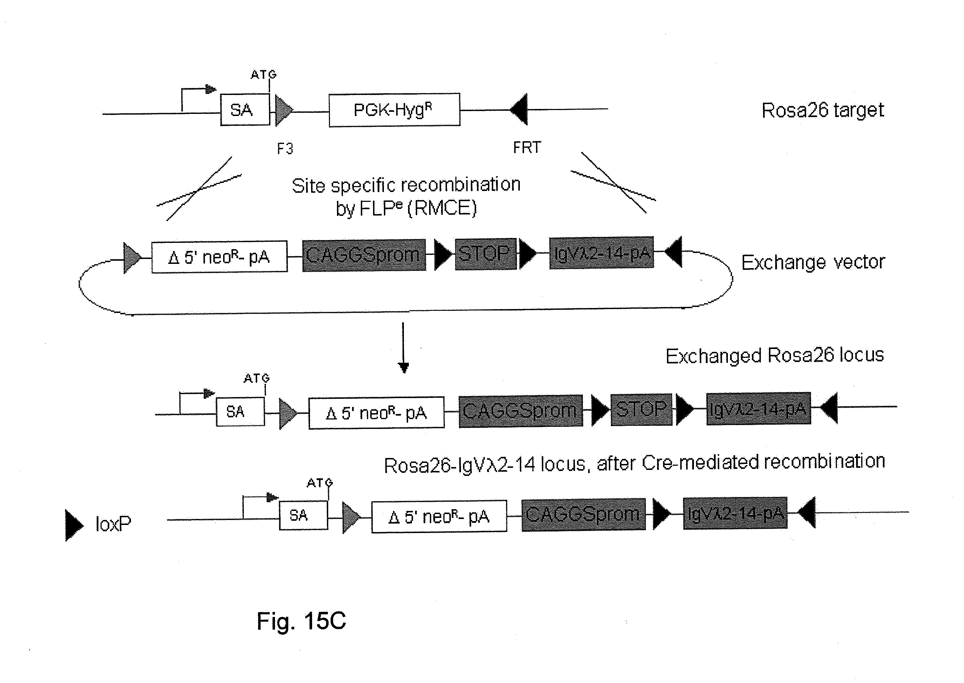

At the 5' end of the expression cassette, a NotI site was introduced and on the 3' site a NheI site. Both sites are used for cloning in the CAGGS expression module as described by TaconicArtemis. After gene assembly according to methods used by GeneArt, the insert was digested with NotI-NheI and cloned into the expression module containing a CAGGS promoter, a stopper sequence flanked by LoxP sites ('floxed'), a polyadenylation signal sequence and, at the 5' and 3' end, sequences to facilitate homologous recombination into the Rosa26 locus of mouse ES cell lines. To construct the final ROSA26 RMCE targeting vector, promoter and/or cDNA fragments were amplified by PCR. Amplified products were confirmed by sequencing and/or cloned directly from delivered plasmids into an RMCE exchange vector harbouring the indicated features. A schematic drawing and the confirmed sequence of the final targeting vector pCAGGS-IgVL2-14 is shown in figure 15A and 15B. The targeting strategy is depicted in figure 15C.

### Example 7

### Expression of IGKV1-39/J-Ck in HEK293 cell lines (pSELECT-IGKV1-39/J-Ck)

This example describes a method to verify that the IGKV1-39/J-Ck constructs described in example 5 enable expression and detection of the IGKV1-39/J-Ck L chain in HEK293 cells. The IGKV1-39/J insert (figure 6) was modified at the 5' end by changing the NotI site into a SalI site. This change is required for cloning of the product into the expression cassette plasmid pSELECT-hygro (InvivoGen). The CAGGS expression insert IGKV1-39/J-Ck and pSELECT-hygro were digested with Sall and NheI, ligated and used to transform competent XL1-Blue cells using standard techniques. Colonies were picked and DNA purified using Qiagen Midi-prep columns according to the manufacturer's procedures. The resulting light chain (LC) expressing vector named 0817676_pSELECT_0815426 was used to transfect HEK293 cells with Fugene6 (Roche) according to the manufacturer's protocols. Supernatants were screened for the presence of IGKVI-39/J-Ck light chains by ELISA and western blot using anti-rat-Ck antibodies (Beckton Dickinson #550336 and 553871) and protocols used in the art.

The VH of anti-tetanus toxoid (TT) IgG MG1494 was cloned into IgG expression vector MV1056 using restriction sites SfiI and BatEII. The resulting clone was sequence verified. HEK293T cells were transfected with five different vector combinations as shown in Table 4 (see example 8 for details of vector 0817878_pSELECT_0815427). Supernatants were harvested and IgG concentrations determined (see Table 4). No IgG could be detected for supernatants A and B containing light chain only as expected (detection antibody recognized Fc part of IgG). IgG concentration in supernatants C and D was comparable to that of positive control supernatant E, indicating correct expression of the light chain constructs.

Binding to TT was analyzed by ELISA to check functionality of the produced antibodies, using hemoglobin as negative control antigen. No TT-specific binding could be detected for supernatants A and B containing light chain only, as expected. TT-specific binding for supernatants C and D was at least as good as for positive control supernatant E, confirming correct expression of the light chain constructs and functional assembly with heavy chain. Antibodies were detected not only using an anti-human IgG secondary antibody, but also an anti-rat Ckappa light chain secondary antibody. The results confirm that the anti-rat Ckappa antibody (BD Pharmingen #553871, clone MRK-1) recognizes the light chain expressed by the pSELECT vectors.

Supernatants were analyzed by non-reducing SDS-PAGE and Western blot (not shown). Detection using an anti-human IgG heavy chain antibody did not show bands for supernatants A and B containing light chain only, as expected. Results for supernatants C and D were comparable to positive control supernatant E, with a band close to the 170 kD marker as expected for intact IgG. Additional lower molecular weight bands were observed as well for supernatants C, D and E, which might represent degradation products, IgG fragments resulting from (partial) reduction and/or irrelevant protein bands due to non-specific binding of the detection antibody.

Detection using an anti-rat Ckappa light chain antibody showed a band close to the 26 kD marker for supernatants A and B, as expected for light chain only. This band was much more intense for A compared to B, indicating that the free IGKV1-39 light chain may be better expressed and/or more stable than the free IGLV2-14 light chain. No bands were detected for control supernatant E as expected, since the expressed IgG contains a human Ckappa light chain. For supernatants C and D, expected bands close to the 170 kD marker were observed; lower molecular weight bands were also observed, but to a lesser extent than above using the anti-human IgG antibody.

In conclusion, transfection of the light chain expression constructs combined with the heavy chain of anti-tetanus toxoid (TT) IgG MG 1494 resulted in IgG production comparable to the positive control construct for both the pSELECT kappa and lambda light chain constructs. Both IgG productions yielded ELISA signals in a TT ELISA that were better than or comparable to the control IgG. SDS-PAGE and Western blot analysis confirmed the presence of intact IgG. The tested anti-rat Ckappa antibody worked efficiently in both ELISA and Western blot. Culture supernatant from cells transfected with light chain constructs only did not result in detectable IgG production nor in detectable TT-specific binding, while free light chain was detected on Western blot.

### Example 8

### Expression of IGLV2-14/J-Ck in HEK293 cell lines (pSELECT-IGLV2-14/J-Ck)

This example describes a method to verify that the IGLV2-14/J constructs described in example 6 enable expression and detection of the IGLV2-14/J-Ck L chain in HEK293 cells. The IGLV2-14/J-Ck insert (figure 7) was modified at the 5' end by changing the NotI site into a SalI site. This change is required for cloning of the product into the expression cassette plasmid pSELECT-hygro (InvivoGen). The CAGGS expression insert IGLV2-14/J-Ck and pSELECT-hygro were digested with SalI and NheI ligated and used to transform competentXL1-Blue cells using standard techniques. Colonies were picked and DNA purified using Qiagen Midi-prep columns according to the manufacturer's procedures. The resulting light chain (LC) expressing vector named 0817678_pSELECT_0815427 was used to transfect HEK293 cells with Fugene6 (Roche) according to the manufacturer's protocols. Supernatants were screened for the presence of IGLV2-14/J-Ck light chains by ELISA and western blot using anti-rat-Ck antibodies (Becton Dickinson #550336 and 553871) and protocols used in the art. See example 7 for details and results.

### Example 9

### Construction of a VK promoter-driven expression construct containing an IGKV1-39/J insert and multiple enhancer elements derived from the murine CK locus (VkP-IGKV1-39/J-Ck; VkP-O12)

This example describes the construction of an expression cassette that contains relevant elements to enable B-cell and developmental/differentiation stage-specific expression of the rearranged human IGKV1-39 VK region, based on the IGKV1-39 VK promoter region, leader containing an intron, germline V-gene, CDR3, IGKJ segment, mouse intergenic region located between Jk and CK, rat Ck allele a open reading frame, and a mouse intergenic fragment from the 3' end of the mouse CK gene ending just 3' of the 3'CK enhancer.

Optimized open reading frames of the leader, IGKV1-39 rearranged gene, and rat CK allele a gene, as described in example 5, was used for the construction of the expression cassette. The VK promoter region was obtained by gene synthesis procedures (GeneArt, GmbH) and is almost identical to the sequence of the human IGKV1-39 region between -500 bp and the ATG (start site) of the gene. The only deviation from the natural sequence is the introduction of a GCCACCATGG Kozak sequence at the ATG (start) site in order to promote translation. A genomic fragment from a mouse BAC clone (TaconicArtemis) is used as the basis for the introduction of individual elements. This fragment is identical to the sequence of the mouse VK locus starting with the intron donor site located directly 3' of the JK5 region and ending just 3' of the 3' CK enhancer and covers approximately 12.5 kb.

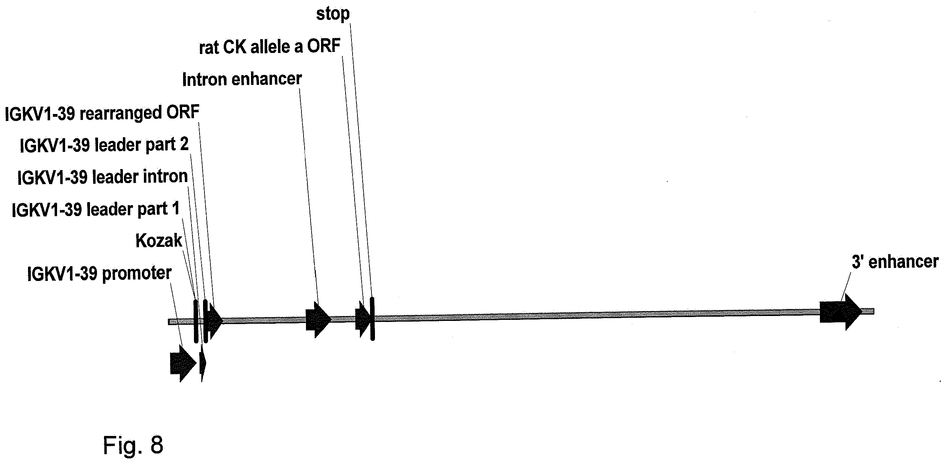

The final construct contains from 5' to 3' end the following elements: human genomic IGKV1-39 promoter (500 bp), a Kozak sequence, a human IGKV1-39 leader part 1 (optimized), a human IGKV1-39 leader intron, a human IGKV1-39 leader part 2 (optimized), a human IGKV1-39 germline gene (optimized), a human J-region (optimized), a mouse intergenic region including the intron enhancer element, a rat (Rattus norvegicus) kappa constant region (optimized), and a mouse intergenic region including the 3' kappa enhancer. The elements of this expression cassette are shown in figure 8 and named VkP-IGKV1-39/J-Ck (VkP-O12). An outline of the pVkP-O12 vector and the targeting strategy is depicted in figure 20A and 21A. The vector was introduced into ES cells following standard procedures (see example 14).

### Example 10

Construction of a VK promoter-driven expression construct containing an IGLV2-14/J clone and multiple CK locus-derived enhancer elements (VkP-IGLVL2-14/J-Ck; VkP.2a2).

This example describes the same construct as described in example 9, except that the IGKV1-39 gene and J-region are replaced by the optimized human IGLV2-14 germline gene including a unique V-J region (VkP-IGLV2-141J-Ck; VkP-2a2; figure 9).

### Example 11

### Construction of a VK promoter-driven expression construct containing an IGKV1-39 clone lacking the CK intron enhancer element (VkP-IGKV1-39/J-Ck-Δ1; VkP-O12-del1)

The construct described in example 9 was modified by removing the CK intron enhancer element, located in the intergenic region between the human J region and the rat CK region by standard PCR modification and DNA cloning methodologies (GeneArt, GmBH). The resulting expression cassette is shown in figure 10 and named VkP-IGKV1-39/J-Ck-Δ1 (VkP-O12-del1).

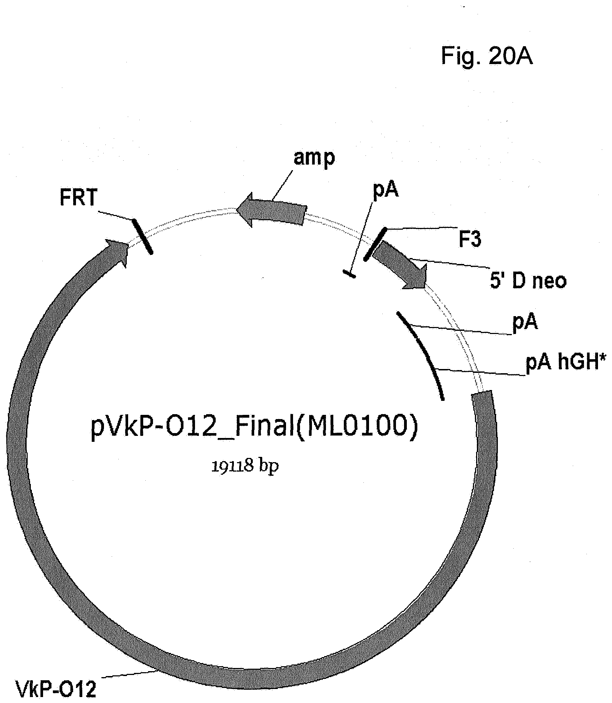

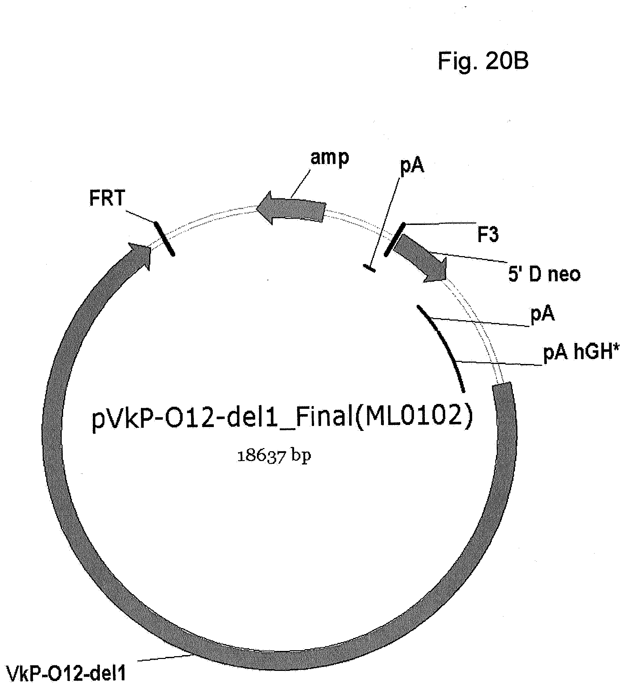

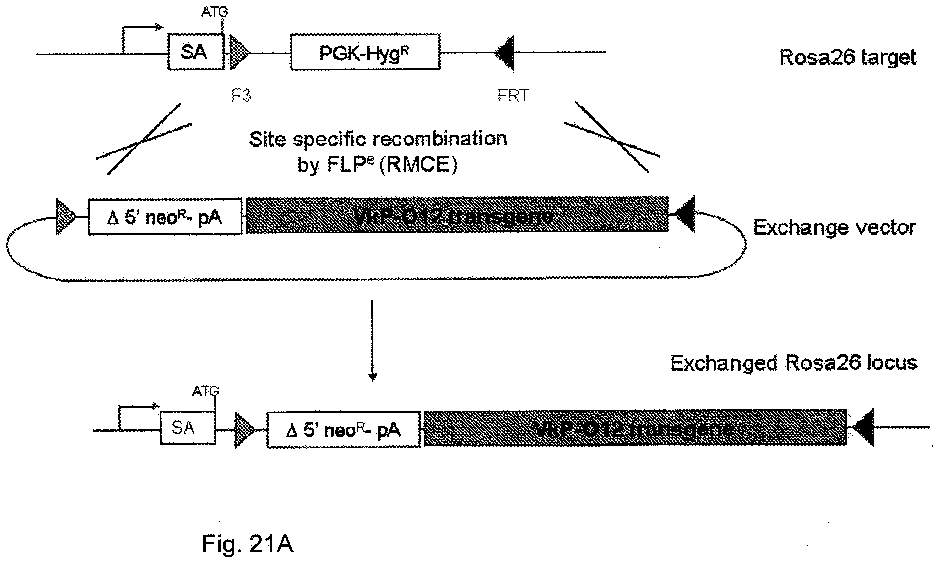

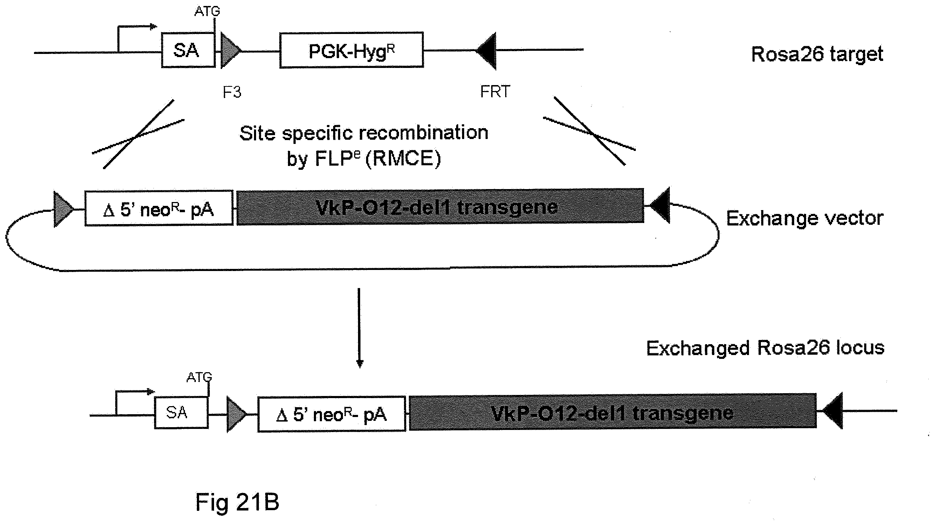

An outline of the pVkP-O12-del1 vector and the targeting strategy is depicted in figure 20B and 21B. The vector was introduced into ES cells following standard procedures (see example 14).

### Example 12

### Construction of a VK promoter-driven expression construct containing an IGKV1-39 clone lacking the CK intron enhancer element and a truncated 3' CK enhancer element (VKP-IGKV1-39/J-Ck-Δ2; VkP-O12-del2)

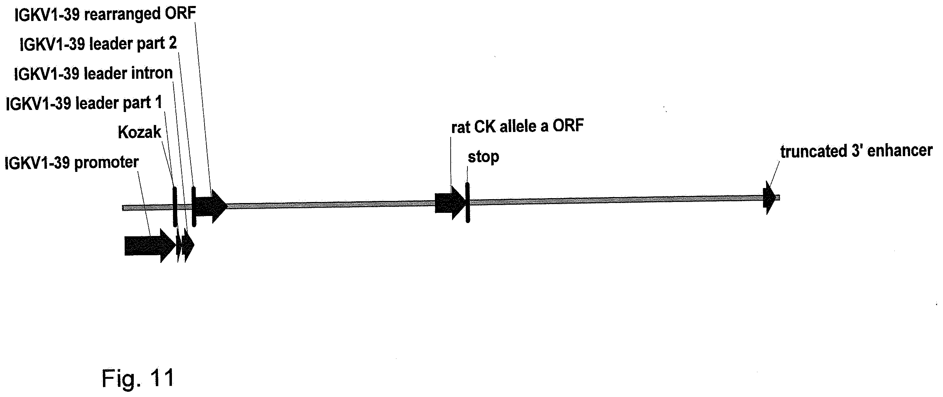

The construct described in example 11 was modified by truncating the 3' CK enhancer element and deleting part of the intergenic region 3' of the rat Ck gene, to remove potential inhibitory elements. This was achieved by removing the intergenic sequence between an EcoRV site (located 3' of the rat Ck gene) and the NcoI site present in the 3' enhancer (5993 bp) and further removing the sequence between the 3' enhancer BstXI site and the BatXI site 3' of the 3'enhancer (474 bp) using standard methods. The resulting expression cassette is shown in figure 11 and named VkP-IGKV1-39/J-Ck-Δ2 (VkP-O12-del2).

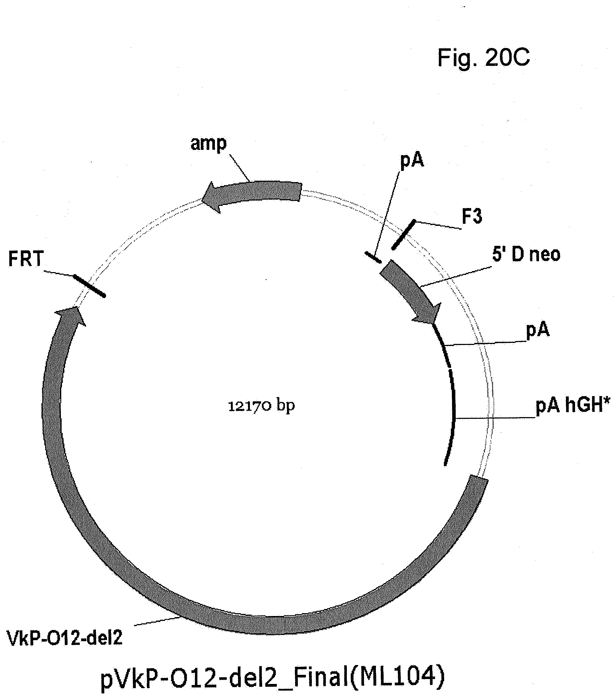

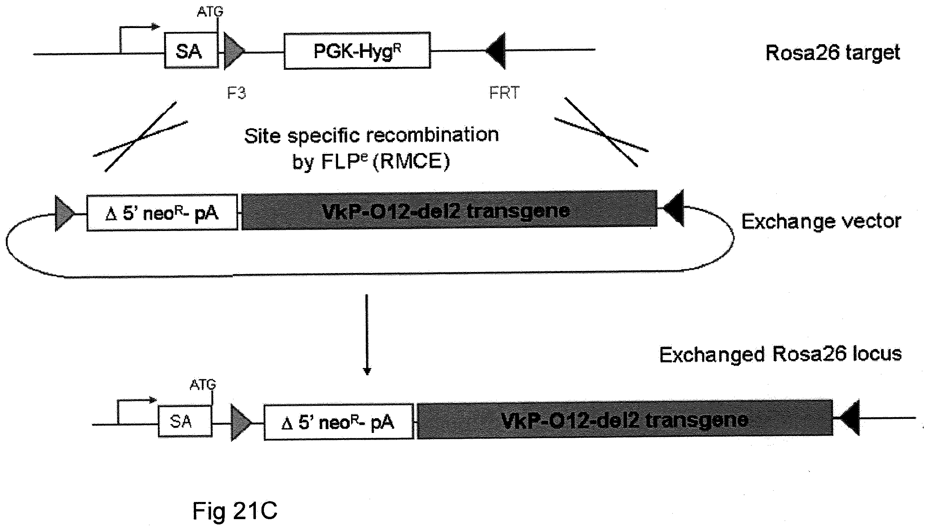

An outline of the pVkP-O12-del2 vector and the targeting strategy is depicted in figure 20C and 21C. The vector was introduced into ES cells following standard procedures (see example 14).

### Example 13

### Expression of Vk constructs in cell lines

The constructs described in example 9-12 are tested for their ability to produce light chain proteins in the myeloma cell lines MPC11 (ATCC CCL167), B-cell lymphoma WEHI231 (ATCC CRL-1702), the T-cell lymphoma EL4 (ATCC TIB-39) and in HEK293 (ATCC CRL1573). The enhancer and promoter elements in the construct enable expression in the B-cell lines but not in cell lines derived form other tissues. After transfection of the cell lines using purified linearized DNA and Fugene6 (Roche) cells are cultured for transient expression. Cells and supernatant are harvested and subjected to SDS-PAGE analysis followed by western blotting using a specific anti-rat-C-kappa antibody. Supernatants are analyzed in ELISA for secreted L chains using the anti-rat CK antibody (Beckton Dickinson #550336).

### Example 14

### Generation of transgenic ES lines

All constructs as described in examples 3, 4, 5, 6, 9, 10, 11 and 12 were used to generate individual stable transgenic ES lines by means of homologous recombination. The methods for generation of transgenic ES lines via homologous recombination are known in the field (e.g. Eggan et al., PNAS 98, 6209-6214; Seibler J, Zevnik B, Küter-Luks B, Andreas S, Kern H, Hennek T, Rode A, HeimannC, Faust N, Kauselmann G, Schoor M, Jaenisch R, Rajewsky K, Kühn R, Schwenk F. Nucleic Acids Res. 2003 Feb 15;31(4):e12; Hogan et al. (Cold Spring Harbor Laboratory Press, Cold Spring Harbor NY.), pp. 253-289.).

For all constructs described in examples 5-6, and examples 9-12, the RMCE ES cell line (derived from mouse strain 129S6B6F1-Gt(ROSA)26Sortm10Arte) was grown on a mitotically inactivated feeder layer comprised of mouse embryonic fibroblasts (MEF) in DMEM High Glucose medium containing 15% FBS (PAN 1302-P220821). Leukemia Inhibitory Factor (Chemicon ESG 1107) was added to the medium at a concentration of 900 U/mL. For manipulation, 2x10⁵ ES-cells were plated on 3,5 cm dishes in 2 ml medium. Directly before transfection, 2 ml fresh medium was added to the cells. 3 ul Fugene6 Reagent (Roche; Catalog No. 1 814 443) was mixed with 100 ul serum free medium (OptiMEM I with Glutamax I; Invitrogen; Catalog No. 51985-035) and incubated for 5 min. 100 ul of the Fugene/OptiMEM solution was added to 2 ug circular vector and 2 ug CAGGS-Flp and incubated for 20 min. This transfection complex was added dropwise to the cells and mixed. Fresh medium was added to the cells the following day. From day 2 onwards the medium was replaced daily with medium containing 250 ug/mL G418 (Geneticin; Invitrogen; Catalog No. 10131-019). Seven days after transfection, single clones were isolated, expanded, and molecular analyzed by Southern blotting according to standard procedures.

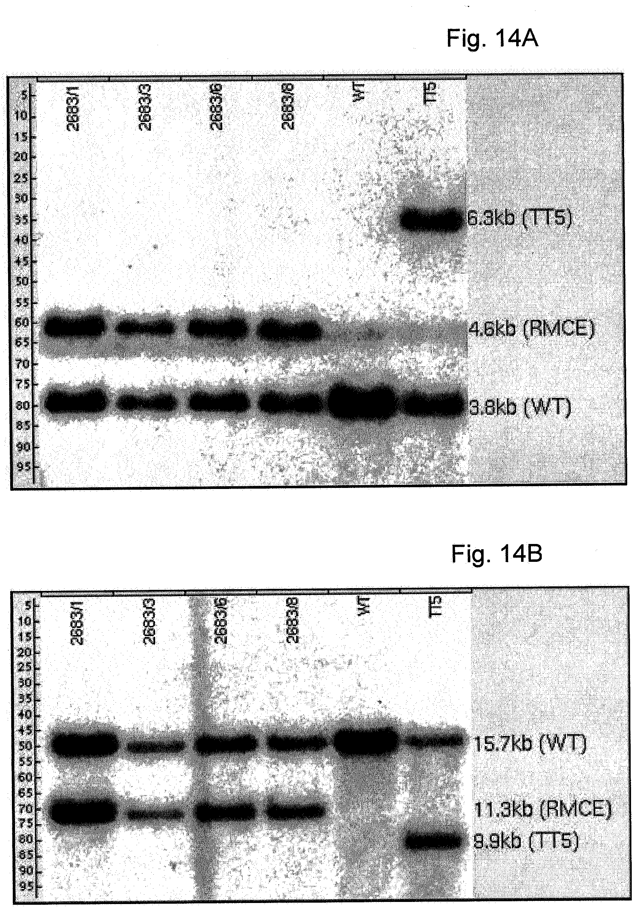

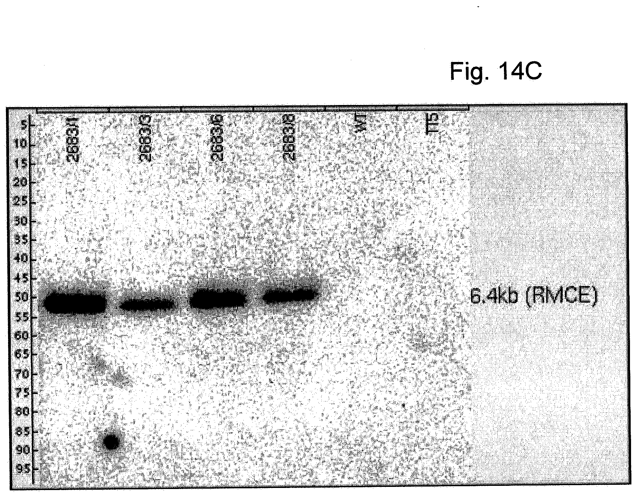

For each construct, analysis of multiple clones by restriction enzyme digestion of genomic DNA of single clones followed by hybridization with 5' probes, 3' probes, and internal probes resulted in clones that comprised a correct, single insertion at the correct position in the Rosa26 locus. An example is provided in figure 14.

### Example 15

### Generation of transgenic mouse strains

All ES cell lines that were generated and verified for their modifications as described in example 14 were used to generate stable transgenic mice by means of tetraploid recombination. The methods are known in the field. In general, after administration of hormones, superovulated Balb/c females were mated with Balb/c males. Blastocysts were isolated from the uterus at dpc 3.5. For microinjection, blastocysts were placed in a drop of DMEM with 15% FCS under mineral oil. A flat tip, piezo actuated microinjection-pipette with an internal diameter of 12 - 15 micrometer was used to inject 10-15 targeted C57BL/6 N.tac ES cells into each blastocyst. After recovery, injected blastocysts were transferred to each uterine horn of 2.5 days post coitum, pseudopregnant NMRI females. Chimerism was measured in chimeras (G0) by coat colour contribution of ES cells to the Balb/c host (black/white). Highly chimeric mice were bred to strain C57BL/6 females. Depending on the project requirements, the C57BL/6 mating partners are non-mutant (W) or mutant for the presence of a recombinase gene (Flp-Deleter or Cre-deleter or CreER inducible deleter or combination of Flp-deleter/CreER). Germline transmission was identified by the presence of black, strain C57BL/6, offspring (G1).

For example, ESC clone IgVK1-39 2683 8 (see examples 5 and 14) was injected in a total of 62 blastocysts in 3 independent experiments. 3 litters were obtained with a total of 6 pups. All pups were chimaeric. 3 heterozygous offspring pups were obtained that were used for further crossing.

ESC Clone Kappa 2692 A-C10 (see examples 3 and 14) was injected in a total of 54 blastocysts in 3 independent experiments. 3 litters were obtained with a total of 11 pups, of which 10 were chimaeric. 8 heterozygous offspring pups were obtained that were used for further crossing.

ESC Clone Kappa 2692 B-C1 (see examples 3 and 14) was injected in a total of 51 blastocysts in 3 independent experiments. 2 litters were obtained with a total of 6 pups, of which 4 were chimaeric. 3 heterozygous offspring pups were obtained that were used for further crossing.

### Example 16

### Breeding

This example describes the breeding for obtaining mice that contain transgenic expression cassettes as described example 14 and knock out mice in which the endogenous lambda and kappa loci have been silenced. The localization of V-lambda on chromosome 16 and CD19 on chromosome 7 allow standard breeding procedures. The breeding of the co-localized Vk locus and Rosa26 locus on chromosome 6 with a distance of about 24 cM requires special attention during the screening as only a percentage of the offspring shows crossover in a way that both modifications are brought together on one chromosome.

All four loci have to be combined in a single mouse strain that is homo- or heterozygous for CD19-cre (not described) and modified Rosa26 transgene and homozygous for the other loci. Breeding is performed by standard breeding and screening techniques as appropriate and offered by commercial breeding companies (e.g. TaconicArtemis).

### Example 17

### Immunizations of mice

Primary and booster immunization of mice are performed using standard protocols.

To validate the transgenic expression of human rearranged V_{K} 012 (IGKV1-39) - rat C_{K} light chains (see example 5, 14-16) in B cells from CD19-HuVκ1 mice and to assess its impact on VH repertoire size, diversity of VH family usage and V(D)J recombination after immunization, the CD19-HuVκ1 transgenic mice are immunized with tetanus toxin vaccine (TT vaccine) and VH sequence diversity of randomly-picked clones from CD18-HuVκ1 mice are compared with TT-immunized wt mice and CD19-Cre HuVk1 negative littermates. Data on the SHM frequency of the human Vκ 012 transgene in the immunized mice are obtained. A diverse collection of at least 40 TT-specific, clonally-unrelated mAbs containing the human Vκ 012 are recovered from CD19-HuVx1 mice by phage display.

For this, three adult CD19-HuVκ1 mice are vaccinated with TT vaccine using standard immunization procedures. After immunization, serum titers are measured using TT specific ELISA (TT: Statens Serum Institute, Art. no. 2674) and spleen suspensions subjected to cell sorting by the FACS procedure after staining with a rat Cκ-specific monoclonal antibody to isolate transgenic B cells (clone RG7/9.1; BD Pharmingen# 553901, Lot# 06548). RNA from rat Cx-positive B cells are extracted and the resulting cDNA material used for library building and SHM analysis.

The standard monoclonal mouse anti-rat Cκ antibody (clone RG7/9.1; BD Pharmingen# 553901, Lot# 06548) is used in FACS analysis of transgene expressing B cells (Meyer et al., 1996, Int. Immunol., 8: 1561). The clone RG7/9.1 antibody reacts with a monotypic (common) kappa chain determinant. This anti-rat Cκ antibody (clone RG719.1 (BD Pharmingen# 553901, Lot# 06548) is labeled with R-phycoerythrin (PE) using the LYNX rapid conjugation kit according to the manufacturer's instructions for FACS analysis and sorting. The labeled antibody is firstly tested by flow cytometry for binding to rat Cκ-containing functional light chain proteins produced into transiently transfected HEK-293T cells; the un-conjugated antibody serves as a positive control. Two other antibodies shown to bind to rat Cκ by ELISA and Western-blot (see example 7) are tested as well by flow cytometry.

Fab-phage display library building is carried out with a set of optimized degenerate PCR primers designed to amplify C57BL/6 VH genes; the minimal library size is 10⁶ clones, and minimal insert frequency is 80%. The vector used, MV1043 (figures 3 and 12), contains the human Vκ 012 fused to a human Cx region. The rat Cκ is therefore exchanged for the human counterpart in the library generation process.

Before selection, VH sequencing of 96 randomly picked clones is performed to validate VH repertoire diversity that is compared to diversity obtained from an unselected library previously generated using the same procedures from BALB/c mice immunized with TT. A library from C57Bl/6 wt mice that are immunized in the same way allows diversity comparison between two preselected libraries sharing the same vaccine and the same genetic background.

Several independent selections are performed on TT coated in immunotubes. Variables that may be included are selections using biotinylated antigens in solution or selections on captured TT. Based on the number and diversity of ELISA-positive clones obtained in the first selections, decisions on additional rounds of selection are made. Clones are considered positive when > 3x positive over a negative control clone. Positive clones are analyzed by ELISA against a panel of negative control antigens to verify antigen specificity. The aim is to identify at least 40 unique VH regions, as based on unique CDR3 sequences and V_{H}DJ_{H} rearrangements.

Amplification of the cDNA material from rat Cκ-positive sorted B cells is performed with a PCR forward primer specific to the human leader sequence and a PCR reverse primer specific to the rat Cκ sequence, in a region not redundant with the mouse Cx sequence, as reported in a recent study (Brady et al., 2006, JIM, 315: 61). Primer combinations and annealing temperatures are firstly tested on cDNA from HEK-293T cells transfected with 0817676_pSELECT_0815426 = pSELECT vector with IGKV1-39 DNA cassette (see example 7).

The amplification products is cloned in pJET-1 vector and after XL1-blue transformation, 96 colonies are sequenced for assessing VL SHM frequency by direct comparison to the Vκ 012 (IGKV1-39) germline sequence. The R/S ratio method, as described in our study on human TT. specific antibodies (de Kruif et al., 2009, J. Mol. Biol., 387: 548) allows discrimination between random mutations and antigen-driven mutations that occurred on VL sequences.

### Example 18

Immunofluorescent analysis of B cell populations in transgenic mouse lines.

This example describes the use of antibodies and flow cytometry to analyze B cell populations in primary (bone marrow) and secondary (spleen, peritoneal) lymphoid organs and blood. Methods and reagents are described in Middendorp et al. (2002) J. Immunol. 168:2695 and Middendorp et al. (2004) J. Immunol. 172:1371. For analysis of early B cell development in bone marrow, cells were surface stained with combinations of antibodies (Becton Dickinson) specific for B220, CD19, CD25, IgM, IgD, mouse Ckappa, mouse Clambda and rat Ckappa to detect pro-B cells, pre-B cells, large pre-B cells, early and late immature B cells and recirculating B cell populations expressing the transgene on their surface. DAPI staining (Invitrogen) was included to exclude dead cells from the analysis and FC block (Becton Dickinson) to inhibit antibody interaction with Fc receptors on myeloid cells. For analysis of surface transgene expression on B cell populations in peripheral lymphoid organs and blood, cells were stained with combinations of antibodies (Becton Dickinson) specific for B220, CD5, CD19, CD21, CD23, IgM, IgD, mouse Ckappa, mouse Clambda and rat Ckappa. DAPI staining was included to exclude dead cells from the analysis and FC block to inhibit antibody interaction with Fc receptors on myeloid cells. In addition, combinations of antibodies (Becton Dickinson) specific for CD3, CD4, CD11b, CD11c and NK1.1 were included to determine if transgene expression occurred in cell types outside of the B cell compartment.

Three mice heterozygous for the human IGKV1-39/rat Ckappa transgene and heterozygous for the CD19-Cre transgene on a C57BL6 background (HuVk1/CD19-Cre) were analysed. As controls for the FACS analysis, three littermate mice wild type for the human IGKV1-39/rat Ckappa transgene and heterozygous for the CD19-Cre transgene on a C57BL6 background (CD19-Cre) and two C57BL6/NTac mice (Wt) were included. All animals were allowed to acclimatize in the animal facility for 1 week before analysis and all mice were male and 6 weeks of age. Lymphocytes were isolated from the femurs, spleens, peritoneal cavity and blood of mice using conventional techniques as previously described (Middendorp et al. (2002) J. Immunol. 168:2695 and Middendorp et al. (2004) J. Immunol. 172:1371). Antibodies were pre-combined as shown in Table 10 and staining was carried out in 96 well plates. Incubation with the PE-conjugated anti-rat C kappa (described above) was carried out before staining with the rat anti-murine antibodies to avoid non-specific binding. After completion of cell staining, labeled cells were analysed on a Becton Dickinson LSR II FACS machine and the acquired data analysed with FlowJo software (v6.4.7).

Transgenic mice were similar in weight, appearance and activity to wild type mice. No gross anatomical alterations were observed during the harvesting of tissues. No difference was observed in the numbers of B cells in the bone marrow (BM) and spleen (Table 11) or in the numbers of B cells, T cells and myeloid cells in peripheral organs between transgenic and wild type mice. In addition, the frequency or proportion of the cells in the different lymphocyte developmental pathways was not altered in transgenic mice when compared to wild type mice. Thus in the double transgenic (HuVk1/CD19-Cre) and transgenic (CD19-Cre) mice lymphoid and most importantly B cell development was indistinguishable from wild type mice.

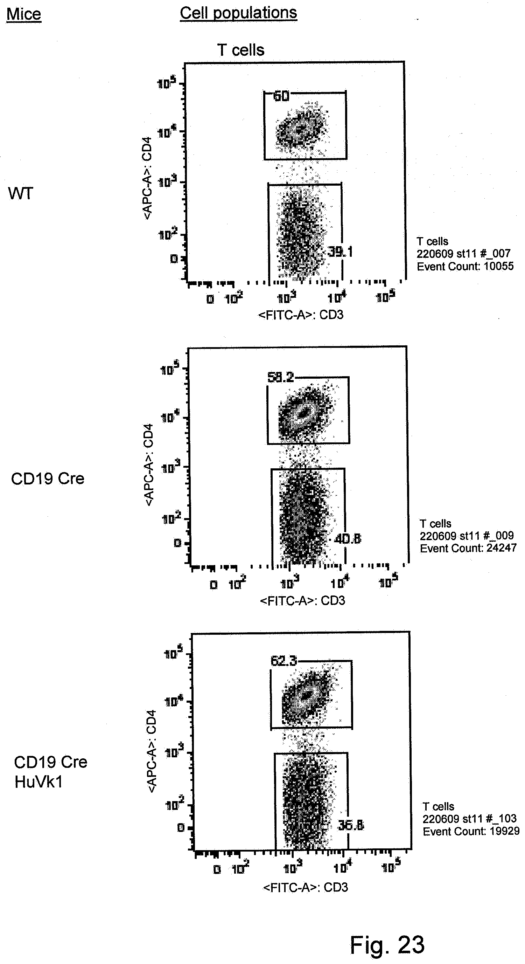

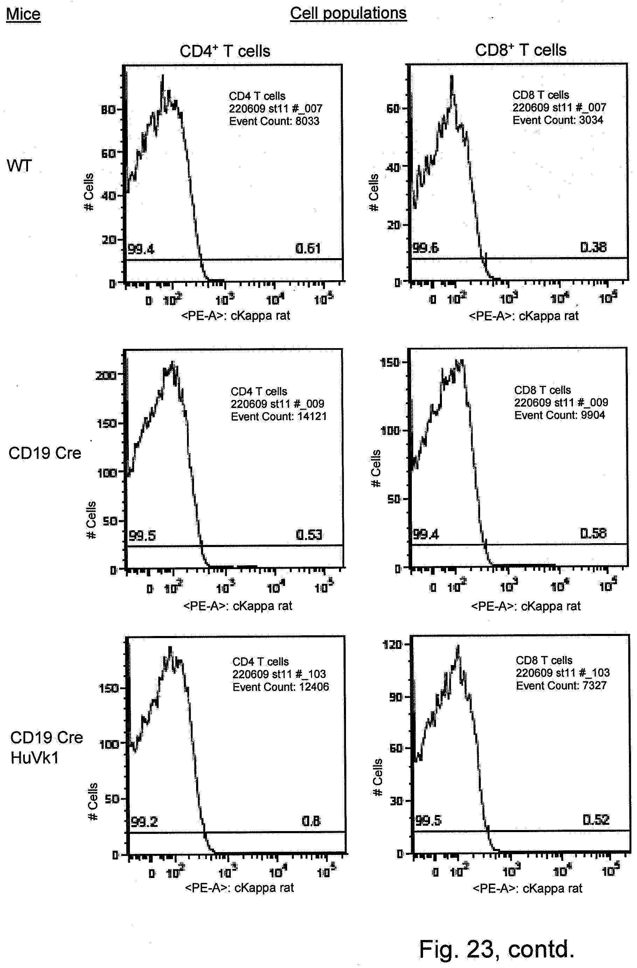

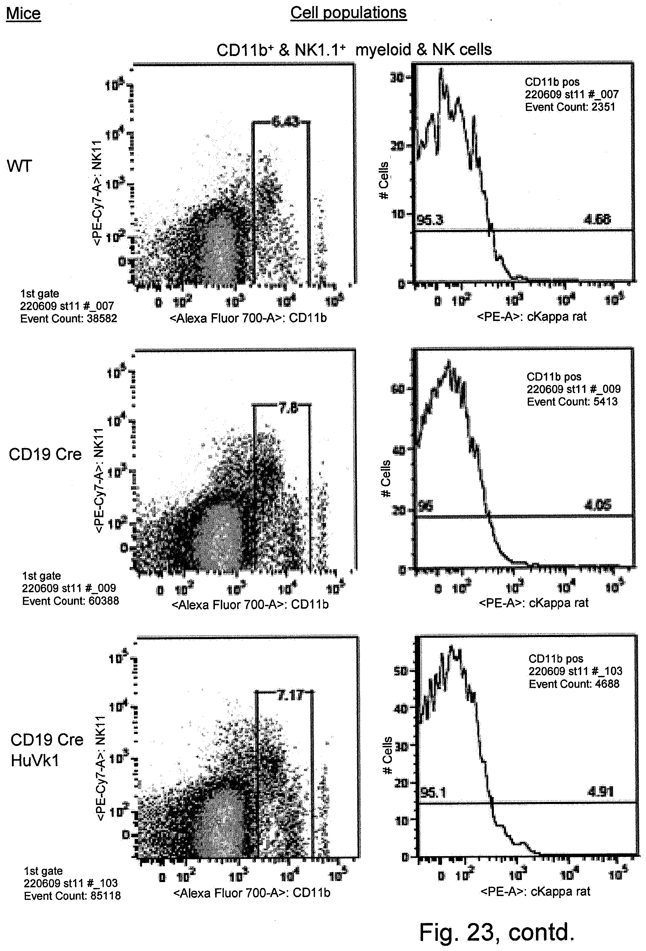

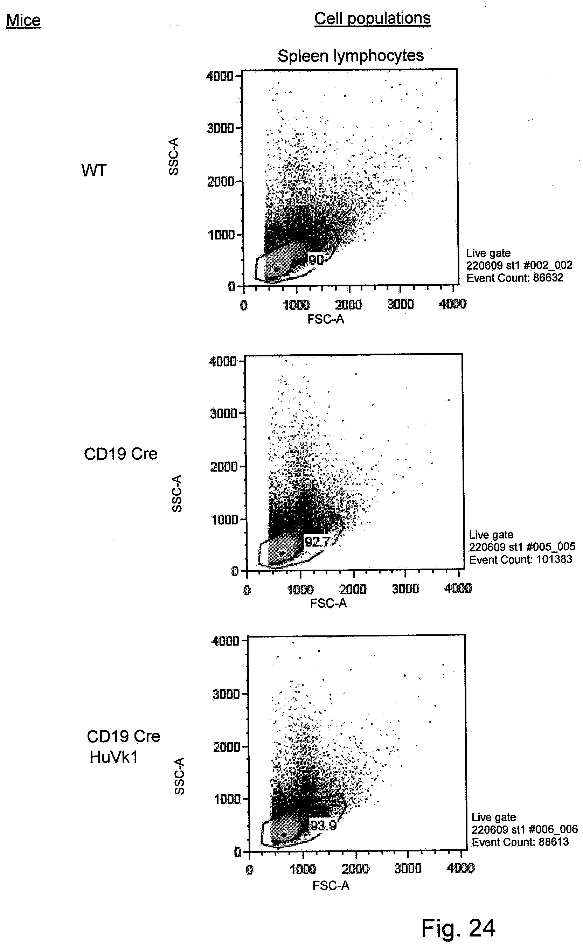

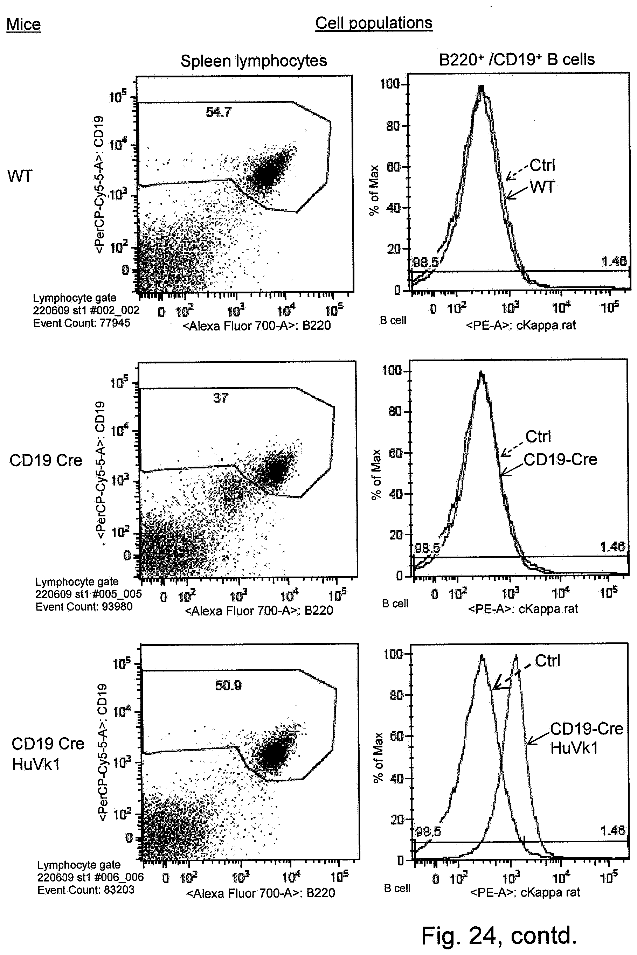

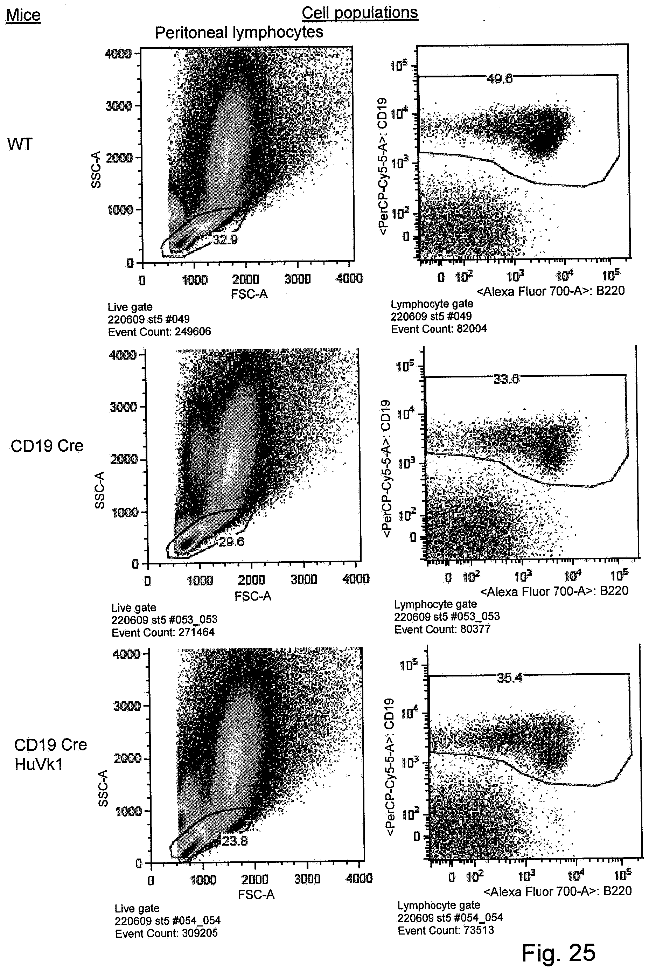

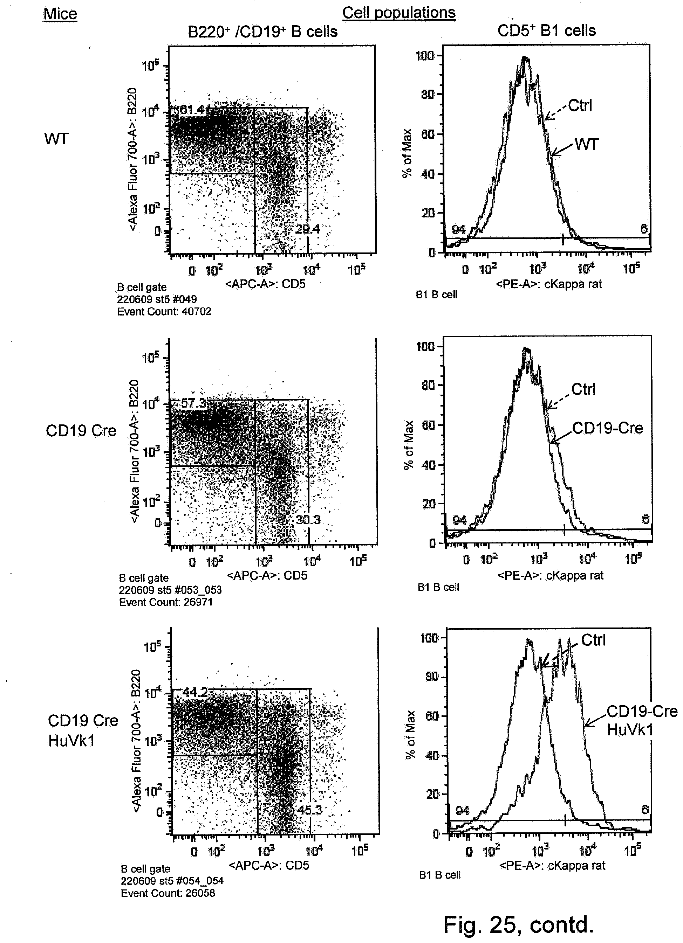

In the peripheral lymphoid organs, staining with the transgene specific antibody (anti-ratCkappa-PE) was only observed in the B cell populations. T cell, myeloid cell and NK cell populations were all negative for surface expression of the transgene in the spleen (Figure 23). In contrast, in cells stained with the pan B cell markers B220 and CD19 all cells were shifted to the right in the FACS plot indicating cell surface expression of the transgene (Figure 24). A similar transgene-specific staining was measured in CD5⁺ B1 cells of the peritoneum, a developmentally distinct population of B cells (Figure 25).

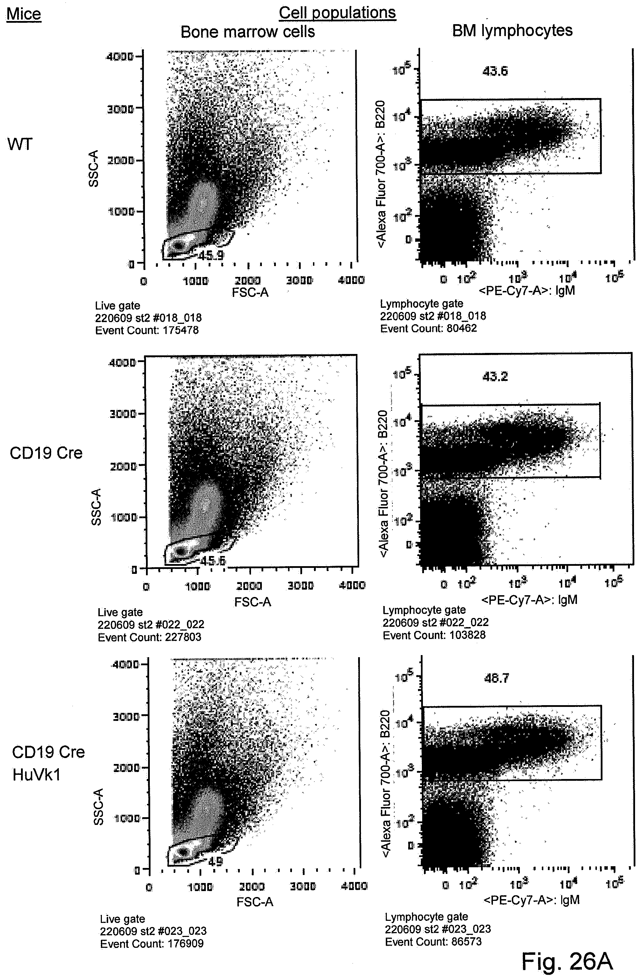

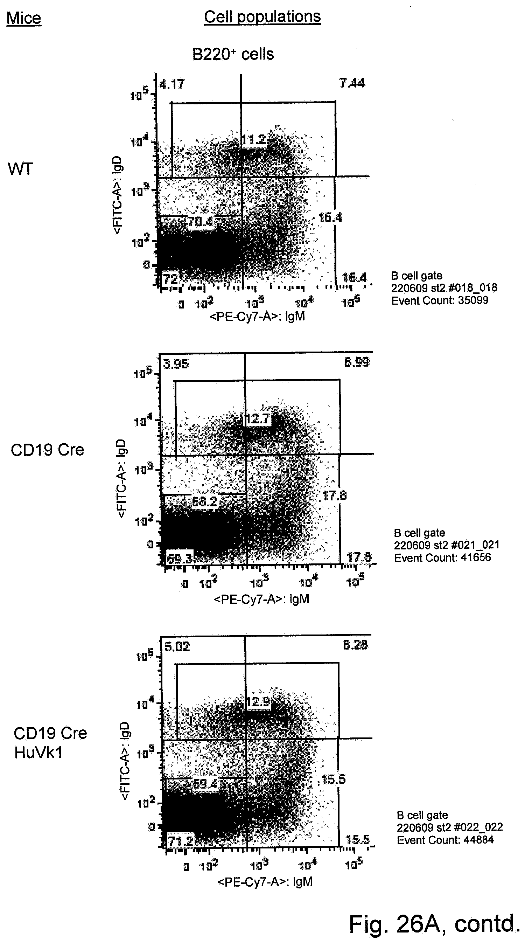

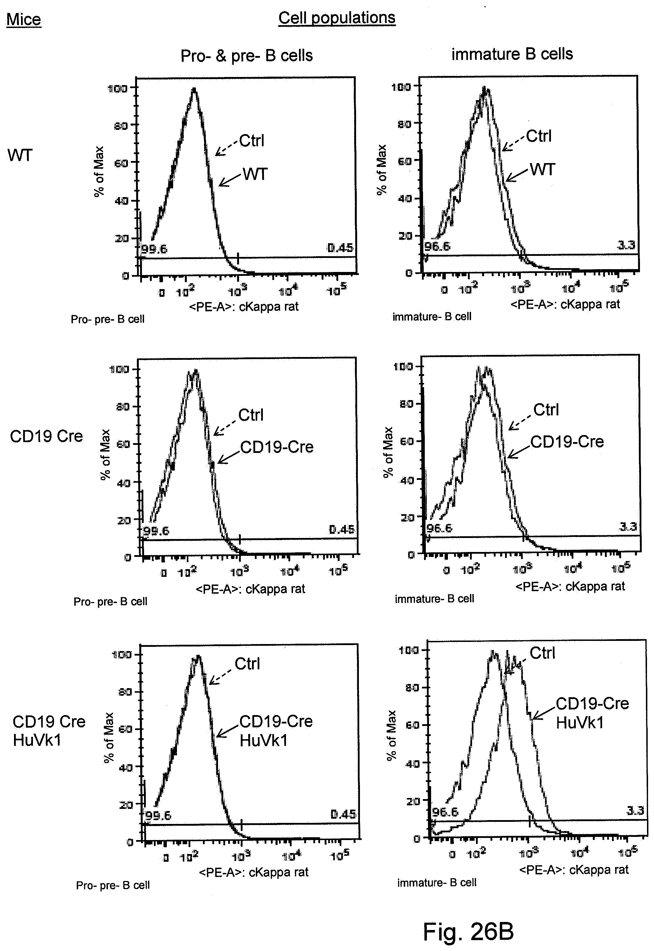

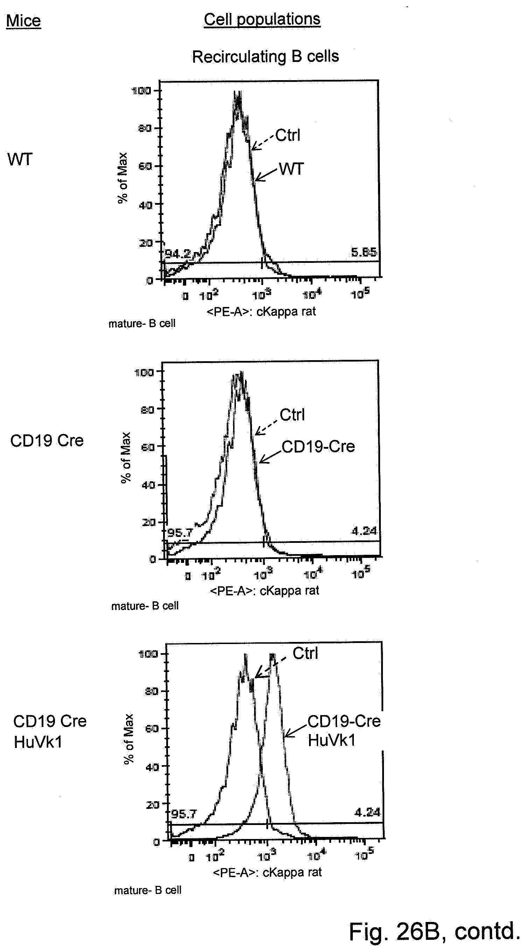

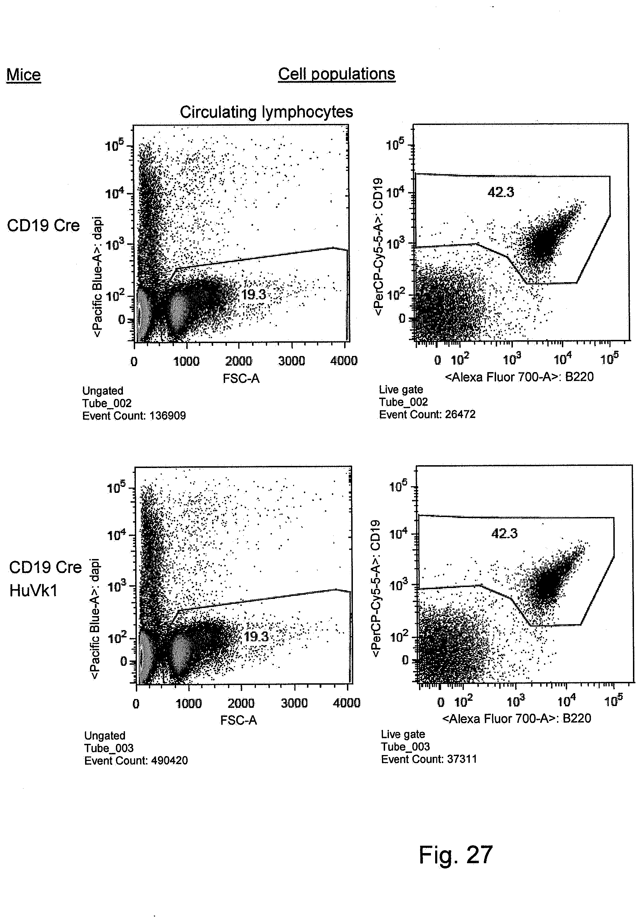

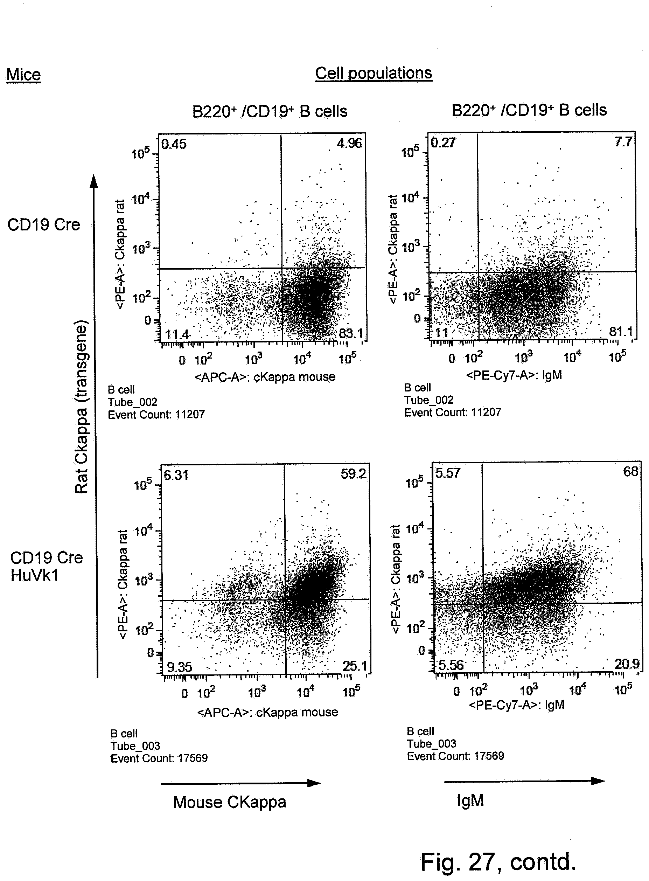

Differentiation of B cells from multilineage precursors to mature B cells occurs in the bone marrow. In the lymphocytes analyzed from the bone marrow, extracellular and transgene expression was not detectable in the earliest B cell progenitors the pro- and pre-B cell consistent with the pattern of normal light chain expression (Figure 26). Transgene expression first becomes detectable in immature B cells, the developmental stage at which the germline murine light chain undergoes rearrangement and is expressed at the cell surface in the context of the preselected heavy chain (Figure 26). Consistent with the staining in the spleen transgenic light chain expression is also detected on mature recirculating B cells (Figure 26). Thus the CD19-Cre driven expression of the transgene is consistent with the normal pattern of light chain expression. The staining with the endogenous light chain- specific antibody is more intense than that of the transgene-specific light chain antibody. This may indicate a higher expression level of the endogenous light chain, a more sensitive staining with the endogenous light chain-specific antibody or a combination of both. Importantly, the intensity of the surface expression of the transgenic light chain is correlated with both endogenous light chain and IgM surface expression as observed in staining of circulating B cells in the blood (Figure 27).

Thus overall this analysis demonstrates that expression of the human IGKV1-39/Ckappa transgene is restricted to the B cell compartment and the temporal regulation of its expression is similar to the endogenous kappa and lambda light chains resulting in normal development of all B cell populations. The apparent lower level of expression of the transgene could be explained by the strength of the promoter in comparison to the promoter and enhancers present on endogenous light chain genes or by a delay in transgene expression that gives the endogenous light chains a competitive advantage in pairing with the rearranged heavy chain. This is consistent with the observation that as B cells mature the relative intensity of transgene staining increases compared to the endogenous light chains. In addition, the observation that B cells numbers are normal and that every surface Ig+ B cell co-expresses an endogenous and transgenic light chain supports the conclusion that the IGKV1-39 variable region is capable of pairing with a normal repertoire of different murine heavy chain variable regions. We conclude from this analysis that insertion of the IGKV1-39/rat Ckappa transgene driven by the CD19-Cre activated CAGGS promoter in the Rosa locus facilitates timely and B cell-specific expression of the transgene and that the transgene is capable of pairing with a normal repertoire of murine heavy chains.

### Example 19

### Epibase^{®} T-cell epitope profile for IGKV1-39.

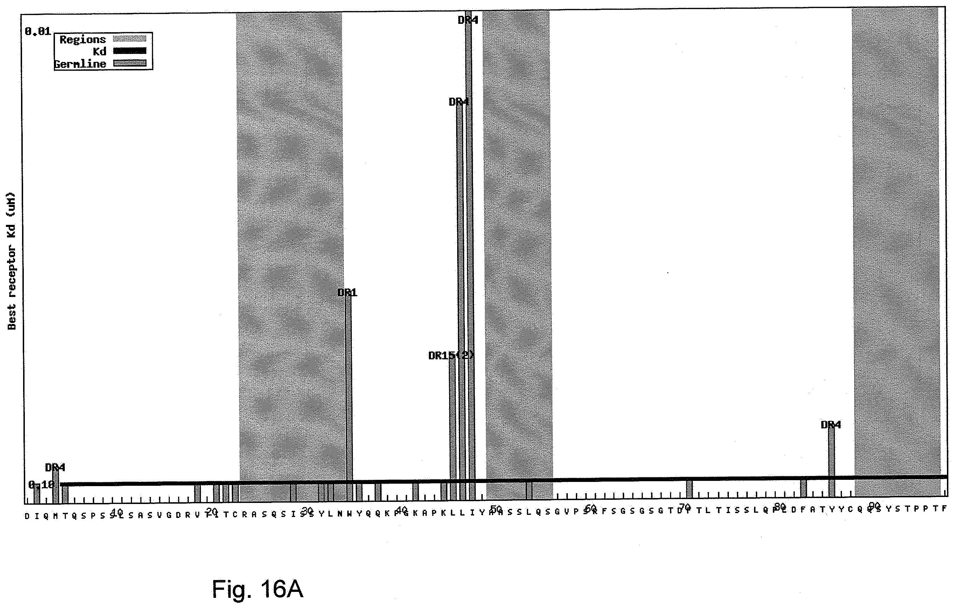

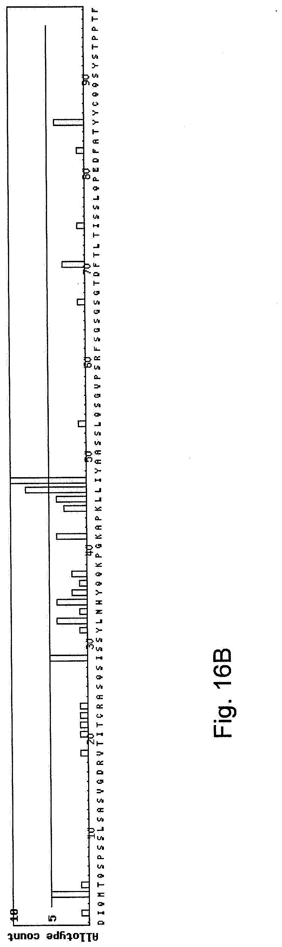

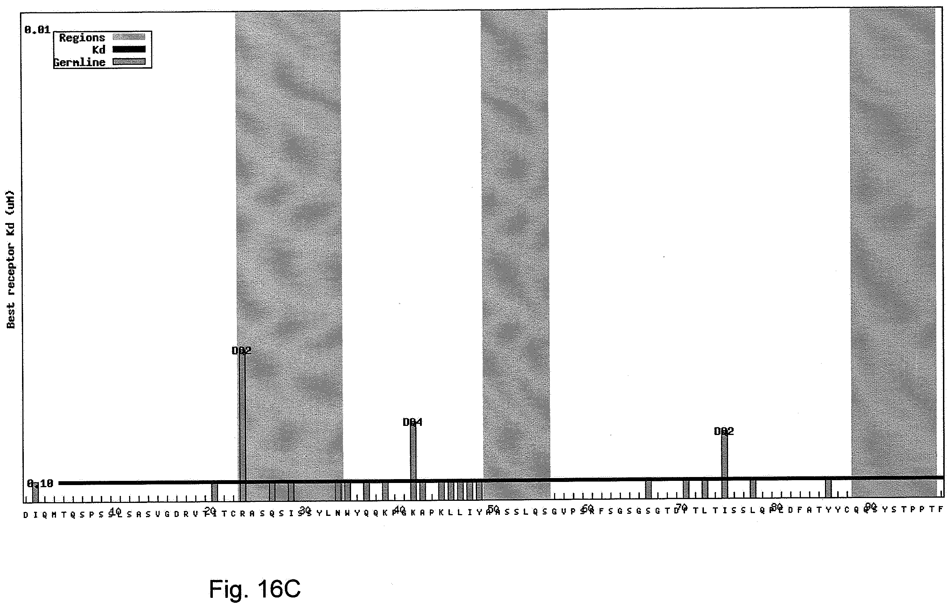

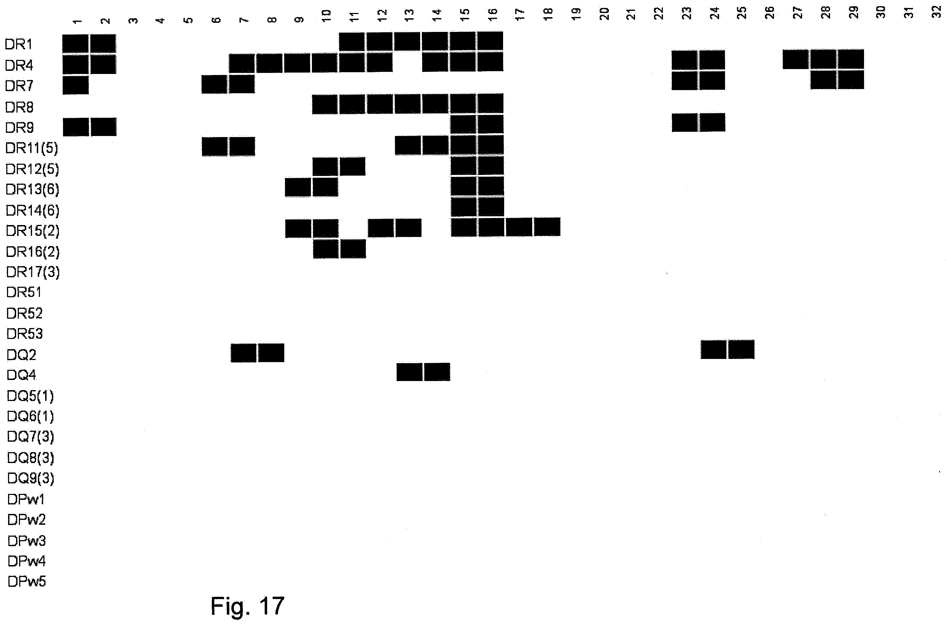

The protein sequence of IGKV1-39 (figure 12, human germline IGKV1-39/J Protein) was scanned for the presence of putative HLA class II restricted epitopes, also known as T_{H}-epitopes. For this, Algonomics' Epibase^{®} platform was applied to IGKV1-39. In short, the platform analyzes the HLA binding specificities of all possible 10-mer peptides derived from a target sequence (Desmet et al. Nature 1992, 356:539-542; Desmet et al. FASEB J. 1997, 11:164-172; Desmet et al. Proteins 2002, 48:31-43; Desmet et al. Proteins 2005, 58:53-69). Profiling is done at the allotype level for 20 DRB1, 7 DRB3/4/5, 13 DQ and 7 DP, i.e. 47 HLA class II receptors in total (see Table 5). Epibase^{®} calculates a quantitative estimate of the free energy of binding ΔG_{bind} of a peptide for each of the 47 HLA class II receptors. These data were then further processed as follows:
- Free energies were converted into Kd-values through ΔG_{bind} = RT In(Kd).
- Peptides were classified as strong (S), medium (M), weak and non (N) binders. The following cutoffs were applied:
   S: strong binder: Kd < 0.1 µM.
   M: medium binder: 0.1 µM 5 Kd < 0.8 µM.
   N: weak and non-binder: 0.8 µM ≤ Kd.
- Peptides corresponding to self-peptides were treated separately. The list of self-peptides was taken from 293 antibody germline sequences. They are referred to as 'germline-filtered' peptides.
S- and M-peptides are mapped onto the target sequence in so-called epitope maps; S-affinities are plotted quantitatively; M-values are presented qualitatively. As a general overview of the results, Table 6 lists the number of strong and medium binders in the analyzed proteins, for the groups of HLA class II receptors corresponding to the DRB1, DQ, DP and DRB3/4/5 genes. Counting was done separately for strong and medium affinity binders. Peptides binding to multiple allotypes of the same group were counted as one. Values between brackets refer to germline-filtered peptides. In Table 7, the sequence is shown in a format suitable for experimental work. The sequence is broken down in consecutive 15-mers overlapping by 12 residues. For each 15mer, the promiscuity is listed (the number of allotypes out of a total of 47 for which the 15-mer contains a critical binder), as well as the implied serotypes. The Epibase^{®} profile and epitope maps are shown in figure 16 and 17.

It was concluded that IGKV1-39 contains no strong non-self DRB1 binders. Typically, significantly more binders were found for DRB1 than for other HLA genes. This is in agreement with experimental evidence that allotypes belonging to the DRB1 group are more potent peptide binders. Medium strength epitopes for DRB1 allotypes are expected to contribute to the population response, and cannot be disregarded. Again, no non-self DRB1 binders were found in IGKV1-39.
In the humoral response raised against an antigen, the observed T_{H} cell activation/proliferation is generally interpreted in terms of the DRB1 specificity. However, one cannot ignore the possible contribution of the DRB3/4/5, DQ and DP genes. Given the lower expression levels of these genes as compared to DRB1, the focus was on the class of strong epitopes for DRB3/4/5, DQ and DP. 'Critical epitopes' are those epitopes that are strong binders for any DRB1, DRB3/4/5, DQ or DP allotype or are medium binders for DRB1, IGKV1-39 contains no strong or medium non-self binders for DRB3/4/5, DQ, or DP.

A number of peptides are also present in germline sequences (values between brackets in Table 6). Such peptides may very well bind to HLA but they are assumed to be self and, hence, non-immunogenic. In total, 6 strong and 16 medium germline-filtered DRB1 binders were found in IGKV1-39. Framework region 1 up to framework region 3 is an exact match for germline V-segment VKI 2-1-(1) 012 (VBase), a.k.a. IGKV1-39*01 (IMGT). Framework region 4 is an exact match for germline J-segment JK1 (V-base) a.k.a. IGKJ1*01(IMGT). It is hardly surprising that these segments do not contain any non-self epitopes.

### Example 20

### Production characteristics of IGKV1-39

There is a great demand for antibody discovery platforms that yield therapeutic antibodies that are thermodynamically stable and give good expression yields. These characteristics are important in ensuring the stability of the drug substance during production and after injection of the drug product into the patient. In addition good expression yields impact directly on the cost of drug manufacture and thus pricing, patient access and profitability. Virtually all therapeutic antibodies in clinical use today are composed of human IgG1 and kappa constant regions but use different heavy and light chain variable regions that confer specificity. Human variable heavy and light chain domains can be divided into families that have greater than 80% sequence divergence. When rearranged examples of these families in germline configuration are combined and compared for stability and yield it is clear that the gene families are not equal in terms of biophysical properties. In particular V_{H}3, V_{H}1 and V_{H}5 have favourable stability for the heavy chains and Vk1 and Vk3 have the best stability and yield of light chains. In addition when mutations are introduced as part of the somatic hypermutation process they can interfere with V_{H}/V_{L} pairing. To assess the effect that different light chain genes with different rates of mutation have on the production characteristics of a fixed V_{H} chain, a Fab phage display library was built of light chains (kappa and lambda) from six naive healthy donors combined with a panel of 44 TT binding heavy chains from immunized donors. After one round of selection TT binding Fab clones were isolated. Several of these shared the same V_{H} gene as the TT clone PG1433 in combination with different light chains. The Fab light chain fragments were recloned into a kappa expression vector and transfected in combination with DNA encoding the heavy chain of PG1433 into 293 cells and specific IgG production measured by ELISA. As demonstrated in table 8 the selected clones containing PG1433 V_{H} combined with different light chains had between 5 and 10 fold lower protein expression PG1433 V_{H} combined with IGKV1-39. Note that all of the light chains contained amino acid mutations within their coding regions that might disrupt V_{H} paring and reduce production stability. Thus, in addition to reducing the chances of unwanted immunogenicity, it is expected that the use of the light chain IGKV1-39 without mutations contributes to improved production stability and yields of various specificity-contributing V_{H} genes. Indeed stable clones generated by the transfection of different V_{H} genes all paired with IGKV1-39 are able to be passaged extensively and still retain robust production characteristics as shown in table 9.

### Example 21

Generation of mice expressing fully human VH and VL regions. Transgenic mice suitable for use according to the invention are crossed with mice that already contain a human VH locus. Examples of appropriate mice comprising a human VH locus are disclosed in Taylor et al. (1992). Nucleic Acids Res 20: 6287-95; Lonberg et al. (1994). Nature 368: 856-9; Green et al. (1994). Nat Genet 7: 13-21; Dechiara et al. (2009). Methods Mol Biol 530: 311-24.).
After crossing and selecting for mice that are at least heterozygous for the IGKV1-39 transgene and the human VH locus, selected mice are immunized with a target. VH genes are harvested as described hereinabove. This method has the advantage that the VH genes are already fully human and thus do not require humanization.

### Example 22

### Isolation, characterization, Oligoclonics formatting and production of antibodies targeting human IL6 for treatment of chronic inflammatory diseases such as rheumatoid arthritis

A spleen VH repertoire from transgenic mice that are immunized with human recombinant IL6 is cloned in a phage display Fab vector with a single human IGKV1-39-C kappa light chain (identical to the mouse transgene) and subjected to panning against the immunogen human IL6. Clones that are obtained after two to four rounds of panning are analyzed for their binding specificity. VH genes encoding IL6-specific Fab fragments are subjected to sequence analysis to identify unique clones and assign VH, DH and JH utilization. The Fab fragments are reformatted as IgG1 molecules and transiently expressed. Unique clones are then grouped based on non-competition in binding assays and subjected to affinity and functional analysis. The most potent anti-IL6 IgG1 mAbs are subsequently expressed as combinations of two, three, four or five heavy chains comprising different VH-regions in the Oligoclonics format, together with one IGKV1-39-C-based kappa light chain and tested in vitro for complex formation with IL-6. The Oligoclonics are also tested in *vivo* for clearance of human IL-6 from mice. An Oligoclonic with the most potent clearance activity is chosen and the murine VH genes humanized according to conventional methods. The humanized IgG 1 are transfected into a mammalian cell line to generate a stable clone. An optimal subclone is selected for the generation of a master cell bank and the generation of clinical trial material.
Many of the protocols described here are standard protocols for the construction of phage display libraries and the panning of phages for binding to an antigen of interest and are described, for example, in Antibody Phage Display: Methods and Protocols. 2002. Editor(s): Philippa M. O'Brien, Robert Aitken. Humana Press, Totowa, New Jersey, USA.

### Immunizations

Transgenic mice receive three immunizations with human IL6 every two weeks using the adjuvant Sigma titerMax according to manufacturer's instructions.

### RNA isolation and cDNA synthesis

Three days after the last immunization, spleens and lymphnodes from the mice are removed and passed through a 70 micron filter into a tube containing PBS pH 7.4 to generate a single cell suspension. After washing and pelleting of lymphocytes, cells are suspended in TRIzol LS Reagent (Invitrogen) for the isolation of total RNA according to the manufacturer's protocol and subjected to reverse transcription reaction using 1 microgram of RNA, Superscript III RT in combination with dT20 according to manufacturer' s procedures (Invitrogen).

The generation of Fab phage display libraries is carried out as described in Example 2.

### Selection of phages on coated immunotubes

Human recombinant IL6 is dissolved in PBS in a concentration of 5 µg/ml and coated to MaxiSorp Nunc-Immuno Tube (Nunc 444474) overnight at 4°C. After discarding the coating solution, the tubes are blocked with 2% skim milk (ELK) in PBS (blocking buffer) for 1 hour at Room Temperature (RT). In parallel, 0.5 ml of the phage library is mixed with 1 ml blocking buffer and incubated for 20 minutes at room temperature. After blocking the phages, the phage solution is added to the IL6 coated tubes and incubated for 2 hours at RT on a slowly rotating platform to allow binding. Next, the tubes are washed 10 times with PBS/0.05% Tween-20 followed by phage elution by incubating with 1 ml 50 mM glycine-HCl pH 2.2 10 min at RT on rotating wheel and directly followed by neutralization of the harvested eluent with 0.5 ml 1 M Tris-HCl pH 7.5.

### Harvesting phage clones

A 5 ml XL1-Blue MRF (Stratagene) culture at O.D. 0.4 is added to the harvested phage solution and incubated for 30 minutes at 37°C without shaking to allow infection of the phages. Bacteria are plated on Carbenicillin/Tetracycline 4% glucose 2*TY plates and grown overnight at 37°C.

### Phage production

Phages are grown and processed as described by Kramer et al. 2003 (Kramer et al. 2008. Nucleic Acids Res. 31(11): e59) using VCSM13 as helper phage strain.

### Phage ELISA

ELISA plates are coated with 100 microliter human recombinant IL6 per well at a concentration of 2.5 microgram/ml in PBS overnight at 4°C. Plates coated with 100 microliter thyroglobulin at a concentration of 2 microgram/ml in PBS are used as a negative control. Wells are emptied, dried by tapping on a paper towel, filled completely with PBS-4% skimmed milk (ELK) and incubated for 1 hour at room temperature to block the wells. After discarding the block solution, phage minipreps pre-mixed with 50 µl blocking solution are added and incubated for 1 hour at RT. Unbound phages are subsequently removed by 5 washing steps with PBS-0.05% Tween-20. Bound phages are detected by incubating the wells with 100 microliter anti-M13-HRP antibody conjugate (diluted 1/5000 in blocking buffer) for 1 hour at room temperature. Free antibody is removed by repeating the washing steps as described above, followed by TMB substrate incubation until color development was visible. The reaction is stopped by adding 100 microliter of 2 M H2SO4 per well and analyzed on an ELISA reader at 450 nm emission wavelength.

### Sequencing

Clones that give signals at least 3 times above the background signal are propagated, used for DNA miniprep procedures (see procedures Qiagen miniPrep manual) and subjected to nucleotide sequence analysis. Sequencing is performed according to the Big Dye 1.1 kit accompanying manual (Applied Biosystems) using a reverse primer (CH1_Rev1, table 1) recognizing a 5' sequence of the CH1 region of the human IgG1 heavy chain (present in the Fab display vector MV1043, figures 3 and 12). The sequences of the murine VH regions are analyzed for diversity of DH and JH gene segments.

### Construction and expression of chimeric IgG1

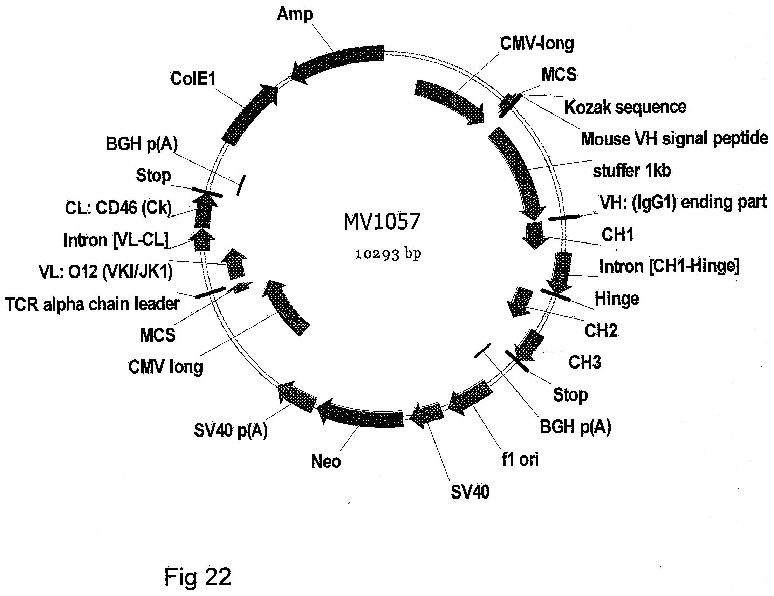

Vector MV1057 (figures 12 and 22) was generated by cloning the transgene (IGKV1-39) L chain fragment into a derivative of vector pcDNA3000Neo (Crucell, Leiden, The Netherlands) that contains the human IgG1- and kappa constant regions. VH regions are cloned into MV1057 and nucleotide sequences for all constructs are verified according to standard techniques. The resulting constructs are transiently expressed in HEK293T cells and supernatants containing chimeric IgG1 are obtained and purified using standard procedures as described before (Throsby, M. 2006. J Virol 80: 6982-92).

### IgG1 binding and competition analysis

IgG1 antibodies are titrated in ELISA using IL6 coated plates as described above and an anti-human IgG peroxidase conjugate. Competition ELISAs to group antibodies based on epitope recognition are performed by incubating Fab phages together with IgG1 or with commercial antibodies against IL6 (e.g. Abcam cat. no. ab9324) in IL6 coated plates, followed by detection of bound Fab phage using an anti-M13 peroxidase conjugate.

### IgG1 affinity measurements

The affinities of the antibodies to IL6 are determined with the Quantitative kinetic protocol on the Octet (ForteBio). Antibodies are captured onto an Anti-Human IgG Fc Capture biosensor and exposed to free IL6 and analyzed using proprietary software to calculate the Kd of each antibody.

### Functional activity of IL6 antibodies

To test the ability of the selected antibodies to inhibit binding between IL6 and IL6 receptor (IL6R), an ELISA based assay is used. Various concentrations of antibody are mixed with a fixed concentration (10 ng/ml) of biotinylated IL6 as described by Naoko et al. 2007, Can. Res. 67: 817-875. The IL6-antibody immune complex is added to immobilized IL6R. The binding of biotinylated IL6 to IL6R is detected with horseradish peroxidase-conjugated streptavidin. The reduction of ELISA signal is a measurement of inhibition. As positive control for inhibition of binding between IL6 and IL6R either anti-IL6R antibody (Abcam cat. no. ab34351; clone B-R6) or anti IL6 antibody (Abcam cat. no. ab9324) is used. *In vitro* blocking activity of the selected anti-IL6 antibodies is measured in a proliferation assay using the IL6 dependent cell line 7TD1. Briefly, cells are incubated with different concentrations of human IL6 with or without the anti-IL6 antibody. The available amount of IL6 determines the degree of proliferation. Thus if an added antibody blocks IL6 binding the proliferation readout is reduced compared to a non binding antibody control. Proliferation is measured by the incorporation of 5-bromo-2'-deoxyuridine (BrdU) into the DNA using the BrdU proliferation kit (Roche cat. no. 11444611001) according to the manufacturer's instructions.

### Generation of anti-IL6 Oligoclonics

The most potent anti-IL6 antibodies are selected from each epitope group. The expression constructs expressing these antibodies are transfected into HEK293T cells in non-competing groups of three in different ratios (1:1:1; 3:1:1; 1:3:1; 1:1:3; 3:3:1; 1:3:3; 3:1:3; 10:1:1; 1:10:1; 1:1:10; 10:10:1; 1:10:10; 10:1:10; 3:10:1; 10:3:1; 1:10:3; 3:1:10; 10:1:3; 1:3:10). Antibody containing supernatants are harvested and purified and characterized as above.

### Complex formation and in vivo clearance of anti-IL6 Oligoclonics

To measure the ability of anti-IL6 Oligoclonics to form immune complexes and to analyze these complexes Size Exclusion Chromatography (SEC) is used according to the approach disclosed by Min-Soo Kim et al. (2007) JMB 374: 1374-1388 to characterize the immune-complexes formed with different antibodies to TNFa. Different molar ratios of the anti-IL6 Oligoclonics are mixed with human IL6 and incubated for 20 hours at 4°C or 25°C. The mixture is analyzed on an HPLC system fitted with a size exclusion column; different elution times are correlated to molecular weight using a molecular weight standards.
The ability of antibodies to form complexes with IL6 is correlated with their ability to rapidly clear the cytokine from the circulation in vivo. This is confirmed by measuring the clearance of radiolabelled IL6 from mice. Briefly, female, 6- to 8-week-old Balb/c mice are obtained and 18 hrs before the experiment, the animals are injected intravenously (IV) via the lateral tail vein with different doses of purified anti-IL6 Oligoclonics. On day 0 the mice are
injected IV with 50 microliters of radiolabeled IL-6 (1x10E7 cpm/mL) under the same conditions. Blood samples (approximately 50 microliters) are collected at several time intervals and stored at 4°C. The samples are centrifuged for 5 minutes at 4000 xg and the radioactivity of the serum determined. All pharmacokinetic experiments are performed simultaneously with three animals for each treatment.

### Generation of anti-IL6 Oligoclonics stable clones and preclinical development

A lead anti-IL6 Oligoclonic is selected based on the in *vitro* and in vivo potency as determined above. The murine VH genes are humanized according to standard methods and combined with the fully human IGKV1-39 light chain in an expression vector as described above. Examples of humanization methods include those based on paradigms such as resurfacing (Padlan, E. A., et al., (1991). Mol. Immunol., 28, 489), superhumanization (Tan, P., D. A., et al., (2002) J. Immunol., 169, 1119) and human string content optimization (Lazar, G. A., et al., (2007). Mol. Immunol., 44, 1986). The three constructs are transfected into PER.C6 cells at the predetermined optimal ratio (described above) under the selective pressure of G418 according to standard methods. A stable high producing anti-IL6 Oligoclonic clone is selected and a working and qualified master cell bank generated.

**Table 1 List of primers**

| **DO-** | **Primer** | **Sequence** |
|---|---|---|
| **0012** | **CH1_Rev1** | **TGCCAGGGGGAAGACCGATG** |
| **0656** | **MVH-1** | **GCCGGCCATGGCCGAGGTRMAGCTTCAGGAGTCAGGAC** |
| **0657** | **MVH-2** | **GCCGGCCATGGCCGAGGTSCAGCTKCAGCAGTCAGGAC** |
| **0656** | **MVH-3** | **GCCGGCCATGGCCCAGGTGCAGCTGAAGSASTCAGG** |
| **0659** | **MVH-4** | **GCCGGCCATGGCCGAGGTGCAGCTTCAGGAGTCSGGAC** |
| **0660** | **MVH-5** | **GCCGGCCATGGCCGARGTCCAGCTGCAACAGTCYGGAC** |
| **0661** | **MVH-6** | **GCCGGCCATGGCCCAGGTCCAGCTKCAGCAATCTGG** |
| **0662** | **MVH-7** | **GCCGGCCATGGCCCAGSTBCAGCTGCAGCAGTCTGG** |
| **0663** | **MVH-8** | **GCCGGCCATGGCCCAGGTYCAGCTGCAGCAGTCTGGRC** |
| **0664** | **MVH-9** | **GCCGGCCATGGCCCAGGTYCAGCTYCAGCAGTCTGG** |
| **0665** | **MVH-10** | **GCCGGCCATGGCCGAGGTCCARCTGCAACAATCTGGACC** |
| **0666** | **MVH-11** | **GCCGGCCATGGCCCAGGTCCACGTGAAGCAGTCTGGG** |
| **0667** | **MVH-12** | **GCCGGCCATGGCCGAGGTCAASSTGGTGGAATCTG** |
| **0668** | **MVH-13** | **GCCGGCCATGGCCGAVGTGAAGYTGGTGGAGTCTG** |
| **0669** | **MVH-14** | **GCCGGCCATGGCCGAGGTGCAGSKGGTGGAGTCTGGGG** |
| **0670** | **MVH-15** | **GCCGGCCATGGCCGAKGTGCAMCTGGTGGAGTCTGGG** |
| **0671** | **MVH-16** | **GCCGGCCATGGCCGAGGTGAAGCTGATGGARTCTGG** |
| **0672** | **MVH-17** | **GCCGGCCATGGCCGAGGTGCARCTTGTTGAGTCTGGTG** |
| **0673** | **MVH-18** | **GCCGGCCATGGCCGARGTRAAGCTTCTCGAGTCTGGA** |
| **0674** | **MVH-19** | **GCCGGCCATGGCCGAAGTGAARSTTGAGGAGTCTGG** |
| **0675** | **MVH-20** | **GCCGGCCATGGCCGAAGTGATGCTGGTGGAGTCTGGG** |
| **0676** | **MVH-21** | **GCCGGCCATGGCCCAGGTTACTCTRAAAGWGTSTGGCC** |
| **0677** | **MVH-22** | **GCCGGCCATGGCCCAGGTCCAACTVCAGCARCCTGG** |
| **0678** | **MVH-23** | **GCCGGCCATGGCCCAGGTYCARCTGCAGCAGTCTG** |
| **0679** | **MVH-24** | **GCCGGCCATGGCCGATGTGAACTTGGAAGTGTCTGG** |
| **0680** | **MVH-25** | **GCCGGCCATGGCCGAGGTGAAGGTCATCGAGTCTGG** |
| **0681** | **ExtMVH-1** | **CAGTCACAGATCCTCGCGAATT*GGCCCAGCCGGCC*ATGGCCSANG** |
| **0682** | **ExtMVH-2** | **CAGTCACAGATCCTCGCGAATT*GGCCCAGCCGGCC*ATGGCCSANC** |
| **0683** | **MIH-Rev1** | **GGGGGTGTCGTTTTGGCTGAGGAGAC *GGTGACC* GTGG** |
| **0684** | **MIH-Rev2** | **GGGGGTGTCGTTTTGGCTGAGGAGAC *TGTGAGA* GTGG** |
| **0685** | **MIH-Rev3** | **GGGGGTGTCGTTTTGGMGCAGAGAC *AGTGACC* AGAG** |
| **0686** | **MIH-Rev4** | **GGGGGTGTCGTTTTGGCTGAGGAGAC *GGTGACT* GAGG** |
| **0687** | **ExtMJH-** | **GGGGGTGTCGTTTTGGCTGAGGAGAC *GGTGACC* GTGG** |
| **0688** | **ExtMJH-** | **GGGGGTGTCGTTTTGGCTGAGGAGAC *GGTGACA* GTGG** |
| **0690** | **ExtMJH-** | **GGGGGTGTCGTTTTGGCTGAGGAGAC *GGTGACC* AGAG** |
| **0691** | **ExtMJH-** | **GGGGGTGTCGTTTTGGCTGAGGAGAC *GGTGACC* GAGG** |

**Table 2**

| Phage ELISA signal levels as measured at 450nm. TT-coated plates represent plates that were coated with tetanus toxoid. Thyroglobulin coated plates are used as negative controls. 10/10 and 15/15 indicate the number of wash steps with PBS-Tween during panning procedures. The 10/10 tetanus toxoid and 10/10 thyroglobulin plates and the 15/15 tetanus toxoid and 15/15 thyroglobulin plates are duplicates from each other except for the coating agent. OD values higher than 3 times the background are assumed specific. | | | | | | | | | | | | |
|---|---|---|---|---|---|---|---|---|---|---|---|---|
| **TT-coated plate 10/10 washings** | | | | | | | | | | | | |
| | **1** | **2** | **3** | **4** | **5** | **6** | **7** | **8** | **9** | **10** | **11** | **12** |
| **A** | 0.139 | 0.093 | 0.089 | 0.121 | 0.117 | 0.598 | 0.146 | 0.115 | 0.18 | 0.155 | 0.543 | **0.601** |
| **B** | 0.136 | 0.404 | 0.159 | 0.187 | 0.489 | 0.134 | 0.216 | 0.092 | 0.222 | 0.108 | 0.181 | **0.484** |
| **C** | 0.197 | 0.526 | 0.09 | 0.213 | 0.395 | 0.155 | 0.108 | 0.12 | 0.183 | 0.136 | 0.092 | **0.866** |
| **D** | 0.143 | 0.258 | 0.101 | 0.422 | 0.088 | 0.243 | 0.485 | 0.251 | 0.304 | 0.198 | 0.478 | **0.091** |
| **E** | 0.445 | 0.169 | 0.526 | 0.481 | 0.206 | 0.285 | 0.111 | 0.119 | 0.128 | 0.2 | 0.118 | **0.098** |
| **F** | 0.237 | 0.291 | 0.594 | 0.139 | 0.206 | 0.565 | 0.543 | 0.091 | 0.136 | 0.227 | 0.228 | **0.099** |
| **G** | 0.459 | 0.102 | 0.152 | 0.659 | 0.203 | 0.452 | 0.152 | 0.133 | 0.094 | 0.102 | 0.375 | 0.098 |
| **H** | 0.341 | 0.623 | 0.745 | 0.415 | 0.682 | 0.527 | 0.655 | 0.114 | 0.258 | 0.284 | 0.685 | 0.113 |

| **TT-coated plate 15/15 washings** | | | | | | | | | | | | |
|---|---|---|---|---|---|---|---|---|---|---|---|---|
| | **1** | **2** | **3** | **4** | **5** | **6** | **7** | **8** | **9** | **10** | **11** | **12** |
| **A** | 0.247 | 0.582 | 0.421 | 0.428 | 0.133 | 0.082 | 0.262 | 0.079 | 0.343 | 0.414 | 0.095 | **0.292** |
| **B** | 0.065 | 0.364 | 0.073 | 0.042 | 0.049 | 0.071 | 0.046 | 0.103 | 0.078 | 0.057 | 0.048 | **0.155** |
| **C** | 0.081 | 0.044 | 0.066 | 0.082 | 0.225 | 0.444 | 0.203 | 0.362 | 0.122 | 0.047 | 0.052 | **0.309** |
| **D** | 0.092 | 0.11 | 0.59 | 0.22 | 0.33 | 0.544 | 0.058 | 0.159 | 0.047 | 0.174 | 0.086 | **0.05** |
| **E** | 0.469 | 0.577 | 0.206 | 0.304 | 0.13 | 0.749 | 0.431 | 0.062 | 0.167 | 0.049 | 0.056 | **0.049** |
| **F** | 0.846 | 0.07 | 0.561 | 0.656 | 0.882 | 0.094 | 0.383 | 0.13 | 0.152 | 0.098 | 0.134 | **0.048** |
| **G** | 0.537 | 0.052 | 0.49 | 0.105 | 0.337 | 0.193 | 0.514 | 0.294 | 0.068 | 0.35 | 0.525 | 0.05 |
| **H** | 0.061 | 0.306 | 0.157 | 0.853 | 0.054 | 0.534 | 0.102 | 0.235 | 0.441 | 0.412 | 0.565 | 0.061 |

| **Thyroglobulin-coated plate 10/10 washings** | | | | | | | | | | | | |
|---|---|---|---|---|---|---|---|---|---|---|---|---|
| | **1** | **2** | **3** | **4** | **5** | **6** | **7** | **8** | **9** | **10** | **11** | **12** |
| **A** | 0.047 | 0.051 | 0.045 | 0.043 | 0.051 | 0.044 | 0.046 | 0.042 | 0.047 | 0.048 | 0.049 | **0.05** |
| **B** | 0.042 | 0.042 | 0.042 | 0.042 | 0.043 | 0.041 | 0.041 | 0.042 | 0.043 | 0.045 | 0.042 | **0.046** |
| **C** | 0.044 | 0.043 | 0.043 | 0.044 | 0.043 | 0.044 | 0.043 | 0.042 | 0.043 | 0.041 | 0.044 | **0.046** |
| **D** | 0.045 | 0.044 | 0.044 | 0.044 | 0.045 | 0.046 | 0.045 | 0.056 | 0.045 | 0.049 | 0.048 | **0.73** |
| **E** | 0.046 | 0.045 | 0.046 | 0.044 | 0.045 | 0.044 | 0.044 | 0.044 | 0.047 | 0.046 | 0.047 | **0.926** |
| **F** | 0.048 | 0.045 | 0.044 | 0.046 | 0.044 | 0.043 | 0.044 | 0.046 | 0.046 | 0.046 | 0.046 | **0.792** |
| **G** | 0.051 | 0.048 | 0.045 | 0.045 | 0.044 | 0.043 | 0.048 | 0.045 | 0.048 | 0.051 | 0.045 | 0.053 |
| **H** | 0.064 | 0.05 | 0.049 | 0.047 | 0.05 | 0.051 | 0.047 | 0.046 | 0.047 | 0.047 | 0.047 | 0.056 |

| **Thyroglobulin-coated plate 15/15 washings** | | | | | | | | | | | | |
|---|---|---|---|---|---|---|---|---|---|---|---|---|
| | **1** | **2** | **3** | **4** | **5** | **6** | **7** | **8** | **9** | **10** | **11** | **12** |
| **A** | 0.036 | 0.049 | 0.045 | 0.044 | 0.046 | 0.047 | 0.046 | 0.042 | 0.042 | 0.043 | 0.042 | **0.041** |
| **B** | 0.045 | 0.042 | 0.041 | 0.043 | 0.043 | 0.043 | 0.045 | 0.045 | 0.047 | 0.048 | 0.044 | **0.045** |
| **C** | 0.049 | 0.047 | 0.047 | 0.046 | 0.046 | 0.046 | 0.045 | 0.047 | 0.046 | 0.045 | 0.045 | **0.052** |
| **D** | 0.047 | 0.049 | 0.048 | 0.048 | 0.048 | 0.048 | 0.047 | 0.052 | 0.048 | 0.046 | 0.048 | **0.456** |
| **E** | 0.049 | 0.047 | 0.047 | 0.047 | 0.047 | 0.049 | 0.047 | 0.048 | 0.047 | 0.046 | 0.048 | **0.412** |
| **F** | 0.05 | 0.047 | 0.046 | 0.046 | 0.046 | 0.046 | 0.046 | 0.046 | 0.046 | 0.047 | 0.048 | **0.528** |
| **G** | 0.05 | 0.048 | 0.045 | 0.045 | 0.046 | 0.049 | 0.048 | 0.046 | 0.053 | 0.049 | 0.05 | 0.057 |
| **H** | 0.057 | 0.05 | 0.046 | 0.045 | 0.047 | 0.049 | 0.047 | 0.047 | 0.046 | 0.047 | 0.053 | 0.048 |

**Table 3**

| Protein sequence analysis of ELISA positive tetanus toxoid binders. CDR3 sequence, CDR3 length, VH family members and specific name, JH origin and DH origin of the clones is indicated. | | | | | |
|---|---|---|---|---|---|
| **CDR3** | **CDR3 length** | **VH** | **DH** | **JH** | **V Gene family** |
| HGAYYTYDEKAWFAY | 15 | musIGHV192 | DSP2.11 | JH3 mouse | VH7183 |
| HGAYYTYDEKAWFAY | 15 | musIGHV192 | DSP2.11 | JH3 mouse | VH7183 |
| HGAYYTYDEKAWFAY | 15 | musIGHV192 | DSP2.11 | JH3 mouse | VH7183 |
| HGAYYTYDEKAWFAY | 15 | musIGHV192 | DSP2.11 | JH3 mouse | VH7183 |
| HGAYYTYDEKAWFAY | 15 | musIGHV192 | DSP2.11 | JH3 mouse | VH7183 |
| HGAYYTYDEKAWFAY | 15 | musIGHV192 | DSP2.11 | JH3 mouse | VH7183 |
| HGAYYTYDEKAWFAY | 15 | musIGHV192 | DSP2.11 | JH3 mouse | VH7183 |
| HGAYYTYDEKAWFAY | 15 | musIGHV192 | DSP2.11 | JH3 mouse | VH7183 |
| HGAYYTYDEKAWFAY | 15 | musIGHV192 | DSP2.11 | JH3 mouse | VH7183 |
| HGAFYTYDEKPWFAY | 15 | muslGHV192 | IGHD2-14*01 | JH3 mouse | VH7183 |
| HISYYRYDEEVSFAY | 15 | musIGHV192 | IGHD2-14*01 | JH3 mouse | VH7183 |
| HISYYRYDEEVSFAY | 15 | musIGHV192 | IGHD2-14*01 | JH3 mouse | VH7183 |
| GWRAFAY | 7 | musIGHV131 | DSP2.9 | JH3 mouse | VH7183 |
| GWRAFAY | 7 | musIGHV131 | DSP2.9 | JH3 mouse | VH7183 |
| GWRAFAY | 7 | muslGHV131 | DSP2.9 | JH3 mouse | VH7183 |
| DRGNYYGMDY | 10 | musIGHV178 | DSP2.1 | JH4 mouse | VH7183 |
| LGDYYVDWFFAV | 12 | musIGHV165 | DFL16.1 | JH1 mouse | VH7183 |
| NFPAWFAF | 8 | musIGHV547 | DST4.3inv | JH3 mouse | VJH558 |
| NFPAWFAY | 8 | musIGHV547 | DSP2.1 | JH3 mouse | VJH558 |
| NFPAWFVY | 8 | muslGHV547 | DSP2.1 | JH3 mouse | VJH558 |
| SFTPVPFYYGYDWYFDV | 17 | musIGHV532 | DSP2.3 | JH1 mouse | VJH558 |
| SFTPVPFYYGYDWYFDV | 17 | musIGHV532 | DSP2.3 | JH1 mouse | VJH558 |
| SDYDWYFDV | 9 | musIGHV286 | DSP2.2 | JH1 mouse | VJH558 |
| SDYDWYFDV | 9 | musIGHV286 | DSP2.2 | JH1 mouse | VJH558 |
| DSKWAYYFDY | 10 | musIGHV532 | DST4.3 | JH2 mouse | VJH558 |
| GDYTGYGMDY | 10 | musIGHV125 | DSP2.13 | JH4 mouse | VHSM7 |
| GDYTGYGMDY | 10 | musIGHV125 | DSP2.13 | JH4 mouse | VHSM7 |
| GGYDGYWFPY | 10 | musIGHV125 | DSP2.9 | JH3 mouse | VHSM7 |

**Table 4**

| Vector combinations that were transfected to HEK293T. | | | | | |
|---|---|---|---|---|---|
| **Code** | **HC vector** | **LC vector** | **Combined vector** | **Prep name** | **Conc. (µg/ml)** |
| **A** | **×** | 0817676_pSELECT_ 0815426 (1GKV1-39) | **×** | PIGKV1-391 P1 | - |
| B | **×** | 0817678_pSELECT_ 0815427 (IGLV2-14) | **×** | PIGLV2-14/ P1 | - |
| C | MV1110 | 0817676_pSELECT_ 0815426 (IGKV1-39) | **×** | PMV1110/ IGKV1-39/P1 | 11.0 |
| D | MV1110 | 0817678_pSELECT_ 0815427 (IGLV2-14) | **×** | PMV1110/ IGLV2-14/P1 | 15.4 |
| E | **×** | × | MG1494 | MG1494/P2 | 16.1 |

**Table 5. HLA allotypes considered in T_{H}-epitope profiling. The corresponding serotypes are shown, as well as allotype frequencies in the Caucasian population (Klitz et al. Tissue Antigens 2003, 62:296-307; Gjertson and Terasake (eds) in: HLA 1997; Gjertson and Terasake (eds) in: HLA 1998; Castelli et al. J. Immunol. 2002, 169:6928-6934). Frequencies can add up to more than 100% since each individual has 2 alleles for each gene. If all allele frequencies of a single gene were known, they would add up to slightly less than 200% due to homozygous individuals.**

| HLA type | Serotype | Population % | HLA type | Serotype | Population % |
|---|---|---|---|---|---|
| DRB1*0101 | DR1 | 17.4 | DRB4*0103 | DR53 | 21 |
| DRB1*0102 | DR1 | 4.9 | DRB5*0101 | DR51 | 15.8 |
| DRB1*0301 | DR17(3) | 21.2 | DRB5*0202 | DR51 | 5.7 |
| DRB1*0401 | DR4 | 11.5 | DQA1*0101/DQB1*0501 | DQ5(1) | 20.5 |
| DRB1*0402 | DR4 | 3.1 | DQA1*0102/DQB1*0502 | DQ5(1) | 2.6 |
| DRB1*0404 | DR4 | 5.5 | DQA1*0102/DQB1*0602 | DQ6(1) | 26.5 |
| DRB1*0405 | DR4 | 2.2 | DQA1*0102/DQB1*0604 | DQ6(1) | 6.7 |
| DRB1*0407 | DR4 | <2 | DQA1*0103/DQB1*0603 | DQ6(1) | 11 |
| DRB1*0701 | DR7 | 23.4 | DQA1*0104/DQB1*0503 | DQ5(1) | 4 |
| DRB1*0801 | DR8 | 3.3 | DQA1*0201/DQB1*0202 | DQ2 | 20.9 |
| DRB1*0802 | DR8 | <2 | DQA1*0201/DQB1*0303 | DQ9(3) | 7.2 |
| DRB1*0901 | DR9 | <2 | DQA1*0301/DQB1*0301 | DQ7(3) | 12.5 |
| DRB1*1101 | DR11(5) | 17 | DQA1*0301/DQB1*0302 | DQ8(3) | 18.3 |
| DRB1*1104 | DR11(5) | 5.7 | DQA1*0401/DQB1*0402 | DQ4 | 4.5 |
| DRB1*1201 | DR12(5) | 3.1 | DQA1*0501/DQBL*0201 | DQ2 | 24.6 |
| DRB1*1301 | DR13(6) | 15.4 | DQA1*0501/DQB1*0301 | DQ7(3) | 20.9 |
| DRB1*1302 | DR13(6) | 10.8 | DPA1*0103/DPB1*0201 | DPw2 | 19.9 |
| DRB1*1401 | DR14(6) | 4.2 | DPA1*0103/DPB1*0401 | DPw4 | 65.1 |
| DRB1*1501 | DR15(2) | 13.2 | DPA1*0103/DPB1*0402 | DPw4 | 24.3 |
| DRB1*1601 | DR16(2) | 5.5 | DPA1*0201/DPB1*0101 | DPw1 | 6.3 |
| DRB3*0101 | DR52 | 24.6 | DPA1*0201/DPB1*0301 | DPw3 | <2 |
| DRB3*0202 | DR52 | 43 | DPA1*0201/DPB1*0501 | DPw5 | <2 |
| DRB3*0301 | DR52 | 10 | DPA1*0201/DPB1*0901 | - | 2.4 |
| DRB4*0101 | DR53 | 25.5 | | | |

**Table 6. T_{H} epitope counts for IGKV1-39. Peptides binding to multiple HLAs of the same group (DRB1, DRB3/4/5, DP, DQ) are counted as one. Values between brackets refer to germline-filtered peptides.**

| | DRB1 | | DRB3/4/5 | | DQ | | DP | |
|---|---|---|---|---|---|---|---|---|
| | Strong | Medium | Strong | Medium | Strong | Medium | Strong | Medium |
| Merus IGEKV1-39 | 0 (+6) | 0 (+16) | 0 (+0) | 0 (+5) | 0 (+3) | 0 (+9) | 0 (+0) | 0 (+9) |

**Table 7. Mapping of Epibase^{®} predictions for Merus IGKV1-39 in the classical 15-mer peptide format. This table shows the allotype count of critical epitopes and implicated serotypes for each of the 15-mers spanning the Merus IGKV1-39 sequence.**

| 15mer | Start position | 15-mer sequence | Allotype count | Implicated serotypes |
|---|---|---|---|---|
| 1 | 1 | DIQMTOSPSSLSASV | 6 | DR1, DR4, DR7, DR9 |
| 2 | 4 | MTQSPSSLSASVGDR | 5 | DR1, DR4, DR9 |
| 3 | 7 | SPSSLSASVGDRVTI | 0 | |
| 4 | 10 | SLSASVGDRVTITCR | 0 | |
| 5 | 13 | ASVGDRVTITCRASQ | 0 | |
| 6 | 16 | GDRVTITCRASQSIS | 2 | DR11(5), DR7 |
| 7 | 19 | VTITCFASQSISSYL | 4 | DQ2, DR11(5), DR4, DR7 |
| 8 | 22 | TCRASQSISSYLNWY | 2 | DQ2, DR4 |
| 9 | 25 | ASQSISSYLNWYQQK | 5 | DR13(6), DR15(2), DR4 |
| 10 | 28 | SISSYLNWYQQKPGK | 8 | DR12(5), DR13(6), DR15(2), DR16(2), DR4, DR8 |
| 11 | 31 | SYLNWYQQKPGKAPK | 10 | DR1, DR12(5), DR16(2), DR4, DR51, DR8 |
| 12 | 34 | NWYQQKPGRAPKLLI | 9 | DR1, DR15(2), DR4, DR51, DR8 |
| 13 | 37 | QQKPGKAPKLLIYAA | 7 | DQ4, DR1, DR11(5), DR15(2), DR51, DR8 |
| 14 | 40 | PGKAPKLLIYAASSL | 7 | DQ4, DR1, DR11(5), DR4, DR8 |
| 15 | 43 | APKLLIYAASSLQSG | 15 | DR1, DR11(5), DR12(5), DR13(6), DR14(6), DR15(2), DR4, DR51, DR8, DR9 |
| 16 | 46 | LLIYAASSLQSGVPS | 15 | DR1, DR11(5), DR12(5), DR13(6), DR14(6), DR15(2), DR4, DR51, DR8, DR9 |
| 17 | 49 | YAASSLQSGVPSRFS | 1 | DR15(2) |
| 18 | 52 | SSLOSGVPSRFSGSG | 1 | DR15(2) |
| 19 | 55 | QSGVPSRFSGSGSGT | 0 | |
| 20 | 58 | VPSRFSGSGSGTDFT | 0 | |
| 21 | 61 | RFSGSGSGTDFTLTI | 0 | |
| 22 | 64 | GSGSGTDFTLTISSL | 1 | DR52 |
| 23 | 67 | SGTDFTLTISSLQPE | 4 | DR4, DR52, DR7, DR9 |
| 24 | 70 | DFTLTISSLQPEDFA | 4 | DQ2, DR4, DR7, DR9 |
| 25 | 73 | LTISSLQPEDFATYY | 1 | DQ2 |
| 26 | 76 | SSLQPEDFATYYCQQ | 0 | |
| 27 | 79 | QPEDFATYYCQQSYS | 1 | DR4 |
| 28 | 82 | DFATYYCQQSYSTPP | 5 | DR4, DR51, DR7 |
| 29 | 85 | TYYCQQSYSTPPTFG | 4 | DR4, DR51, DR7 |
| 30 | 88 | CQQSYSTPPTFGQGT | 0 | |
| 31 | 91 | SYSTPPTFGQGTKVE | 0 | |
| 32 | 94 | TPPTFGQGTKVEIK | 0 | |

**Table 8: The V_{H} gene from PG1433 paired with various light chain genes with differing rates of amino acid mutation were compared for production levels with the original clone containing the IGKV1-39 gene.**

| **IgG name** | **Light chain gene** | **Number of amino acid mutations** | **concentration (µg/ml)** |
|---|---|---|---|
| ***PG1433*** | ***1-39*** | ***0*** | **63, 45.5, 38.6 *(avg* = *49)*** |
| **PG1631** | **1-12** | **4** | **10.5** |
| **PG1632** | **1-27** | **7** | **9.3** |
| **PG1634** | **1D-12** | **10** | **10.8** |
| **PG1635** | **10-33** | **6** | **10.2** |
| **PG1642** | **1-5** | **8** | **7.1** |
| **PG1644** | **1-9** | **3** | **7.8** |
| **PG1650** | **10-39** | **3** | **9.1** |
| **PG1652** | **2D-28** | **3** | **7.1** |
| **PG1653** | **3-15** | **14** | **7** |
| **PG1654** | **3-20** | **2** | **5.2** |
| **PG1674** | **1-40** | **7** | **8.2** |
| **PG1678** | **2-11** | **2** | **8.1** |
| **PG1680** | **2-14** | **15** | **10.8** |
| **PG1682** | **3-1** | **13** | **9.9** |
| **PG1683** | **6-57** | **6** | **13.9** |

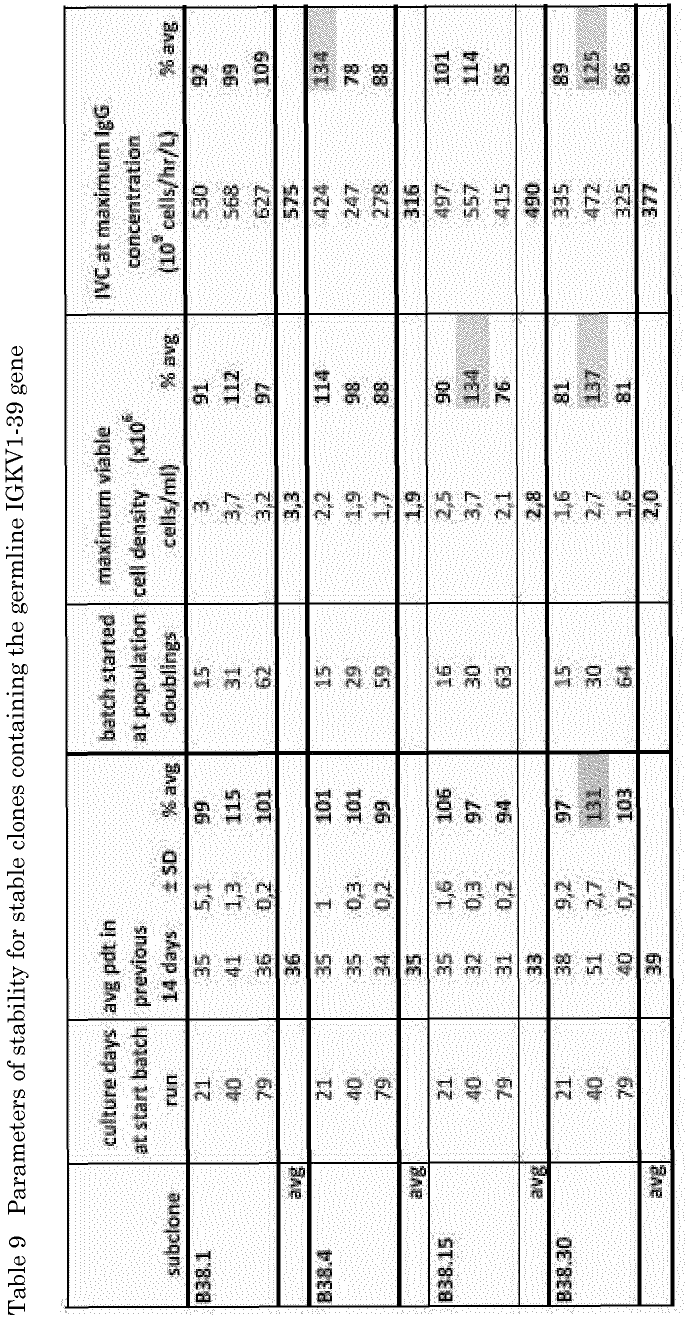

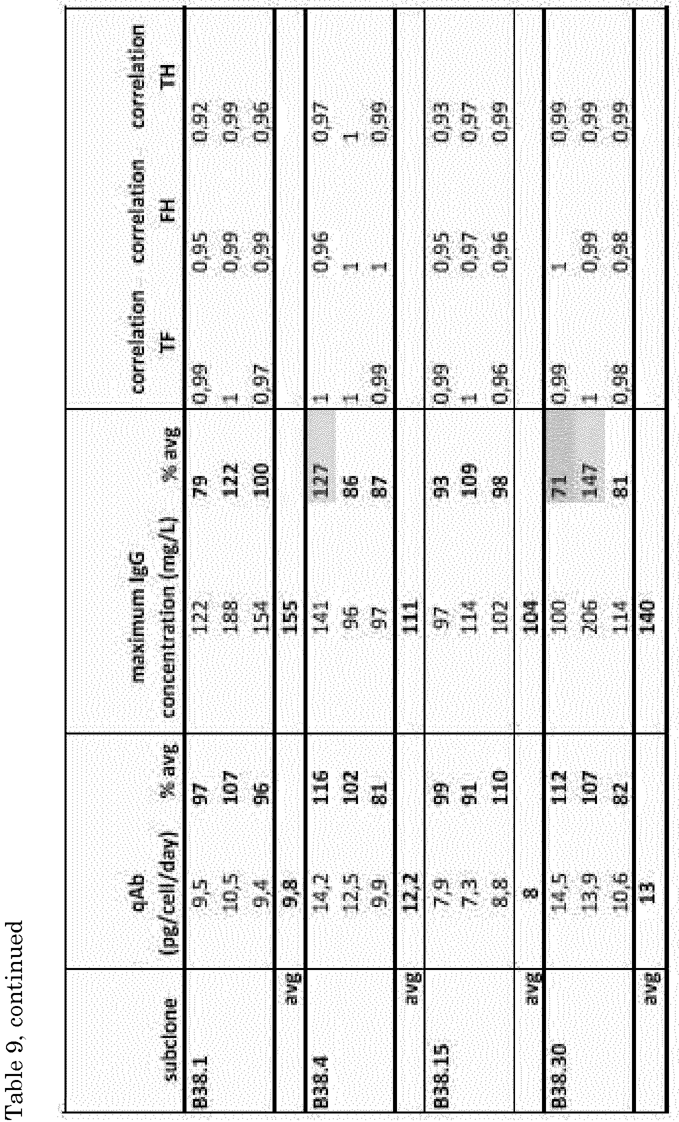

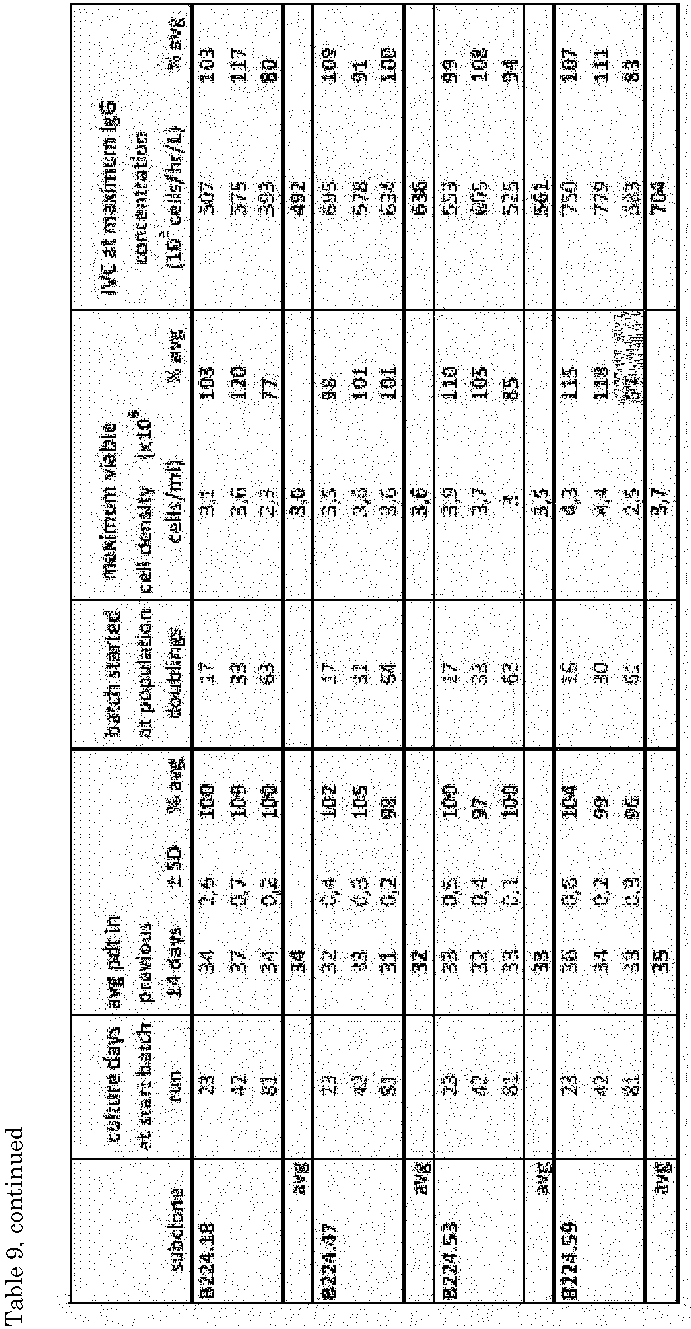

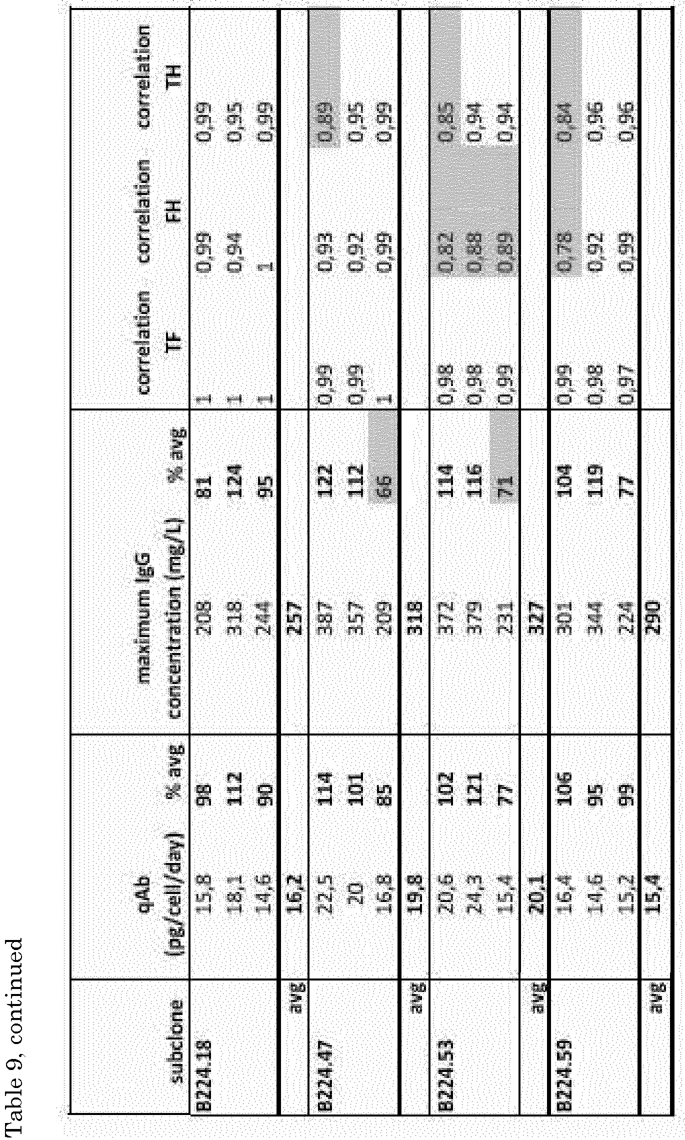

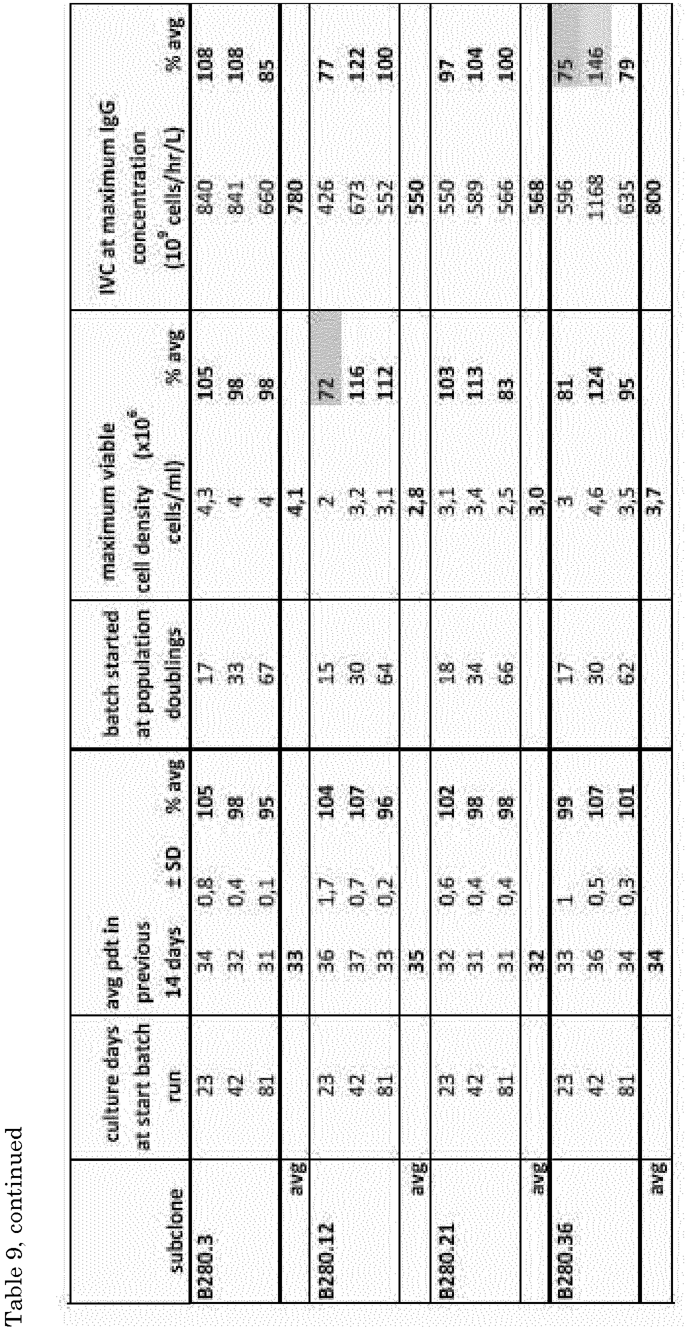

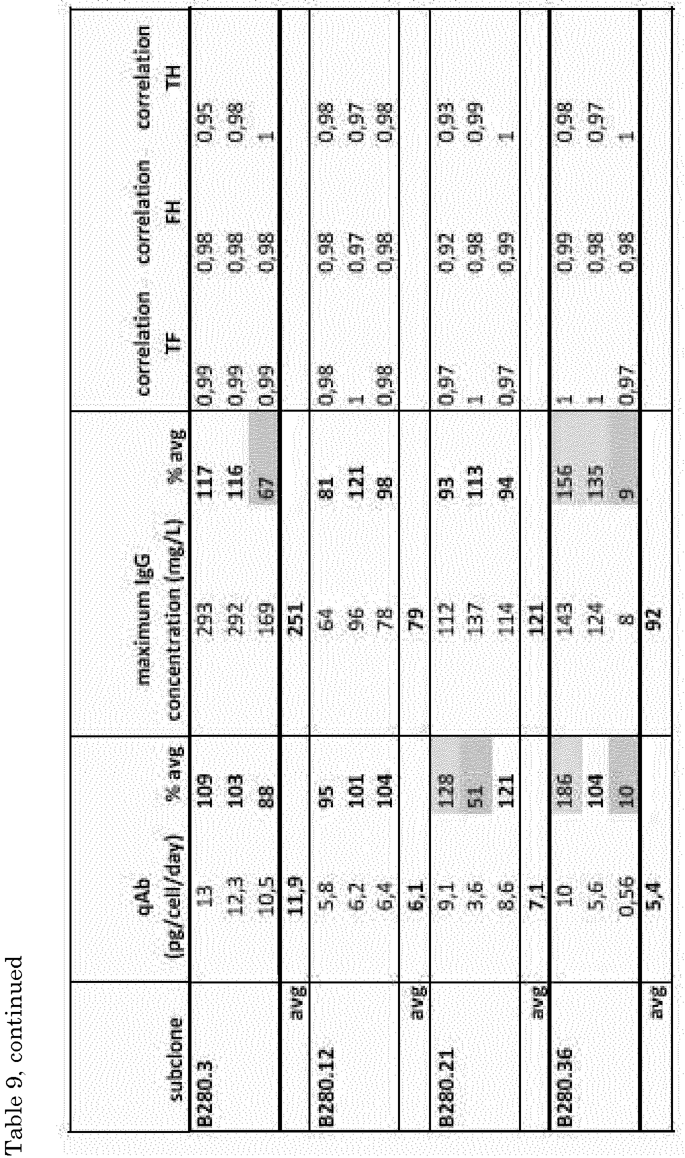

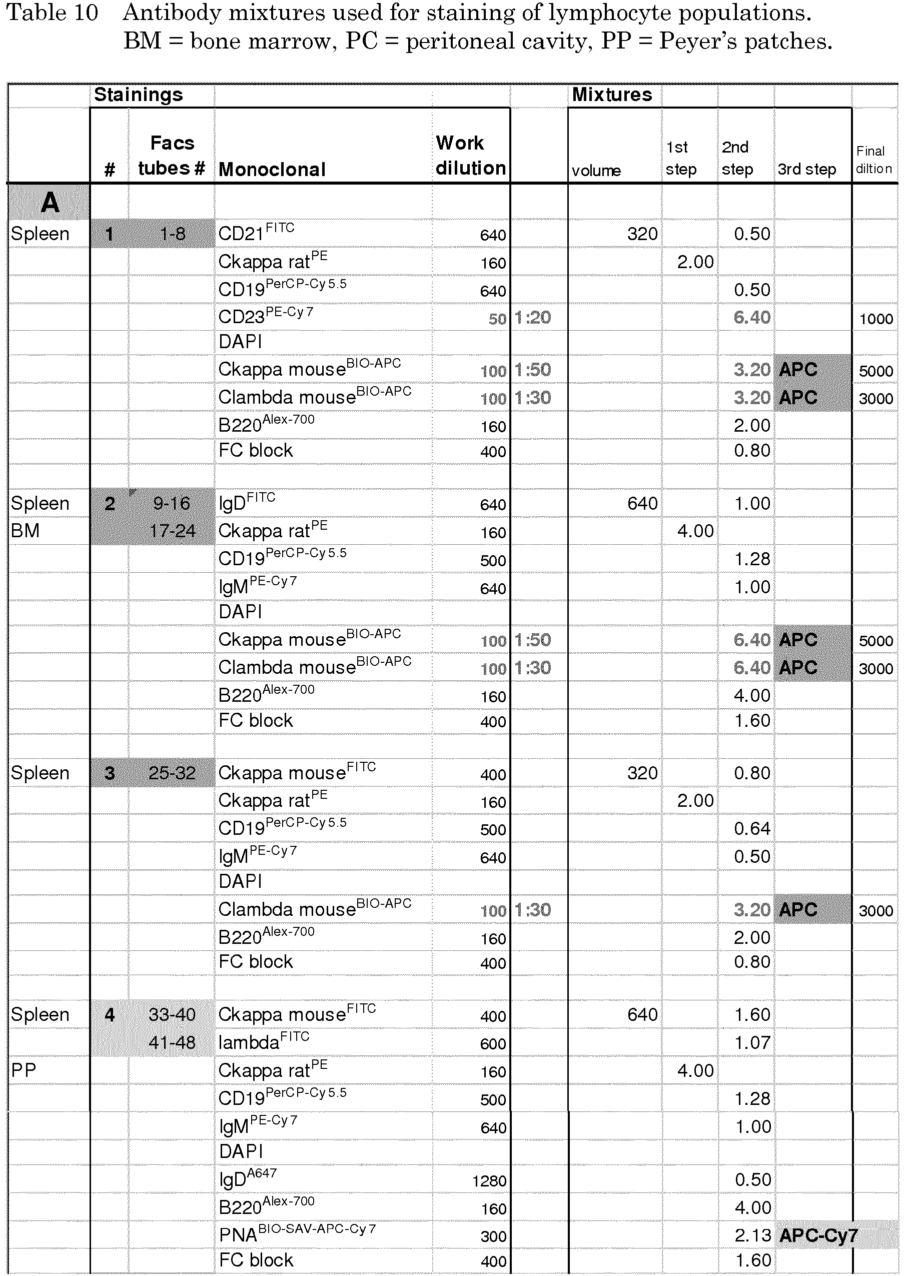

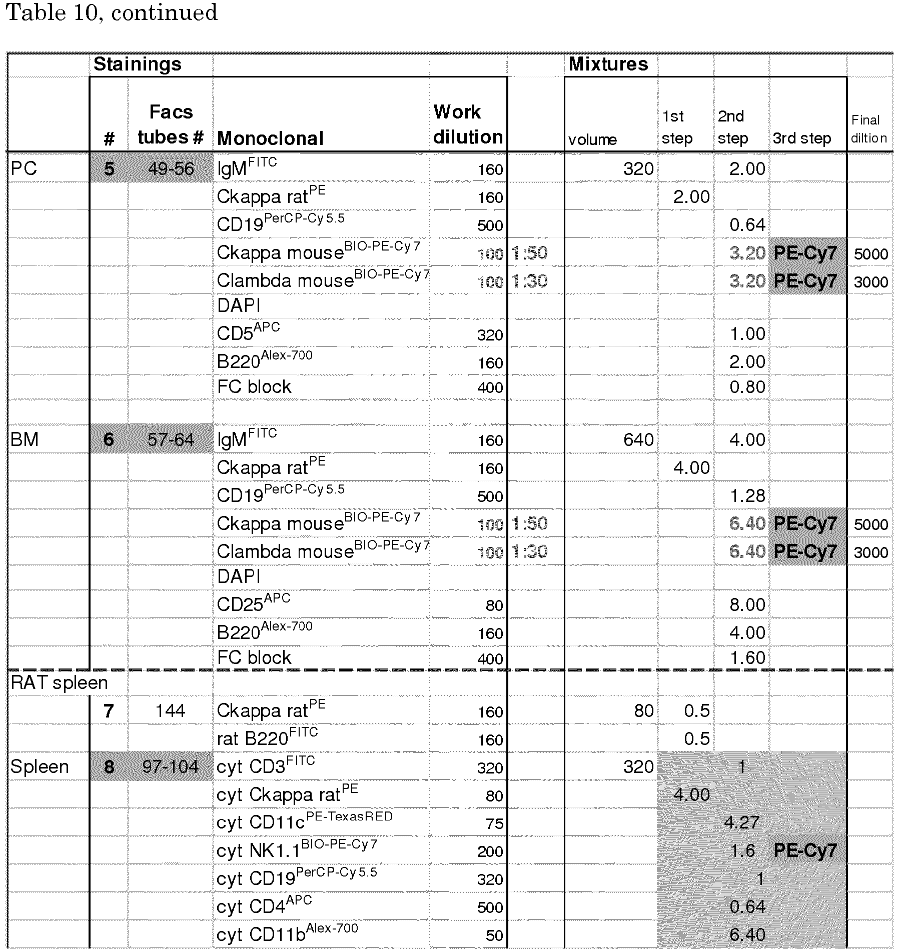

**Table 11 Numbers of lymphocytes harvested from the bone marrow and spleen of wild type and transgenic mice**

| **Bone Marrow** | | | |
|---|---|---|---|
| | ***10/ml** | **total vol** | **total cells** |
| | **cells** | **(ml)** | ***10** |
| **Wt** | **18.82** | **5.05** | **95.0** |
| **Wt** | **19.24** | **4.96** | **95.4** |
| **CD19-Cre** | **23.42** | **5.08** | **119.0** |
| **CD19-Cre** | **20.58** | **4.82** | **99.2** |
| **CD19-Cre** | **25.77** | **5.15** | **132.7** |
| **CD19-Cre / HuVk1** | **17.71** | **5.06** | **89.6** |
| **CD19-Cre / HuVk1** | **12.60** | **6.33** | **67.2** |
| **CD19-Cre / HuVk1** | **18.13** | **5.27** | **95.5** |
| | | | |

| **Spleen** | | | |
|---|---|---|---|
| | ***10s6/ml** | **total vol** | **total cells** |
| | **cells** | **(ml)** | ***10** |
| **Wt** | **41.70** | **5.36** | **223.5** |
| **Wt** | **37.85** | **4.71** | **178.3** |
| **CD19-Cre** | **60.19** | **3.77** | **226.9** |
| **CD19-Cre** | **35.06** | **3.66** | **126.3** |
| **CD19-Cre** | **80.69** | **4.60** | **371.2** |
| **CD19-Cre / HuVk1** | **51.67** | **4.48** | **231.5** |
| **CD19-Cre / HuVk1** | **58.80** | **6.24** | **366.9** |
| **CD19-Cra / HuVk1** | **24.37** | **6.25** | **152.3** |

## Claims

1. Use of a transgenic murine mammal comprising integrated in its genome a rearranged nucleic acid encoding a rearranged human immunoglobulin light chain variable region, wherein said light chain variable region is capable of pairing with at least two different heavy chains encoded by the murine mammal, such that variety in specificity of antibodies is retained through rearrangements and hypermutations in the heavy chains, for the generation of high affinity antibodies comprising said rearranged human immunoglobulin light chain variable region, wherein the antibodies are encoded by VH genes that have undergone somatic hypermutation upon immunization and the non-mutated rearranged human VL gene integrated in the genome.

2. The use according to claim 1, wherein said nucleic acid further encodes an animal light chain constant region.

3. The use according to claim 1 or 2, wherein said antibody is directed against a cellular target.

4. The use according to claim 3, wherein said cellular target is a human surface-expressed or soluble protein or carbohydrate molecule.

5. The use according to claim 3, wherein said cellular target is a surface-expressed protein or carbohydrate molecule that is expressed on the surface of bacteria, viruses, and other pathogens, especially of humans.

6. The use according to claim 3 or 4, wherein said target includes cytokines and chemokines, receptor molecules for cytokines and chemokines, immunoglobulin (Ig) superfamily receptors, tumor necrosis factor family receptors, serine/threonine-protein kinase receptors, tyrosine kinase receptors, EGF receptor family members, insulin receptor family members, PDGF receptor family members, Hepatocyte growth factor receptor family members, Trk receptor family members, AXL receptor family members, LTK receptor family members, TIE receptor family members, ROR receptor family members, DDR receptor family members, KLG receptor family members, RYK receptor family members, MuSK receptor family members, and vascular endothelial growth factor receptor (VEGFR) family members.

7. The use according to claim 6, wherein said antibody is directed against a target that is over-expressed or selectively expressed in tumors.

8. The use according to claim 7, wherein the target is VEGF, CD20, CD38, CD33, CEA, EpCAM, PSMA, CD54, Lewis Y, CD52, CD40, CD22, CD51/CD61, CD74, MUC-1, CD38, CD19, CD262 (TRAIL-R2), RANKL, CTLA4, or CD30.

9. The use according to claim 6, wherein said antibody is directed against a target that is involved in chronic inflammation.

10. The use according to claim 9, wherein the target is CD25, CD11a, TNF, CD4, CD80, CD23, CD3, CD14, IFNgamma, CD40L, CD50, CD122, TGFbeta or TGFalpha.

11. The use according to claim 3 or 5, wherein said target includes surface markers of influenza A and B viruses such as hemagglutinin (HA) and neuraminidase (NA), filoviruses such as Ebola virus, rabies, measles, rubella, mumps, flaviviruses such as Dengue virus types 1-4, tick-borne encephalitis virus, West Nile virus, Japanese encephalitis virus, and Yellow fever virus, Paramyxoviruses including Paramyxovirus such as Parainfluenza 1, 3, Rubulavirus such as Mumpsvirus and Parainfluenza 2, 4, Morbillivirus, and Pneumovirus such as Respiratory syncytial virus, Vaccinia, small pox, coronaviruses, including Severe Acute Respiratory Syndrome (SARS) virus, hepatitis virus A, B and C, Human Immunodeficiency Virus, Herpes viruses, including cytomegalovirus, Epstein Barr virus, Herpes simplex virus, and Varicella zoster virus, parvoviruses such as, for example, B19; Legionella pneumophila; Listeria monocytogenes; Campylobacter jejuni; Staphylococcus aureus; E. coli 0157:H7; Borrelia burgdorferi; Helicobacter pylori; Ehrlichia chaffeensis; Clostridium difficile; Vibrio cholera; Salmonella enterica Serotype Typhimurium; Bartonella henselae; Streptococcus pyogenes (Group A Strep); Streptococcus agalactiae (Group B Strep); Multiple drug resistant S. aureus (e.g. MRSA); Chlamydia pneumoniae; Clostridium botulinum; Vibrio vulnificus; Parachlamydia pneumonia; Corynebacterium amycolatum; Klebsiella pneumonia; Linezolid-resistant enterococci (E. faecalis and E. faecium); and Multiple drug resistant Acinetobacter baumannii.

12. The use according to any one of claims 1-11, wherein a VH gene which has undergone somatic hypermutation and which encodes the VH of a high affinity antibody as generated in any one of claim 1 to 11 is isolated, and wherein said isolated VH gene or copy thereof is integrated into an expression cassette and said high affinity antibody is expressed in a host cell.

## Patentansprüche

1. Verwendung eines transgenen murinen Säugetiers, das eine in seinem Genom integrierte rearrangierte Nukleinsäure umfasst, die für eine rearrangierte variable Region der leichten Kette eines humanen Immunglobulins kodiert, wobei die variable Region der leichten Kette in der Lage ist, sich mit mindestens zwei verschiedenen schweren Ketten zu paaren, die von dem murinen Säugetiers kodiert werden, so dass die Vielfalt der Spezifität von Antikörpern durch Umlagerungen ("Rearrangements") und Hypermutationen in den schweren Ketten erhalten bleibt, zur Erzeugung von Antikörpern mit hoher Affinität, die die rearrangierte variable Region der leichten Kette des humanen Immunglobulins umfassen, wobei die Antikörper von VH-Genen, die bei der Immunisierung eine somatische Hypermutation erfahren haben, und dem in das Genom integrierten nicht-mutierten rearrangierten humanen VL-Gen kodiert werden.

2. Verwendung nach Anspruch 1, wobei die Nukleinsäure ferner für eine konstante Region der leichten Kette eines Tieres kodiert.

3. Verwendung nach Anspruch 1 oder 2, wobei der Antikörper gegen ein zelluläres Ziel gerichtet ist.

4. Verwendung nach Anspruch 3, wobei das zelluläre Ziel ein humanes oberflächenexprimiertes oder lösliches Protein oder Kohlenhydratmolekül ist.

5. Verwendung nach Anspruch 3, wobei das zelluläre Ziel ein oberflächenexprimiertes Protein oder Kohlenhydratmolekül ist, das auf der Oberfläche von Bakterien, Viren und anderen Pathogenen, insbesondere des Menschen, exprimiert wird.

6. Verwendung nach Anspruch 3 oder 4, wobei das Ziel Zytokine und Chemokine, Rezeptormoleküle für Zytokine und Chemokine, Rezeptoren der Immunglobulin (Ig)-Superfamilie, Rezeptoren der Tumornekrosefaktor-Familie, Serin/Threonin-Proteinkinase-Rezeptoren, Tyrosinkinase-Rezeptoren, Mitglieder der EGF-Rezeptorfamilie, Mitglieder der Insulin-Rezeptorfamilie, Mitglieder der PDGF-Rezeptorfamilie, Mitglieder der Hepatozyten-Wachstumsfaktor-Rezeptorfamilie, Mitglieder der Trk-Rezeptorfamilie, Mitglieder der AXL-Rezeptorfamilie, Mitglieder der LTK-Rezeptorfamilie, Mitglieder der TIE-Rezeptorfamilie, Mitglieder der ROR-Rezeptorfamilie, Mitglieder der DDR-Rezeptorfamilie, Mitglieder der KLG-Rezeptorfamilie, Mitglieder der RYK-Rezeptorfamilie, Mitglieder der MuSK-Rezeptorfamilie und Mitglieder der VEGFR (vascular endothelial growth factor receptor) -Rezeptorfamilie einschließe.

7. Verwendung nach Anspruch 6, wobei der Antikörper gegen ein Ziel gerichtet ist, das in Tumoren überexprimiert oder selektiv exprimiert wird.

8. Verwendung nach Anspruch 7, wobei das Ziel VEGF, CD20, CD38, CD33, CEA, EpCAM, PSMA, CD54, Lewis Y, CD52, CD40, CD22, CD51/CD61, CD74, MUC-I, CD38, CD19, CD262 (TRAIL-R2), RANKL, CTLA4 oder CD30 ist.

9. Verwendung nach Anspruch 6, wobei der Antikörper gegen ein Ziel gerichtet ist, das an einer chronischen Entzündung beteiligt ist.

10. Verwendung nach Anspruch 9, wobei das Ziel CD25, CD11a, TNF, CD4, CD80, CD23, CD3, CD14, IFNgamma, CD40L, CD50, CD122, TGFbeta oder TGFalpha ist.

11. Verwendung nach Anspruch 3 oder 5, wobei das Ziel Oberflächenmarker von Influenza A- und B-Viren wie Hämagglutinin (HA) und Neuraminidase (NA), Filoviren wie Ebola-Virus, Tollwut, Masern, Röteln, Mumps, Flaviviren wie Dengue-Virus Typ 1-4, Zeckenenzephalitis-Virus, West-Nil-Virus, Japanisches Enzephalitis-Virus und Gelbfieber-Virus, Paramyxoviren einschließlich Paramyxovirus wie Parainfluenza 1, 3, Rubulavirus wie Mumpsvirus und Parainfluenza 2, 4, Morbillivirus und Pneumovirus wie Respiratorisches Synzytialvirus, Vaccinia, kleine Pocken, Coronaviren, einschließlich des Virus des Schweren Akuten Respiratorischen Syndroms (SARS), Hepatitis-Virus A, B und C, Humanes Immundefizienz-Virus, Herpes-Viren, einschließlich Cytomegalovirus, Epstein-Barr-Virus, Herpessimplex-Virus und Varicella-Zoster-Virus, Parvoviren wie z. B. B19; Legionella pneumophila; Listeria monocytogenes; Campylobacter jejuni; Staphylococcus aureus; E. coli 0157:H7; Borrelia burgdorferi; Helicobacter pylori; Ehrlichia chaffeensis; Clostridium difficile; Vibrio cholera; Salmonella enterica Serotyp Typhimurium; Bartonella henselae; Streptococcus pyogenes (Gruppe A Strep); Streptococcus agalactiae (Gruppe B Strep); mehrfach resistente S. aureus (z. B. MRSA); Chlamydia pneumoniae; Clostridium botulinum; Vibrio vulnificus; Parachlamydia pneumonia; Corynebacterium amycolatum; Klebsiella pneumonia; Linezolid-resistente Enterokokken (E. faecalis und E. faecium); und mehrfach resistenter Acinetobacter baumannii einschließt.

12. Verwendung nach einem der Ansprüche 1 bis 11, wobei ein VH-Gen, das eine somatische Hypermutation erfahren hat und das für das VH eines Antikörpers mit hoher Affinität, wie er in einem der Ansprüche 1 bis 11 erzeugt wurde, kodiert, isoliert wird, und wobei das isolierte VH-Gen oder eine Kopie davon in eine Expressionskassette integriert wird und der Antikörper mit hoher Affinität in einer Wirtszelle exprimiert wird.

## Revendications

1. Utilisation d'un mammifère murin transgénique comprenant dans son génome un acide nucléique réarrangé codant pour une région variable de chaîne légère d'immunoglobuline humaine réarrangée, dans laquelle ladite région variable de chaîne légère est capable de s'apparier avec au moins deux chaînes lourdes différentes codées par le mammifère murin, de sorte que la variété de la spécificité des anticorps est conservée par des réarrangements et des hypermutations dans les chaînes lourdes, pour la production d'anticorps de haute affinité comprenant cette région variable de chaîne légère d'immunoglobuline humaine réarrangée, dans laquelle les anticorps sont codés par des gènes VH qui ont subi une hypermutation somatique lors de l'immunisation et le gène VL humain réarrangé non muté intégré dans le génome.

2. Utilisation selon la revendication 1, dans laquelle ledit acide nucléique code en outre pour une région constante de chaîne légère animale.

3. Utilisation selon la revendication 1 ou 2, dans laquelle ledit anticorps est dirigé contre une cible cellulaire.

4. Utilisation selon la revendication 3, dans laquelle ladite cible cellulaire est une protéine humaine exprimée en surface ou soluble ou une molécule d'hydrate de carbone.

5. Utilisation selon la revendication 3, dans laquelle ladite cible cellulaire est une protéine exprimée en surface ou une molécule d'hydrate de carbone exprimée à la surface des bactéries, des virus et d'autres agents pathogènes, en particulier chez l'homme.

6. Utilisation selon la revendication 3 ou 4, dans laquelle ladite cible comprend des cytokines et des chimiokines, des molécules réceptrices de cytokines et de chimiokines, des récepteurs de la superfamille des immunoglobulines (Ig), des récepteurs de la famille du facteur de nécrose tumorale, des récepteurs de sérine/thréonine-protéine kinase, des récepteurs de tyrosine kinase, des membres de la famille des récepteurs de l'EGF, des membres de la famille des récepteurs de l'insuline, des membres de la famille des récepteurs du PDGF, les récepteurs du facteur de croissance des hépatocytes, les récepteurs Trk, les récepteurs AXL, les récepteurs LTK, les récepteurs TIE, les récepteurs ROR, les récepteurs DDR, les récepteurs KLG, les récepteurs RYK, les récepteurs MuSK et les récepteurs du facteur de croissance de l'endothélium vasculaire (VEGFR).

7. Utilisation selon la revendication 6, dans laquelle ledit anticorps est dirigé contre une cible qui est surexprimée ou sélectivement exprimée dans les tumeurs.

8. Utilisation selon la revendication 7, dans laquelle la cible est VEGF, CD20, CD38, CD33, CEA, EpCAM, PSMA, CD54, Lewis Y, CD52, CD40, CD22, CD51/CD61, CD74, MUC-I, CD38, CD19, CD262 (TRAIL-R2), RANKL, CTLA4 ou CD30.

9. Utilisation selon la revendication 6, dans laquelle ledit anticorps est dirigé contre une cible impliquée dans l'inflammation chronique.

10. Utilisation selon la revendication 9, dans laquelle la cible est CD25, CD11a, TNF, CD4, CD80, CD23, CD3, CD14, IFNgamma, CD40L, CD50, CD122, TGFbeta ou TGFalpha.

11. Utilisation selon la revendication 3 ou 5, dans laquelle ladite cible comprend des marqueurs de surface des virus de la grippe A et B tels que l'hémagglutinine (HA) et la neuraminidase (NA), des filovirus tels que le virus Ebola, la rage, la rougeole, la rubéole, les oreillons, des flavivirus tels que le virus de la dengue de types 1 à 4, le virus de l'encéphalite à tiques, le virus du Nil occidental, le virus de l'encéphalite japonaise et le virus de la fièvre jaune, des paramyxovirus, y compris des paramyxovirus tels que le Parainfluenza 1, 3, les Rubulavirus tels que le virus des oreillons et le Parainfluenza 2, 4, le Morbillivirus, et les Pneumovirus tels que le virus respiratoire syncytial, le Vaccinia, la variole, les coronavirus, y compris le virus du syndrome respiratoire aigu sévère (SRAS), les virus de l'hépatite A, B et C, le virus de l'immunodéficience humaine, les virus de l'herpès, y compris le cytomégalovirus, le virus d'Epstein Barr, le virus de l'herpès simplex et le virus de la varicelle et du zona, les parvovirus tels que, par exemple, le B19 ; Legionella pneumophila ; Listeria monocytogenes ; Campylobacter jejuni ; Staphylococcus aureus ; E. coli 0157:H7 ; Borrelia burgdorferi ; Helicobacter pylori ; Ehrlichia chaffeensis ; Clostridium difficile ; Vibrio cholera ; Salmonella enterica Serotype Typhimurium ; Bartonella henselae ; Streptococcus pyogenes (streptocoque du groupe A) ; Streptococcus agalactiae (streptocoque du groupe B) ; S. aureus (par exemple SARM) ; Chlamydia pneumoniae ; Clostridium botulinum ; Vibrio vulnificus ; Parachlamydia pneumonia ; Corynebacterium amycolatum ; Klebsiella pneumonia ; Entérocoques résistants au linézolide (E. faecalis et E. faecium) ; et Acinetobacter baumannii résistant à plusieurs médicaments.

12. Utilisation selon l'une des revendications 1 à 11, dans laquelle un gène VH ayant subi une hypermutation somatique et codant pour le VH d'un anticorps de haute affinité tel que généré dans l'une des revendications 1 à 11 est isolé, et dans laquelle ledit gène VH isolé ou une copie de celui-ci est intégré dans une cassette d'expression et ledit anticorps de haute affinité est exprimé dans une cellule hôte.
